(19) Europäisches Patentamt
European Patent Office
Office européen des brevets

(11) EP 4 696 329 A1

(12) EUROPEAN PATENT APPLICATION
published in accordance with Art. 153(4) EPC

(43) Date of publication:
**18.02.2026 Bulletin 2026/08**

(21) Application number: **24788142.8**

(22) Date of filing: **11.04.2024**

(51) International Patent Classification (IPC):
***A61K 47/68*** *(2017.01)* ***A61K 45/00*** *(2006.01)*
***A61P 35/00*** *(2006.01)* ***C07K 16/28*** *(2006.01)*

(52) Cooperative Patent Classification (CPC):
**A61K 45/00; A61K 47/68; A61P 35/00; C07K 16/28**

(86) International application number:
**PCT/CN2024/087225**

(87) International publication number:
**WO 2024/213040 (17.10.2024 Gazette 2024/42)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **11.04.2023 CN 202310381704**

(71) Applicants:
- **BioRay Pharmaceutical Co., Ltd.**
  **Jiaojiang District,**
  **Taizhou**
  **Zhejiang 318000 (CN)**
- **Bioray Pharmaceutical (Hangzhou) Co., Ltd.**
  **Hangzhou, Zhejiang 311404 (CN)**
- **Bioray Pharmaceutical Corp.**
  **San Diego, CA 92121 (US)**

(72) Inventors:
- **ZHU, Jie**
  **San Diego, CA 92121 (US)**
- **ZHAO, Xiaobei**
  **San Diego, CA 92121 (US)**
- **WU, Zhenhua**
  **Taizhou, Zhejiang 318000 (CN)**
- **NIE, Lei**
  **Taizhou, Zhejiang 318000 (CN)**
- **MEI, Xiaofen**
  **Taizhou, Zhejiang 318000 (CN)**
- **WANG, Xinzeng**
  **Taizhou, Zhejiang 318000 (CN)**
- **WANG, Xiaoze**
  **Taizhou, Zhejiang 318000 (CN)**
- **PAN, Chenxiao**
  **Taizhou, Zhejiang 318000 (CN)**
- **ZHOU, Yaqiong**
  **Taizhou, Zhejiang 318000 (CN)**
- **CHEN, Gang**
  **San Diego, CA 92121 (US)**
- **WANG, Haibin**
  **Hangzhou, Zhejiang 311404 (CN)**

(74) Representative: **Kador & Partner Part mbB**
**Corneliusstraße 15**
**80469 München (DE)**

Remarks:
The complete document including Reference Table(s) and the Sequence Listing(s) can be downloaded from the EPO website

# (54) ANTI-ROR1 ANTIBODY AND DRUG CONJUGATE THEREOF

(57) An anti-ROR1 antibody, and a multispecific antibody prepared on the basis of the anti-ROR1 antibody. Further provided is an antibody-drug conjugate comprising the anti-ROR1 antibody or the multispecific antibody.

EP 4 696 329 A1

## Description

TECHNICAL FIELD

The present disclosure relates to the field of antibody drugs and particularly to an anti-ROR1 antibody and a drug conjugate thereof, as well as use of the anti-ROR1 antibody and the drug conjugate thereof.

BACKGROUND

ROR1 is a transmembrane receptor tyrosine kinase protein and a member of the type I receptor tyrosine kinase (RTK) family. Human ROR1 consists of an extracellular immunoglobulin-like domain (Ig), two cysteine-rich domains (FZD), a membrane-proximal kringle domain, a single-pass transmembrane structure and an intracellular tyrosine kinase domain (TKD), two serine/threonine-rich domains (S/TRD), and a proline-rich domain (PRD). ROR1 is highly expressed during embryonic and infant development and plays an important role in a variety of physiological processes, including the regulation of cell division, proliferation, and migration and cell chemotaxis, and in the generation of nerves, bones, and vascular organs. In subsequent developmental processes, ROR1 expression significantly decreases. ROR1 expression is absent in normal adult tissues except for B lymphocyte precursor cells. However, ROR1 is highly expressed in a variety of hematological tumors and solid tumors. Hematological tumors that highly express ROR1 include B cell chronic lymphocytic leukemia (CLL), acute lymphoblastic leukemia (ALL), non-Hodgkin lymphoma (NHL), and myeloid hematological cancer. Among solid tumors, tumors that express ROR1 include triple-negative breast cancer, colon cancer, lung cancer, pancreatic cancer, ovarian cancer, etc. Moreover, ROR1 expression is closely related to disease progression and therapeutic effects. Studies have found that ROR1 can participate in Wnt5a-mediated non-canonical Wnt signaling pathways. Wnt5a activates the receptor ROR1 or FZD5, resulting in Dvl2/3 activation and Akt phosphorylation. Akt, in turn, promotes IKK$\alpha$ phosphorylation, activating the IKK complex. The IKK complex degrades I$\kappa$B$\alpha$ and facilitates the phosphorylation of the NF-$\kappa$B subunit p65. Phosphorylated p65 is transferred to the nucleus, facilitating the transcriptional expression of target genes, including Wnt5a. Then the secretion of Wnt5a facilitates a new autonomous feedback cycle. The activation of the ROR1/Akt/p65 pathway of the autonomous feedback cycle will further facilitates the secretion of pro-inflammatory factors (such as IL-6) and chemokines (such as CCL2). In addition, studies suggest that ROR1 is associated with EMT in tumor cells or the activation of YAP/TAZ transcription, thereby enhancing tumorigenesis and chemoresistance.

SUMMARY

The objective of the present disclosure is to provide an anti-ROR1 antibody and an antibody-drug conjugate (ADC) comprising the anti-ROR1 antibody. The present disclosure further provides use of the anti-ROR1 antibody or the antibody-drug conjugate in the treatment of a tumor or cancer.

In a first aspect, the present disclosure provides an anti-ROR1 antibody, wherein the anti-ROR1 antibody comprises a heavy chain variable region and a light chain variable region, wherein:

(Z1) the heavy chain variable region comprises an HCDR1 set forth in SEQ ID NO: 61, an HCDR2 set forth in SEQ ID NO: 46, and an HCDR3 set forth in SEQ ID NO: 65, and the light chain variable region comprises an LCDR1 set forth in SEQ ID NO: 63, an LCDR2 set forth in SEQ ID NO: 64, and an LCDR3 set forth in SEQ ID NO: 50;

(Z2) the heavy chain variable region comprises an HCDR1 set forth in SEQ ID NO: 45, an HCDR2 set forth in SEQ ID NO: 46, and an HCDR3 set forth in SEQ ID NO: 66, and the light chain variable region comprises an LCDR1 set forth in SEQ ID NO: 63, an LCDR2 set forth in SEQ ID NO: 64, and an LCDR3 set forth in SEQ ID NO: 50;

(Z3) the heavy chain variable region comprises an HCDR1 set forth in SEQ ID NO: 45, an HCDR2 set forth in SEQ ID NO: 46, and an HCDR3 set forth in SEQ ID NO: 65, and the light chain variable region comprises an LCDR1 set forth in SEQ ID NO: 67, an LCDR2 set forth in SEQ ID NO: 68, and an LCDR3 set forth in SEQ ID NO: 50;

(Z4) the heavy chain variable region comprises an HCDR1 set forth in SEQ ID NO: 45, an HCDR2 set forth in SEQ ID NO: 46, and an HCDR3 set forth in SEQ ID NO: 65, and the light chain variable region comprises an LCDR1 set forth in SEQ ID NO: 63, an LCDR2 set forth in SEQ ID NO: 69, and an LCDR3 set forth in SEQ ID NO: 50;

(Z5) the heavy chain variable region comprises an HCDR1 set forth in SEQ ID NO: 45, an HCDR2 set forth in SEQ ID NO: 46, and an HCDR3 set forth in SEQ ID NO: 65, and the light chain variable region comprises an LCDR1 set forth in SEQ ID NO: 63, an LCDR2 set forth in SEQ ID NO: 64, and an LCDR3 set forth in SEQ ID NO: 50;

(Z6) the heavy chain variable region comprises an HCDR1 set forth in SEQ ID NO: 17, an HCDR2 set forth in SEQ ID NO: 32, and an HCDR3 set forth in SEQ ID NO: 19, and the light chain variable region comprises an LCDR1 set forth in SEQ ID NO: 20, an LCDR2 set forth in SEQ ID NO: 21, and an LCDR3 set forth in SEQ ID NO: 10; or

(Z7) the heavy chain variable region comprises an HCDR1 set forth in SEQ ID NO: 17, an HCDR2 set forth in SEQ ID NO: 18, and an HCDR3 set forth in SEQ ID NO: 19, and the light chain variable region comprises an LCDR1 set forth in SEQ ID NO: 20, an LCDR2 set forth in SEQ ID NO: 21, and an LCDR3 set forth in SEQ ID NO: 10.

In some embodiments, provided is the aforementioned anti-ROR1 antibody, wherein the anti-ROR1 antibody comprises a heavy chain variable region and a light chain variable region, wherein:

(Z1) the heavy chain variable region comprises an HCDR1 set forth in SEQ ID NO: 61, an HCDR2 set forth in SEQ ID NO: 46, and an HCDR3 set forth in SEQ ID NO: 65, and the light chain variable region comprises an LCDR1 set forth in SEQ ID NO: 63, an LCDR2 set forth in SEQ ID NO: 64, and an LCDR3 set forth in SEQ ID NO: 50; or

(Z6) the heavy chain variable region comprises an HCDR1 set forth in SEQ ID NO: 17, an HCDR2 set forth in SEQ ID NO: 32, and an HCDR3 set forth in SEQ ID NO: 19, and the light chain variable region comprises an LCDR1 set forth in SEQ ID NO: 20, an LCDR2 set forth in SEQ ID NO: 21, and an LCDR3 set forth in SEQ ID NO: 10.

In some embodiments, provided is the anti-ROR1 antibody according to any one of the foregoing, wherein the CDRs are defined according to the Kabat numbering scheme.

In some embodiments, the anti-ROR1 antibody according to any one of the foregoing is a murine antibody, a chimeric antibody, or a humanized antibody.

In some embodiments, the anti-ROR1 antibody according to any one of the foregoing is a humanized antibody and comprises FRs of a human antibody. In some embodiments, provided is the aforementioned anti-ROR1 antibody, wherein the heavy chain variable region comprises a sequence having at least 85%, 86%, 87%, 88%, 89%, 90%, 91%, 92%, 93%, 94%, 95%, 96%, 97%, 98%, or 99% sequence identity to the amino acid sequence set forth in SEQ ID NO: 72, and the light chain variable region comprises a sequence having at least 85%, 86%, 87%, 88%, 89%, 90%, 91%, 92%, 93%, 94%, 95%, 96%, 97%, 98%, or 99% sequence identity to the amino acid sequence set forth in SEQ ID NO: 71; or

the heavy chain variable region comprises a sequence having at least 85%, 86%, 87%, 88%, 89%, 90%, 91%, 92%, 93%, 94%, 95%, 96%, 97%, 98%, or 99% sequence identity to the amino acid sequence set forth in SEQ ID NO: 73, and the light chain variable region comprises a sequence having at least 85% sequence identity to the amino acid sequence set forth in SEQ ID NO: 71; or

the heavy chain variable region comprises a sequence having at least 85%, 86%, 87%, 88%, 89%, 90%, 91%, 92%, 93%, 94%, 95%, 96%, 97%, 98%, or 99% sequence identity to the amino acid sequence set forth in SEQ ID NO: 74, and the light chain variable region comprises a sequence having at least 85%, 86%, 87%, 88%, 89%, 90%, 91%, 92%, 93%, 94%, 95%, 96%, 97%, 98%, or 99% sequence identity to the amino acid sequence set forth in SEQ ID NO: 75; or

the heavy chain variable region comprises a sequence having at least 85%, 86%, 87%, 88%, 89%, 90%, 91%, 92%, 93%, 94%, 95%, 96%, 97%, 98%, or 99% sequence identity to the amino acid sequence set forth in SEQ ID NO: 74, and the light chain variable region comprises a sequence having at least 85%, 86%, 87%, 88%, 89%, 90%, 91%, 92%, 93%, 94%, 95%, 96%, 97%, 98%, or 99% sequence identity to the amino acid sequence set forth in SEQ ID NO: 76; or

the heavy chain variable region comprises a sequence having at least 85%, 86%, 87%, 88%, 89%, 90%, 91%, 92%, 93%, 94%, 95%, 96%, 97%, 98%, or 99% sequence identity to the amino acid sequence set forth in SEQ ID NO: 74, and the light chain variable region comprises a sequence having at least 85%, 86%, 87%, 88%, 89%, 90%, 91%, 92%, 93%, 94%, 95%, 96%, 97%, 98%, or 99% sequence identity to the amino acid sequence set forth in SEQ ID NO: 71; or

the heavy chain variable region comprises a sequence having at least 85%, 86%, 87%, 88%, 89%, 90%, 91%, 92%, 93%, 94%, 95%, 96%, 97%, 98%, or 99% sequence identity to the amino acid sequence set forth in SEQ ID NO: 22, and the light chain variable region comprises a sequence having at least 85%, 86%, 87%, 88%, 89%, 90%, 91%, 92%, 93%, 94%, 95%, 96%, 97%, 98%, or 99% sequence identity to the amino acid sequence set forth in SEQ ID NO: 23; or

the heavy chain variable region comprises a sequence having at least 85%, 86%, 87%, 88%, 89%, 90%, 91%, 92%, 93%, 94%, 95%, 96%, 97%, 98%, or 99% sequence identity to the amino acid sequence set forth in SEQ ID NO: 38, and the light chain variable region comprises a sequence having at least 85%, 86%, 87%, 88%, 89%, 90%, 91%, 92%,

93%, 94%, 95%, 96%, 97%, 98%, or 99% sequence identity to the amino acid sequence set forth in SEQ ID NO: 23.

In some embodiments, provided is the aforementioned anti-ROR1 antibody, wherein:

the heavy chain variable region comprises the amino acid sequence set forth in SEQ ID NO: 72, and the light chain variable region comprises the amino acid sequence set forth in SEQ ID NO: 71; or

the heavy chain variable region comprises the amino acid sequence set forth in SEQ ID NO: 73, and the light chain variable region comprises the amino acid sequence set forth in SEQ ID NO: 71; or

the heavy chain variable region comprises the amino acid sequence set forth in SEQ ID NO: 74, and the light chain variable region comprises the amino acid sequence set forth in SEQ ID NO: 75; or

the heavy chain variable region comprises the amino acid sequence set forth in SEQ ID NO: 74, and the light chain variable region comprises the amino acid sequence set forth in SEQ ID NO: 76; or

the heavy chain variable region comprises the amino acid sequence set forth in SEQ ID NO: 74, and the light chain variable region comprises the amino acid sequence set forth in SEQ ID NO: 71; or

the heavy chain variable region comprises the amino acid sequence set forth in SEQ ID NO: 22, and the light chain variable region comprises the amino acid sequence set forth in SEQ ID NO: 23; or

the heavy chain variable region comprises the amino acid sequence set forth in SEQ ID NO: 38, and the light chain variable region comprises the amino acid sequence set forth in SEQ ID NO: 23.

In some embodiments, provided is the aforementioned anti-ROR1 antibody, wherein the heavy chain variable region comprises the amino acid sequence set forth in SEQ ID NO: 72, and the light chain variable region comprises the amino acid sequence set forth in SEQ ID NO: 71; or
the heavy chain variable region comprises the amino acid sequence set forth in SEQ ID NO: 38, and the light chain variable region comprises the amino acid sequence set forth in SEQ ID NO: 23.
In some embodiments, the aforementioned anti-ROR1 antibody comprising heavy chain variable region CDRs and light chain variable region CDRs selected from any one of groups (Z1)-(Z5) binds to an epitope located at amino acid positions 70-130 of an ROR1 protein, and the aforementioned anti-ROR1 antibody comprising (Z6) or (Z7) binds to an epitope located at amino acid positions 130-165 of an ROR1 protein, wherein the amino acid sequence of the ROR1 protein is set forth in SEQ ID NO: 87.
In some embodiments, the anti-ROR1 antibody comprises a heavy chain constant region; in some embodiments, the heavy chain constant region is of subtype IgG1 or IgG4.
In some embodiments, the anti-ROR1 antibody comprises a light chain constant region; in some embodiments, the light chain constant region is of type κ.
In some embodiments, the anti-ROR1 antibody comprises a heavy chain constant region and a light chain constant region. In some embodiments, the heavy chain constant region comprises the amino acid sequence set forth in SEQ ID NO: 11 or a variant thereof, and/or the light chain constant region comprises the amino acid sequence set forth in SEQ ID NO: 12 or a variant thereof. In some embodiments, the heavy chain constant region variant and the light chain constant region variant are both conventional variants.
In some embodiments, the anti-ROR1 antibody comprises:

a heavy chain having an amino acid sequence as set forth in SEQ ID NO: 77 and a light chain having an amino acid sequence as set forth in SEQ ID NO: 78; or

a heavy chain having an amino acid sequence as set forth in SEQ ID NO: 30 and a light chain having an amino acid sequence as set forth in SEQ ID NO: 31; or

a heavy chain having an amino acid sequence as set forth in SEQ ID NO: 44 and a light chain having an amino acid sequence as set forth in SEQ ID NO: 31.

In some embodiments, the anti-ROR1 antibody according to the foregoing is an antibody fragment; in some embodiments, the antibody fragment is selected from the group consisting of Fab, Fab', $F(ab')_2$, Fd, Fv, scFv, dsFv, and dAb.

In a second aspect, the present disclosure provides a multispecific antibody comprising the anti-ROR1 antibody according to the first aspect of the present disclosure.

In some embodiments, the multispecific antibody targets an ROR1 protein and a non-ROR1 protein simultaneously, or the multispecific antibody targets multiple different epitopes of an ROR1 protein simultaneously.

In some embodiments, the non-ROR1 protein includes tumor-associated antigens.

In some embodiments, the multispecific antibody is a bispecific antibody comprising a first antigen-binding domain and a second antigen-binding domain, wherein the first antigen-binding domain and the second antigen-binding domain bind to different epitopes on human ROR1; in some embodiments, the first antigen-binding domain does not block the binding of the second antigen-binding domain to human ROR1; in some embodiments, the first antigen-binding domain binds to the amino acids at positions 130-165 of human ROR1, and the second antigen-binding domain binds to the amino acids at positions 70-130 of human ROR1; the amino acid sequence of the ROR1 protein is set forth in SEQ ID NO: 87.

In some embodiments, the first antigen-binding domain comprises a heavy chain variable region VH1 and a light chain variable region VL1, and the second antigen-binding domain comprises a heavy chain variable region VH2 and a light chain variable region VL2, wherein:

the heavy chain variable region VH1 comprises an HCDR1 set forth in SEQ ID NO: 17, an HCDR2 set forth in SEQ ID NO: 32, and an HCDR3 set forth in SEQ ID NO: 19, and the light chain variable region VL1 comprises an LCDR1 set forth in SEQ ID NO: 20, an LCDR2 set forth in SEQ ID NO: 21, and an LCDR3 set forth in SEQ ID NO: 10; or

the heavy chain variable region VH1 comprises an HCDR1 set forth in SEQ ID NO: 17, an HCDR2 set forth in SEQ ID NO: 18, and an HCDR3 set forth in SEQ ID NO: 19, and the light chain variable region VL1 comprises an LCDR1 set forth in SEQ ID NO: 20, an LCDR2 set forth in SEQ ID NO: 21, and an LCDR3 set forth in SEQ ID NO: 10; and

the heavy chain variable region VH2 comprises an HCDR1 set forth in SEQ ID NO: 61, an HCDR2 set forth in SEQ ID NO: 46, and an HCDR3 set forth in SEQ ID NO: 65, and the light chain variable region VL2 comprises an LCDR1 set forth in SEQ ID NO: 63, an LCDR2 set forth in SEQ ID NO: 64, and an LCDR3 set forth in SEQ ID NO: 50; or

the heavy chain variable region VH2 comprises an HCDR1 set forth in SEQ ID NO: 45, an HCDR2 set forth in SEQ ID NO: 46, and an HCDR3 set forth in SEQ ID NO: 66, and the light chain variable region VL2 comprises an LCDR1 set forth in SEQ ID NO: 63, an LCDR2 set forth in SEQ ID NO: 64, and an LCDR3 set forth in SEQ ID NO: 50; or

the heavy chain variable region VH2 comprises an HCDR1 set forth in SEQ ID NO: 45, an HCDR2 set forth in SEQ ID NO: 46, and an HCDR3 set forth in SEQ ID NO: 65, and the light chain variable region VL2 comprises an LCDR1 set forth in SEQ ID NO: 67, an LCDR2 set forth in SEQ ID NO: 68, and an LCDR3 set forth in SEQ ID NO: 50; or

the heavy chain variable region VH2 comprises an HCDR1 set forth in SEQ ID NO: 45, an HCDR2 set forth in SEQ ID NO: 46, and an HCDR3 set forth in SEQ ID NO: 65, and the light chain variable region VL2 comprises an LCDR1 set forth in SEQ ID NO: 63, an LCDR2 set forth in SEQ ID NO: 69, and an LCDR3 set forth in SEQ ID NO: 50; or

the heavy chain variable region VH2 comprises an HCDR1 set forth in SEQ ID NO: 45, an HCDR2 set forth in SEQ ID NO: 46, and an HCDR3 set forth in SEQ ID NO: 65, and the light chain variable region VL2 comprises an LCDR1 set forth in SEQ ID NO: 63, an LCDR2 set forth in SEQ ID NO: 64, and an LCDR3 set forth in SEQ ID NO: 50.

In some embodiments, provided is the bispecific antibody according to the foregoing, wherein the first antigen-binding domain comprises: the heavy chain variable region VH1 comprises an HCDR1 set forth in SEQ ID NO: 17, an HCDR2 set forth in SEQ ID NO: 32, and an HCDR3 set forth in SEQ ID NO: 19, and the light chain variable region VL1 comprises an LCDR1 set forth in SEQ ID NO: 20, an LCDR2 set forth in SEQ ID NO: 21, and an LCDR3 set forth in SEQ ID NO: 10; and the second antigen-binding domain comprises:
the heavy chain variable region VH2 comprises an HCDR1 set forth in SEQ ID NO: 61, an HCDR2 set forth in SEQ ID NO: 46, and an HCDR3 set forth in SEQ ID NO: 65, and the light chain variable region VL2 comprises an LCDR1 set forth in SEQ ID NO: 63, an LCDR2 set forth in SEQ ID NO: 64, and an LCDR3 set forth in SEQ ID NO: 50.

In some embodiments, provided is the bispecific antibody according to the foregoing, wherein the first antigen-binding domain comprises:

the heavy chain variable region VH1 comprises the amino acid sequence set forth in SEQ ID NO: 38, and the light chain variable region VL1 comprises the amino acid sequence set forth in SEQ ID NO: 23; or

the heavy chain variable region VH1 comprises the amino acid sequence set forth in SEQ ID NO: 22, and the light chain variable region VL1 comprises the amino acid sequence set forth in SEQ ID NO: 23;

and the second antigen-binding domain comprises:

the heavy chain variable region VH2 comprises the amino acid sequence set forth in SEQ ID NO: 72, and the light chain variable region VL2 comprises the amino acid sequence set forth in SEQ ID NO: 71; or

the heavy chain variable region VH2 comprises the amino acid sequence set forth in SEQ ID NO: 73, and the light chain variable region VL2 comprises the amino acid sequence set forth in SEQ ID NO: 71; or

the heavy chain variable region VH2 comprises the amino acid sequence set forth in SEQ ID NO: 74, and the light chain variable region VL2 comprises the amino acid sequence set forth in SEQ ID NO: 75; or

the heavy chain variable region VH2 comprises the amino acid sequence set forth in SEQ ID NO: 74, and the light chain variable region VL2 comprises the amino acid sequence set forth in SEQ ID NO: 76; or

the heavy chain variable region VH2 comprises the amino acid sequence set forth in SEQ ID NO: 74, and the light chain variable region VL2 comprises the amino acid sequence set forth in SEQ ID NO: 71.

In some embodiments, provided is the bispecific antibody according to the foregoing, wherein the first antigen-binding domain comprises a heavy chain variable region VH1 set forth in SEQ ID NO: 38 and a light chain variable region VL1 set forth in SEQ ID NO: 23, and the second antigen-binding domain comprises a heavy chain variable region VH2 set forth in SEQ ID NO: 72 and a light chain variable region VL2 set forth in SEQ ID NO: 71.

In some embodiments, the bispecific antibody comprises an Fc region, wherein the Fc region comprises a first subunit Fc1 and a second subunit Fc2 that are capable of associating with each other, and the Fc1 and Fc2 each independently have one or more amino acid substitutions that reduce homodimerization of the Fc region.

In some embodiments, provided is the bispecific antibody according to the foregoing, wherein the Fc1 has a knob structure according to the knob-in-hole technique, and the Fc2 has a hole structure according to the knob-in-hole technique.

In some embodiments, provided is the bispecific antibody according to any one of the foregoing, wherein the bispecific antibody comprises an Fc region, and the Fc region comprises a first subunit Fc1 and a second subunit Fc2 that are capable of associating with each other, wherein the Fc1 has a knob structure according to the knob-into-hole technique, and the Fc2 has a hole structure according to the knob-into-hole technique; in some embodiments, the amino acid at position 366 of the Fc1 is W, the amino acid at position 366 of the Fc2 is S, the amino acid at position 368 of the Fc2 is A, and the amino acid at position 407 is V of the Fc2; the amino acid positions are numbered according to the EU index.

In some embodiments, provided is the bispecific antibody according to any one of the foregoing, wherein the bispecific antibody comprises an Fc region, and the Fc region comprises a first subunit Fc1 and a second subunit Fc2 that are capable of associating with each other, wherein the Fc1 comprises the amino acid sequence of SEQ ID NO: 79, and the Fc2 comprises the amino acid sequence of SEQ ID NO: 80.

In some embodiments, provided is the bispecific antibody according to any one of the foregoing, wherein the bispecific antibody comprises four chains represented by (a)-(d):

(a) [heavy chain variable region VH1]-[CH1]-[Fc1],

(b) [light chain variable region VL1]-[CL1],

(c) [heavy chain variable region VH2]-[CH1]-[Fc2], and

(d) [light chain variable region VL2]-[CL2]; or

the bispecific antibody comprises four chains represented by (e), (b), (f), and (d):

(e) [heavy chain variable region VH1]-[CH1]-[Fc2],

(b) [light chain variable region VL1]-[CL1],

(f) [heavy chain variable region VH2]-[CH1]-[Fc1], and

(d) [light chain variable region VL2]-[CL2];

wherein the structures represented by formulas (a), (b), (c), (d), (e), and (f) are arranged from the N-terminus to the C-terminus; the CL1 and CL2 are each independently antibody light chain constant regions, and the amino acid sequences thereof may be identical or different; the CH1 is a first portion of an antibody heavy chain constant region.

In some embodiments, provided is the bispecific antibody according to the foregoing, wherein the heavy chain variable region VH1 comprises the amino acid sequence set forth in SEQ ID NO: 38, the light chain variable region VL1 comprises the amino acid sequence set forth in SEQ ID NO: 23, the heavy chain variable region VH2 comprises the amino acid sequence set forth in SEQ ID NO: 72, and the light chain variable region VL2 comprises the amino acid sequence set forth in SEQ ID NO: 71.

In some embodiments, provided is the bispecific antibody according to any one of the foregoing, wherein the CH1 is a CH1 sequence of an IgG. In some embodiments, the CH1 is a CH1 of IgG1. In some embodiments, the CH1 comprises the amino acid sequence set forth in SEQ ID NO: 88.

In some embodiments, provided is the bispecific antibody according to any one of the foregoing, wherein the CL1 and CL2 are antibody light chain constant regions. In some embodiments, provided is the bispecific antibody according to any one of the foregoing, wherein the CL1 or CL2 is a light chain constant region of kappa or lambda. In some embodiments, the CL1 and/or CL2 comprise/comprises the amino acid sequence set forth in SEQ ID NO: 12.

In some embodiments, provided is the bispecific antibody according to the foregoing, wherein the bispecific antibody comprises the following four chains:

a chain 1 comprising the amino acid sequence set forth in SEQ ID NO: 81;

a chain 2 comprising the amino acid sequence set forth in SEQ ID NO: 82;

a chain 3 comprising the amino acid sequence set forth in SEQ ID NO: 83; and

a chain 4 comprising the amino acid sequence set forth in SEQ ID NO: 84.

In a third aspect, the present disclosure provides a nucleic acid molecule encoding the anti-ROR1 antibody according to the first aspect of the present disclosure or the multispecific antibody according to the second aspect of the present disclosure.

Preparation methods for the nucleic acid molecule described in the present disclosure are conventional in the art and preferably include the following preparation methods: obtaining a nucleic acid molecule encoding the above monoclonal antibody by gene cloning technology such as PCR, or obtaining a nucleic acid molecule encoding the above monoclonal antibody by artificial complete sequence synthesis.

It is known to those skilled in the art that substitutions, deletions, alterations, insertions, or additions may be appropriately introduced into the nucleic acid molecule encoding the amino acid sequence of the above antibody that binds to human ROR1 to provide a polynucleotide homolog. The polynucleotide homolog of the present disclosure may be produced by substituting, deleting, or adding one or more bases of the gene encoding the antibody that binds to human ROR1 within a range in which the activity of the antibody is maintained.

In a fourth aspect, the present disclosure provides a vector comprising the nucleic acid molecule according to the third aspect of the present disclosure.

In some embodiments, the vector is an expression vector; in some embodiments, the expression vector includes eukaryotic expression vectors, prokaryotic expression vectors, and viral vectors. In one embodiment, the expression vector is a conventional expression vector in the art and refers to an expression vector comprising an appropriate regulatory sequence, such as a promoter sequence, a terminator sequence, a polyadenylation sequence, an enhancer sequence, or a marker gene and/or sequence, and other appropriate sequences. In some embodiments, the expression vector may be a virus or a plasmid, such as an appropriate phage or phagemid. For more technical details, see, e.g., Sambrook et al., Molecular Cloning: A Laboratory Manual, Second Edition, Cold Spring Harbor Laboratory Press, 1989. Many known techniques and protocols for nucleic acid manipulation can be found in Current Protocols in Molecular Biology, Second Edition, Eds. Ausubel et al.

In a fifth aspect, the present disclosure provides a host cell comprising the nucleic acid molecule according to the third aspect of the present disclosure or the vector according to the fourth aspect of the present disclosure.

In some embodiments, the host cell includes eukaryotic cells and prokaryotic cells. In some embodiments, the eukaryotic cells are mammalian cells. In some embodiments, the host cell is a COS, CHO (Chinese hamster ovary), NS0, sf9, sf21, DH5$\alpha$, BL21(DE3), or TG1 cell.

In some embodiments, the host cell is an immune cell, such as a T cell or an NK cell.

In a sixth aspect, the present disclosure provides an antibody-drug conjugate represented by the following formula or a pharmaceutically acceptable salt thereof:

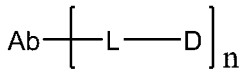

wherein:

Ab is an antibody comprising the anti-ROR1 antibody according to the first aspect of the present disclosure or the multispecific antibody according to the second aspect of the present disclosure;

L is a linker;

D is a drug;

n is an integer or decimal from 1 to 10.

In some embodiments, the L has a structure of $-L^a-L^b-L^c-L^d-$, wherein $L^a$ is attached to the antibody, and $L^d$ is attached to the drug, wherein:

$L^a$ is selected from the group consisting of

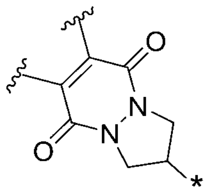, 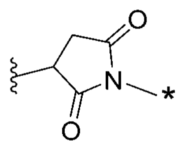,

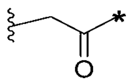, and 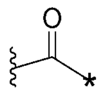,

wherein the wavy line ~ represents a point of attachment to Ab, and * represents a point of attachment to $L^b$;
$L^b$ is selected from the group consisting of $-(CH_2)m-C(O)-$, $-NH-(CH_2-CH_2-O)p-(CH_2)s-C(O)-$, $-C(O)-NH-(CH_2)q-C(O)-$, $-NH-(CH_2)r-C(O)-$, and a bond, wherein:

m is an integer from 0 to 10 (including 0, 1, 2, 3, 4, 5, 6, 7, 8, 9, and 10, or any interval); preferably, m is 0, 2, 3, or 5; p is an integer from 1 to 8 (including 1, 2, 3, 4, 5, 6, 7, and 8, or any interval), and s is an integer from 0 to 6 (including 0, 1, 2, 3, 4, 5, and 6, or any interval); preferably, p is 4, and s is 2;

q is an integer from 1 to 5 (including 1, 2, 3, 4, and 5, or any interval); preferably, q is 2;

r is an integer from 1 to 5 (including 1, 2, 3, 4, and 5, or any interval); preferably, r is 2;

$L^c$ is a peptide residue composed of 1 to 7 (including 1, 2, 3, 4, 5, 6, and 7) amino acids, or is a bond, wherein the amino acids include valine, citrulline, glycine, phenylalanine, alanine, proline, isoleucine, lysine, serine, glutamic acid, and aspartic acid; the amino acids are unsubstituted or are each independently substituted with one or more substituents, wherein each of the substituents is independently selected from the group consisting of halogen, hydroxyl, cyano, amino, $C_{1-6}$ alkyl, $C_{1-6}$ haloalkyl, $C_{1-6}$ alkoxy, and $C_{3-7}$ cycloalkyl;

$L^d$ is selected from the group consisting of $-NH-CH_2-O-CH_2-C(O)-$, $-NH-R^a-CH_2-O-C(O)-$, and a bond, wherein $R^a$ is phenyl or 5-6 membered heterocyclyl, and the phenyl and 5-6 membered heterocyclyl are unsubstituted or are each independently substituted with one or more substituents, wherein each of the substituents is independently

selected from the group consisting of

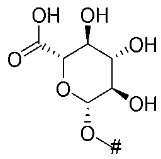,

halogen, oxo, hydroxyl, cyano, amino, $C_{1-6}$ alkyl, $C_{1-6}$ haloalkyl, and $C_{1-6}$ alkoxy, wherein # represents a point of attachment to the phenyl or 5-6 membered heterocyclyl.

In some embodiments, the L has a structure selected from the group consisting of:

i) $L^a$ is

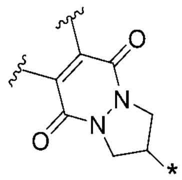,

$L^b$ is -C(O)- or -C(O)-NH-$(CH_2)_2$-C(O)-, $L^c$ is -glycine-glycine-phenylalanine-glycine-, -valine-citrulline-, -glycine-, or a bond, and $L^d$ is

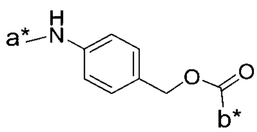, 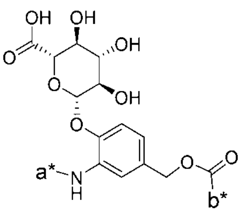,

-NH-$CH_2$-O-$CH_2$-C(O)-, or a bond; or

ii) $L^a$ is

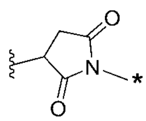,

and $L^b$ is -$(CH_2)$m-C(O)-, wherein m is 2 or 5, $L^c$ is -valine-citrulline- or -glycine-, and $L^d$ is

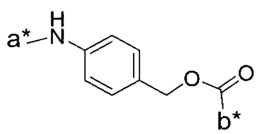 or 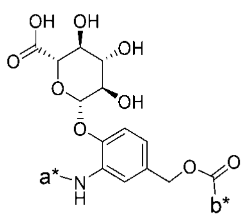;

or

iii) $L^a$ is

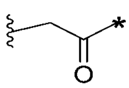,

$L^b$ is $-NH-(CH_2-CH_2-O)_4-(CH_2)_2-C(O)-$, $-NH-(CH_2)_2-C(O)-$, or a bond, $L^c$ is -valine-citrulline-, -glycine-, -glycine-glycine-phenylalanine-glycine-, or a bond, and $L^d$ is

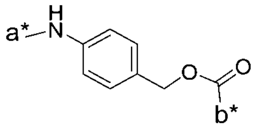,
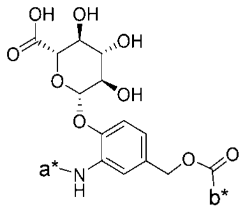,

or $-NH-CH_2-O-CH_2-C(O)-$; and

iii) $L^a$ is

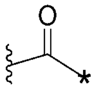,

$L^b$ is $-(CH_2)_3-C(O)-$, L° is -valine-citrulline-, and $L^d$ is

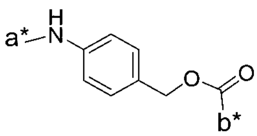;

wherein the wavy line ~ represents a point of attachment to Ab, * represents a point of attachment to $L^b$, a* represents a point of attachment to $L^c$, and b* represents a point of attachment to the drug.

In some embodiments, L has the following structure:

$L^a$ is

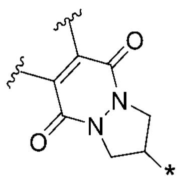,

$L^b$ is -C(O)-, $L^c$ is -valine-citrulline-, and $L^d$ is

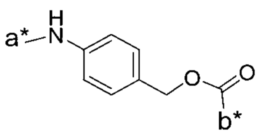.

In some embodiments, L has the following structure:

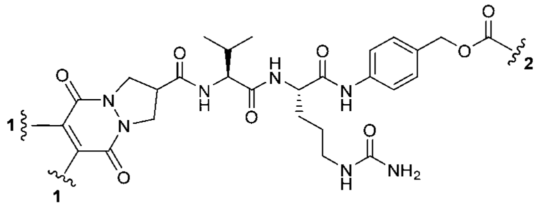,
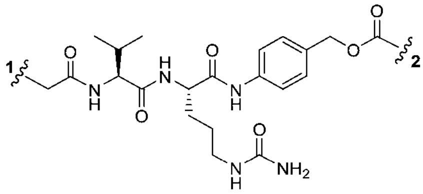,

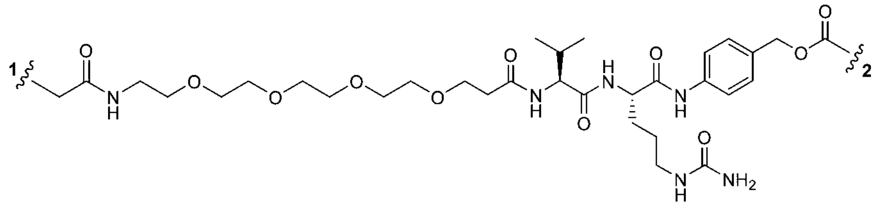

,

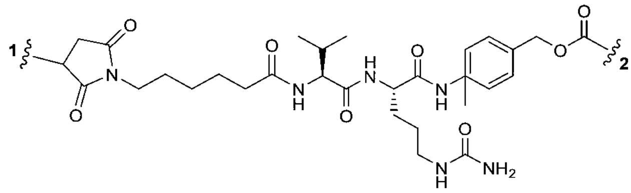

,

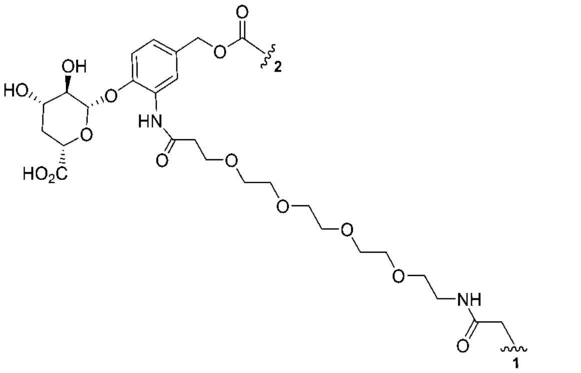

,

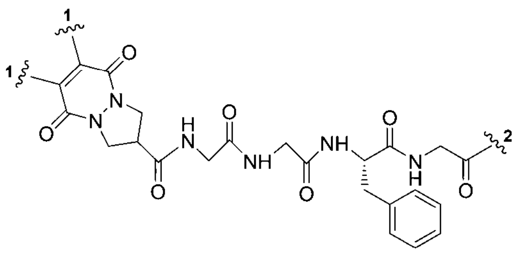

,

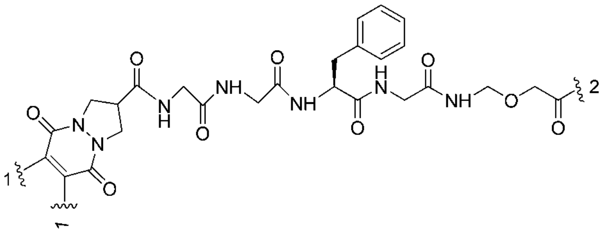

,

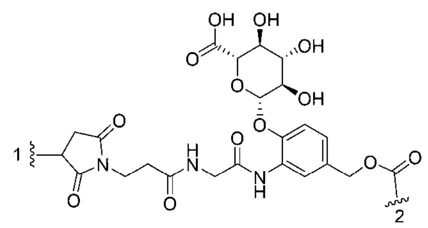

,

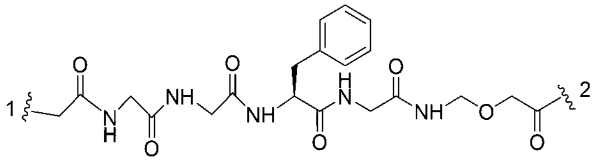

,

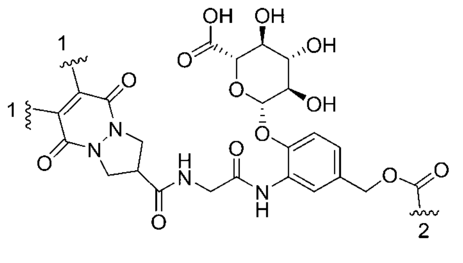

,

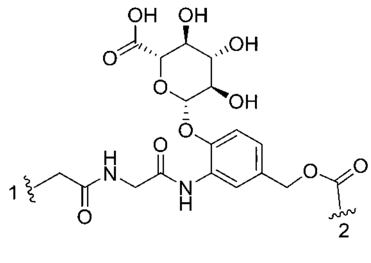

,

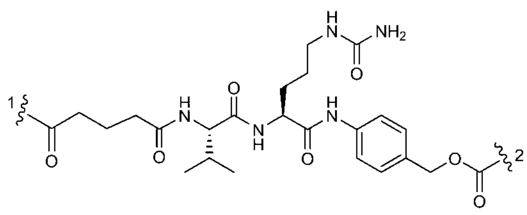

,

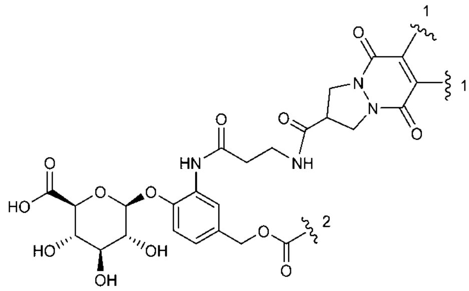

,

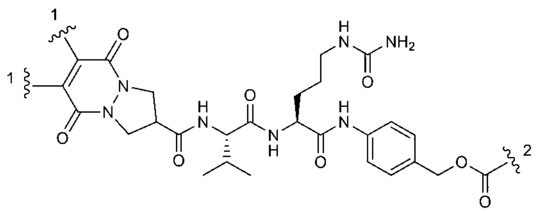

,

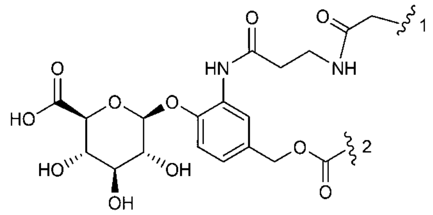

,

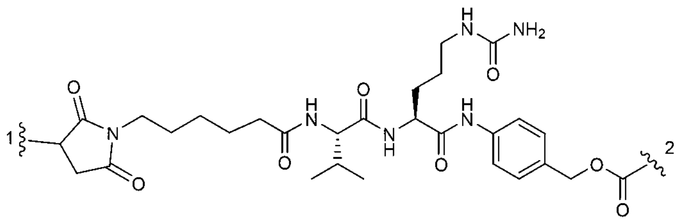

,

or

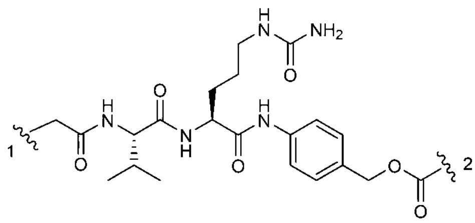

,

wherein 1 represents a point of attachment to antibody Ab, and 2 represents a point of attachment to toxin D.

In some embodiments, the drug is selected from the group consisting of a cytotoxic compound, an immunomodulator, an enzyme, and a hormone inhibitor.

In some embodiments, the drug is selected from the group consisting of monomethyl auristatin E (MMAE), monomethyl auristatin F (MMAF), eribulin, exatecan, maytansine, SN-38, and a combination thereof.

In some embodiments, the drug is eribulin.

In some embodiments, the antibody-drug conjugate has the following structure:

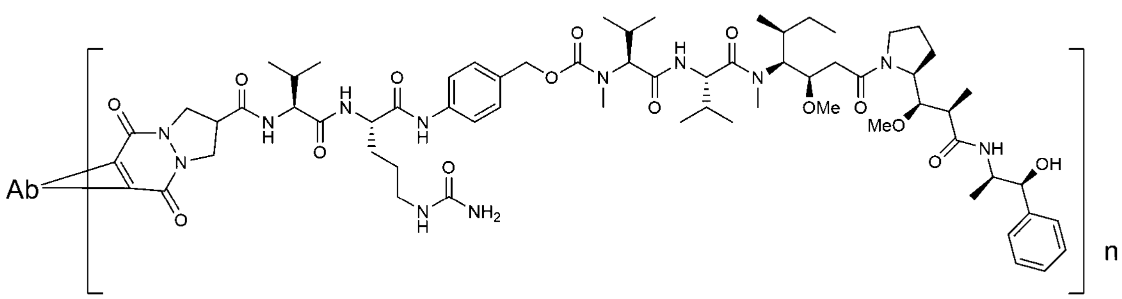

,

or

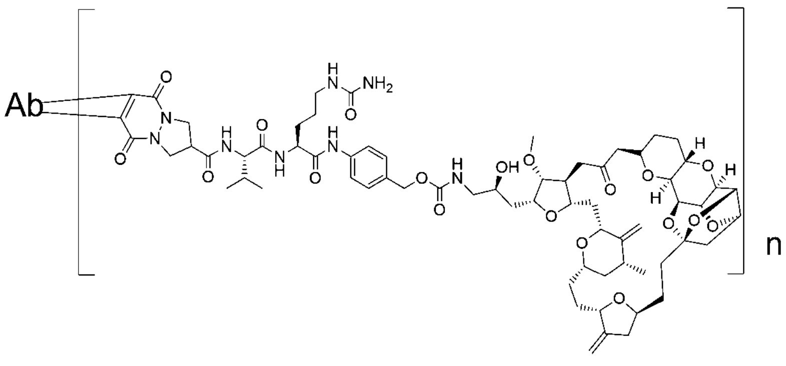

,

or

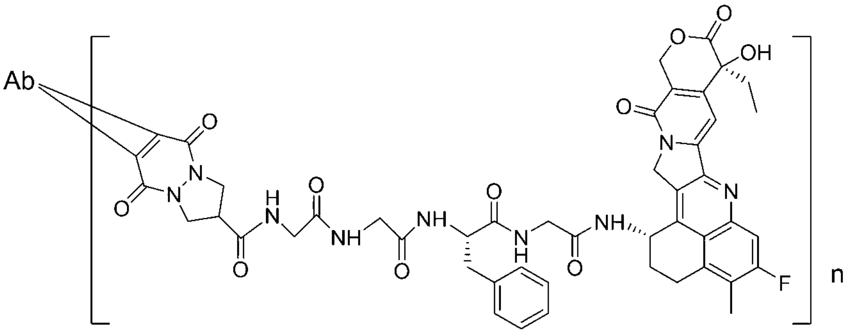

.

In some embodiments, Ab comprises a heavy chain set forth in SEQ ID NO: 77 and a light chain set forth in SEQ ID NO: 78, or Ab comprises the following four polypeptide chains:

a chain 1 comprising the amino acid sequence set forth in SEQ ID NO: 81;

a chain 2 comprising the amino acid sequence set forth in SEQ ID NO: 82;

a chain 3 comprising the amino acid sequence set forth in SEQ ID NO: 83; and

a chain 4 comprising the amino acid sequence set forth in SEQ ID NO: 84;

n is 3.5-4.5.

In a seventh aspect, the present disclosure provides a pharmaceutical composition comprising: (a) the anti-ROR1 antibody according to the first aspect of the present disclosure, or the multispecific antibody according to the second aspect of the present disclosure, or the antibody-drug conjugate or the pharmaceutically acceptable salt thereof according to the sixth aspect of the present disclosure; and (b) a pharmaceutically acceptable carrier.

In an eighth aspect, the present disclosure provides a method for preparing the antibody-drug conjugate or the pharmaceutically acceptable salt thereof according to the sixth aspect of the present disclosure, and the method comprises the following steps:

(1) providing the anti-ROR1 antibody according to the first aspect of the present disclosure or the multispecific antibody according to the second aspect of the present disclosure as an antibody moiety; and

(2) conjugating the antibody moiety in step (1) to a linker-drug compound to obtain the antibody-drug conjugate.

In some embodiments, the linker-drug compound is selected from the group consisting of the LD-1 to LD-17 described in the present disclosure.

In some embodiments, the linker-drug compound is selected from the group consisting of:

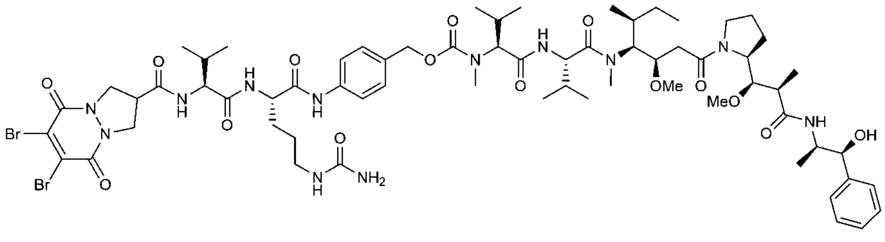

(LD-1),

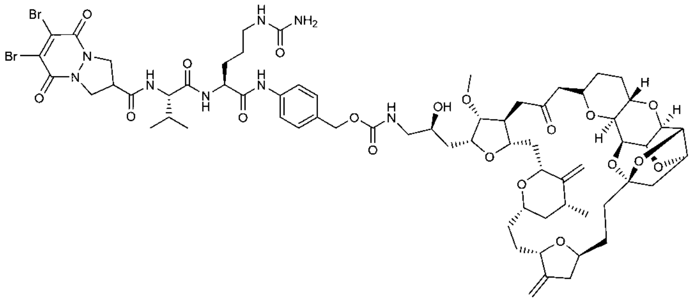

(LD-14),

and

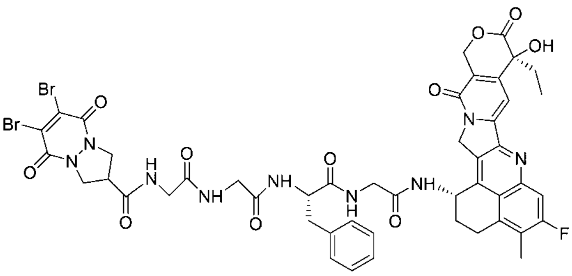

(LD-6).

In a ninth aspect, the present disclosure provides use of the anti-ROR1 antibody according to the first aspect of the present disclosure, or the multispecific antibody according to the second aspect of the present disclosure, or the antibody-drug conjugate or the pharmaceutically acceptable salt thereof according to the sixth aspect of the present disclosure, or the pharmaceutical composition according to the seventh aspect of the present disclosure in the preparation of a medicament for treating a tumor or cancer.

In a tenth aspect, the present disclosure provides a method for treating a tumor or cancer, and the method comprises administering to a subject in need thereof the anti-ROR1 antibody according to the first aspect of the present disclosure, or the multispecific antibody according to the second aspect of the present disclosure, or the antibody-drug conjugate or the pharmaceutically acceptable salt thereof according to the sixth aspect of the present disclosure, or the pharmaceutical composition according to the seventh aspect of the present disclosure.

In some embodiments, the subject in need thereof includes humans or non-human mammals.

In an eleventh aspect, the present disclosure provides the anti-ROR1 antibody according to the first aspect of the present disclosure, or the multispecific antibody according to the second aspect of the present disclosure, or the antibody-drug conjugate or the pharmaceutically acceptable salt thereof according to the sixth aspect of the present disclosure, or the pharmaceutical composition according to the seventh aspect of the present disclosure for use as a medicament and, in some embodiments, for use as a medicament for treating a tumor or cancer. In some embodiments, the tumor or cancer is a tumor or cancer expressing an ROR1 protein.

In a twelfth aspect, the present disclosure provides use of the anti-ROR1 antibody according to the first aspect of the present disclosure, or the multispecific antibody according to the second aspect of the present disclosure, or the antibody-drug conjugate or the pharmaceutically acceptable salt thereof according to the sixth aspect of the present disclosure, or the pharmaceutical composition according to the seventh aspect of the present disclosure as a medicament (i.e., use for treatment).

In some embodiments, the tumor or cancer includes solid tumors and hematological tumors.

In some embodiments, the tumor or cancer is selected from the group consisting of breast cancer, pancreatic cancer, lung cancer, esophageal cancer, non-small cell lung cancer, laryngeal tumors, sarcoma, pharyngeal tumors, oral

tumors, gastric cancer, ovarian cancer, prostate cancer, bladder cancer, colon cancer, colorectal cancer, urothelial cancer, cervical cancer, lymphoma, and leukemia.

In some embodiments, the hematological tumors include B cell chronic lymphocytic leukemia (CLL), acute lymphoblastic leukemia (ALL), non-Hodgkin lymphoma (NHL), and myeloid hematological cancer.

In some embodiments, the leukemia includes B cell chronic lymphocytic leukemia (CLL), acute lymphoblastic leukemia (ALL), and non-Hodgkin lymphoma (NHL).

In some embodiments, the tumor or cancer is selected from the group consisting of breast cancer and hematological tumors.

In some embodiments, the lymphoma and leukemia include various subtypes of pre-B cell acute lymphoblastic leukemia (B-ALL), B cell chronic lymphocytic leukemia (CLL), and non-Hodgkin lymphoma (NHL), including diffuse large B cell lymphoma (DLBCL), follicular lymphoma (FL), mantle cell lymphoma (MCL), and marginal zone lymphoma (MZL).

It will be appreciated that within the scope of the present disclosure, the above technical features of the present disclosure and those specifically described below (e.g., in the examples) can be combined with one another to form new or preferred technical solutions. Due to limited space, such technical solutions are not described herein.

## BRIEF DESCRIPTION OF THE DRAWINGS

FIG. 1 shows the tumor inhibition activity of ADCs in an SCID female mouse model of HCC1187 breast cancer.

FIG. 2 shows the tumor inhibition activity of ADCs in an SCID female mouse model of Jeko-1 mantle cell lymphoma.

FIG. 3 shows the tumor inhibition activity of ADCs in a graft mouse model of the ovarian cancer cell line PA-1.

## DETAILED DESCRIPTION

Through extensive and in-depth studies, the present disclosure designs and constructs a range of anti-ROR1 antibodies with brand-new CDR sequences that specifically bind to human ROR1 protein with high affinity. The anti-ROR1 antibodies can specifically bind to an epitope located at amino acid positions 130-165 of the ROR1 protein or an epitope located at amino acid positions 70-130 of the ROR1 protein. Based on the anti-ROR1 antibodies prepared by the present disclosure, multispecific antibodies that bind to antigens, including the ROR1 protein, can be prepared. Specifically, the present disclosure provides a biepitopic antibody that targets the ROR1 protein, and the antibody has a much higher ability to recognize the ROR1 antigen than monoclonal antibodies. The present disclosure further constructs an anti-ROR1 monoclonal antibody-drug conjugate and a drug conjugate of the biepitopic antibody that targets the ROR1 protein. *In vivo* and *in vitro* experiments show that the antibody-drug conjugates of the present disclosure exhibited significant tumor inhibition effects on cell lines derived from a variety of cancers or tumors (e.g., breast cancer, ovarian cancer, and lymphocytic cancer) and in tumor graft mouse models, and the effects were superior to that of the positive control, which indicates that the antibody-drug conjugates of the present disclosure can be used as therapeutic drug for a variety of solid tumors and hematological tumors for the treatment of tumors or cancers.

The present disclosure is achieved on this basis.

### Terminology

For a better understanding of the present disclosure, the following terms are defined.

Unless otherwise stated, all singular terms also encompass the plural, active tense, and past tense forms of a term.

Unless otherwise clearly indicated in the context., the term "about" includes values that are within a standard deviation of the stated value.

Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to". Unless otherwise stated, "comprise" includes "consist of". For example, an HCDR1 comprising the amino acid sequence of SEQ ID NO: 45 explicitly encompasses an HCDR1 having an amino acid sequence as set forth in SEQ ID NO: 45.

The "subject" or "patient" according to the present disclosure is an animal, including a human patient in need of anti-cancer treatment or therapy. In certain aspects, the present disclosure may also be applied in veterinary practice to any mammal or other animals in need of such ROR1-targeted anti-cancer treatment. The term may include, e.g., non-human primates, canines, felines, porcines, equines, and any other animals in need of ROR1-targeted anti-cancer

treatment.

The term "antibody" is used in the broadest sense and encompasses a variety of antibody structures, including but not limited to monoclonal antibodies, polyclonal antibodies, monospecific antibodies, multispecific antibodies (e.g., bispecific antibodies), full-length antibodies, and antibody fragments (or antigen-binding fragments, or antigen-binding moieties), as long as they exhibit the desired antigen-binding activity. For example, a native IgG antibody is a heterotetrameric glycoprotein of about 150,000 Daltons composed of two identical light chains and two identical heavy chains combined by disulfide bonds. From the N-terminus to the C-terminus, each heavy chain comprises one variable region (VH), also known as variable heavy domain or heavy chain variable region, followed by three constant domains (CH1, CH2, and CH3). Similarly, from the N-terminus to the C-terminus, each light chain comprises one variable region (VL), also known as variable light domain or light chain variable domain, followed by one constant light domain (light chain constant region, CL). Light chains are classified as $\kappa$ or $\lambda$. Heavy chains are classified as $\gamma$, $\mu$, $\alpha$, $\delta$, or $\varepsilon$, and isotypes of antibodies are defined as IgG, IgM, IgA, IgD, and IgE. In the light and heavy chains, the variable region and the constant region are linked by a "J" region of about 12 or more amino acids, and the heavy chain also comprises a "D" region of about 10 or more amino acids. (See generally, Fundamental Immunology, Paul, W., ed., 2nd ed. Raven Press, N.Y., 1989, Ch. 7, which is incorporated by reference in its entirety for all purposes).

The term "antibody fragment" refers to a molecule different from an intact antibody, which comprises a moiety of an intact antibody that retains the antigen-binding ability of the intact antibody. Examples of antibody fragments include, but are not limited to, Fv, Fab, Fab', Fab'-SH, $F(ab')_2$, single-domain antibodies, single-chain Fab (scFab), diabodies, linear antibodies, and single-chain antibody molecules (e.g., scFv), wherein: (i) the Fab fragment has VL, CL, VH, and CH1 domains having one disulfide bond between the heavy chain and the light chain; (ii) the Fab' fragment is a Fab fragment that has one or more cysteine residues at the C-terminus of the CH1 domain; (iii) an Fd fragment has VH and CH1 domains; (iv) an Fd' fragment has VH and CH1 domains and has one or more cysteine residues at the C-terminus of the CH1 domain; (v) the Fv fragment has the VL and VH domains of one arm of an antibody; (vi) a dAb fragment consists of a VH domain; (vii) hingeless antibodies comprise at least VL, VH, CL, and CH1 domains and lack a hinge region; (viii) a $F(ab)_2$ fragment is a bivalent fragment comprising two Fab' fragments linked by a disulfide bridge in the hinge region; (ix) single-chain antibody molecules (e.g., single-chain Fv; scFv); (x) "diabodies" have two antigen-binding sites and comprise a heavy chain variable domain (VH) and a light chain variable domain (VL) that are linked to each other in the same polypeptide chain; (xi) "linear antibodies" comprise a pair of tandem Fd fragments (VH-CH1-VH-CH1) that, together with complementary light chain polypeptides, form a pair of antigen-binding regions; (xii) dsFv refers to a fragment formed by combining polypeptides obtained by substituting one amino acid residue in each of VH and VL with a cysteine residue, via the S-S bond between the cysteine residues.

Monoclonal antibodies generally require isolation and purification. This means that the purity of the antibody is generally at least 50% with respect to interfering proteins and other contaminants generated during the production or purification of the antibody, but does not exclude the possibility of binding the monoclonal antibody to an excess of pharmaceutically acceptable carriers or other carriers intended to facilitate its use. Sometimes, the monoclonal antibody has a purity of at least 60%, 70%, 80%, 90%, 95%, or 99% w/w with respect to the interfering proteins and contaminants during production or purification.

The term "bispecific antibody" refers to an antibody (including an antibody or an antigen-binding fragment thereof, such as a single-chain antibody) capable of specifically binding to two different antigens or at least two different epitopes of the same antigen. Bispecific antibodies of various structures have been disclosed in the prior art; the bispecific antibodies can be classified into IgG-like bispecific antibodies and antibody-fragment-type bispecific antibodies according to the integrity of IgG molecules; the bispecific antibodies can be classified into bivalent, trivalent, tetravalent or higher-valent bispecific antibodies according to the number of the antigen-binding regions; the bispecific antibodies can be classified into symmetric bispecific antibodies and asymmetric bispecific antibodies according to the presence of symmetry in their structures. Bispecific antibodies based on antibody fragments such as Fab fragments, which lack an Fc fragment; 2 or more Fab fragments are combined in one molecule to form a bispecific antibody; they have relatively low immunogenicity, small molecular weight, and relatively high tumor tissue permeability; typical antibody structures of this type are, e.g., $F(ab)_2$, scFv-Fab, and $(scFv)_2$-Fab. IgG-like bispecific antibodies (e.g., having an Fc fragment) have relatively large relative molecular weight, and the Fc fragment facilitates the purification of the antibodies and increase their solubility and stability; the Fc fragment may also bind to the receptor FcRn, increasing the serum half-life of the antibodies; typical structural models of bispecific antibodies are, e.g., KiH, CrossMAb, Triomab quadroma, Fc$\Delta$Adp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP- DART, CODV-Fab-TL, HLE-BiTE, $F(ab)_2$-CrossMAb, IgG-$(scFv)_2$, Bs4Ab, DVD-Ig, tetravalent-DART-Fc, $(scFv)_4$-Fc, CODV-Ig, $mAb_2$, and $F(ab)_4$-CrossMAb (see Aran F. Labrijn et al., Nature Reviews Drug Discovery volume 18, pages 585-608 (2019); Chen S1 et al., J Immunol Res. 2019 Feb 11; 2019:4516041).

The specific binding of an antibody to its target antigen means there is an affinity of at least $10^6$, $10^7$, $10^8$, $10^9$, or $10^{10}$ $M^{-1}$. Specific binding is detectably higher in magnitude and different from non-specific binding occurring to at least one unrelated target. Specific binding may be the result of forming bonds between specific functional groups or specific spatial

matches (e.g., lock and key type), while nonspecific binding is typically the result of van der Waals forces. However, specific binding does not necessarily mean that the monoclonal antibody binds to one and only one target.

The term "variable region" or "variable domain" refers to a domain in an antigen-binding molecule that binds to an antigen. The VH and VL each comprise four conserved framework regions (FRs) and three complementarity-determining regions (CDRs). The term "complementarity determining region" or "CDR" refers to a region in the variable domain that primarily contributes to antigen binding; "framework" or "FR" refers to variable domain residues other than CDR residues. A VH comprises 3 CDRs: HCDR1, HCDR2, and HCDR3; a VL comprises 3 CDRs: LCDR1, LCDR2, and LCDR3. Each VH and each VL are, from the N-terminus to the C-terminus: FR1, CDR1, FR2, CDR2, FR3, CDR3, FR4. A single VH or VL may be sufficient to provide antigen binding specificity.

The boundaries of the amino acid sequences of CDRs can be determined by a variety of well-known schemes, such as the "Kabat" numbering scheme (see Kabat et al. (1991), "Sequences of Proteins of Immunological Interest", 5th Edition, Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), and the ImMunoGenTics (IMGT) numbering scheme (Lefranc, M.P. et al., Dev. Comp. Immunol., 27, 55-77 (2003); Front Immunol. 2018 Oct 16; 9:2278); the corresponding relationships between various numbering systems are well known to those skilled in the art. Exemplary numbering schemes of the present disclosure are shown in the table below.

The relationships between CDR numbering systems

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

It will be appreciated by those skilled in the art that, even under a certain particular numbering scheme, the numbering of the positions of the CDRs of a certain particular antibody may differ by several amino acids; for example, for some antibodies, the position of LCDR1 is 24-33, and the position of HCDR1 is 98-106, under the Kabat scheme. Unless otherwise stated, the "Kabat" numbering scheme applies to the variable region and CDR sequences in the examples of the present disclosure. Although one numbering system (e.g., Kabat) is employed to define amino acid residues in specific embodiments, technical solutions corresponding to other numbering systems are considered equivalent technical solutions.

The term "Fc region" or "fragment crystallizable region" is used to define the C-terminal region of the heavy chain of an antibody, including native Fc regions and engineered Fc regions. In some embodiments, the Fc region comprises two identical or different subunits. In some embodiments, the Fc region of a human IgG heavy chain is defined as extending from the amino acid residue at position Cys226 or from Pro230 to its carboxy-terminus. Suitable native sequence Fc regions for the antibodies described herein include human IgG1, IgG2 (IgG2A and IgG2B), IgG3, and IgG4. Unless otherwise stated, the numbering scheme for the Fc region is the EU index. The C-terminus of the Fc region may be an intact C-terminus ending with amino acid residues PGK or a truncated C-terminus, e.g., a truncated C-terminus in which one or two C-terminal amino acid residues are removed. In one preferred aspect, the C-terminus of the heavy chain is a shortened C-terminus ending with PG. Thus, in some embodiments, a composition of intact antibodies may comprise antibody populations with all K447 residues and/or G446 + K447 residues removed. In some embodiments, a composition of intact antibodies may comprise antibody populations with K447 residues and/or G446 + K447 residues unremoved. In some embodiments, a composition of intact antibodies comprises antibody populations having a mixture of antibodies with and without a K447 residue and/or G446 + K447 residues.

In some embodiments, the Fc region of the present disclosure comprises engineering according to the knob-into-hole (KIH) technique, which involves introducing a knob structure at the interface of the first subunit and a hole structure at the interface of the second subunit. Thus, the knob structure can be positioned in the hole structure, promoting the formation of heterodimers and inhibiting the production of homodimers. The knob structure is constructed by substituting a small amino acid side chain at the interface of the first subunit with a larger side chain (e.g., tyrosine or tryptophan). The hole structure is created at the interface of the second subunit by substituting a large amino acid side chain with a smaller amino acid side chain (e.g., alanine or threonine). The knob structure and the hole structure are prepared by altering the

nucleic acid encoding the polypeptide. Optional amino acid substitutions are shown in the table below:

KIH mutation combinations

| First subunit | T366Y | T366W | T394W | F405W | T366W | T366Y F405A | T366W F405W | F405W Y407A |
|---|---|---|---|---|---|---|---|---|
| Second subunit | Y407T | Y407A | F405A | T394S | T366S L368A Y407V | T394W Y407T | T394W Y407A | T366W T394S |

In addition to the knob-into-hole technique, other techniques for modifying the CH3 domain of the heavy chain to achieve heterodimerization are also known in the art, e.g., the techniques described in WO1996027011A1, WO1998050431, EP1870459, WO2007110205, WO2009089004, WO2010129304, WO201190754, WO2011143545, WO2012058768, WO2013157954, and WO2013096291.

The term "epitope" refers to a site on an antigen to which an antibody binds. An epitope can be formed from contiguous amino acids or non-contiguous amino acids by tertiary folding of one or more proteins. Epitopes formed from contiguous amino acids are typically retained in denaturing solvents, whereas epitopes formed by tertiary folding are typically lost in denaturing solvents. An epitope typically includes at least 3, and more generally at least 5 or 8-10 amino acids forming a unique spatial conformation. Methods for determining spatial conformation of an epitope include, for example, X-ray crystallography and 2-dimensional nuclear magnetic resonance. See, e.g., Epitope Mapping Protocols, in Methods in Molecular Biology, Vol. 66, Glenn E. Morris, Ed. (1996).

Antibodies that recognize the same or overlapping epitopes can be detected by a simple immunoassay to detect the ability of one antibody to compete with another antibody for binding to a target antigen. The epitope where an antibody binds to an antigen can also be determined by X-ray crystallography to determine interacting residues. Alternatively, two antibodies have the same epitope if all amino acid mutations in the antigen that cause reduced or eliminated binding of one antibody also reduce or eliminate binding of the other. Two antibodies have overlapping epitopes if some amino acid mutations in the antigen that cause reduced or eliminated binding of one antibody reduce or eliminate binding of the other.

Competition between antibodies can be determined by an assay in which a test antibody inhibits specific binding of a reference antibody to a common antigen (e.g., Junghans et al., Cancer Res. 50:1495, 1990). In a competitive binding assay, a test antibody competes with a reference antibody if the test antibody is in excess of the reference antibody (e.g., at least 2-fold, 5-fold, 10-fold, 20-fold, or 100-fold) and inhibits binding of the reference antibody by at least 50% but preferably 75%, 90%, or 99% in the competitive binding assay. Antibodies that can be identified by the competition assay (competing antibodies) include antibodies binding to the same epitope as the reference antibody, and antibodies binding to an adjacent epitope as the reference antibody.

To distinguish amino acid substitutions as being conservative or non-conservative, amino acids are classified into: class I (hydrophobic side chain): Met, Ala, Val, Leu, and Ile; class II (neutral hydrophilic side chain): Cys, Ser, and Thr; class III (acidic side chain): Asp and Glu; class IV (basic side chain): Asn, Gln, His, Lys, and Arg; class V (residues that influence chain orientation): Gly and Pro; and class VI (aromatic side chain): Trp, Tyr, and Phe. Conservative substitutions include substitutions between amino acids of the same class. Non-conservative replacements are substitutions of different classes of amino acids.

The term "sequence identity" refers to the degree (percentage) to which the amino acids/nucleic acids of two sequences are identical at equivalent positions when the two sequences are optimally aligned. In the alignment process, gaps may be allowed to be introduced, if necessary, to achieve the maximum percent sequence identity, but any conservative substitution is not considered a part that constitutes sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

Antibody effector functions refer to functions produced by the Fc domain of an Ig. For example, these functions may be antibody-dependent cytotoxicity, antibody-dependent cellular phagocytosis, or complement-dependent cytotoxicity. For example, binding of an Fc effector domain to an Fc receptor on an immune cell with phagocytic or lytic activity, or binding of the Fc effector domain to a component of a complement system, can result in an effector function. Generally, effects mediated by Fc-binding cells or complement components result in growth inhibition and/or apoptosis of target cells expressing ROR1. The Fc region of the antibody can recruit Fc receptor (FcR)-expressing cells and juxtapose them with

target cells that bind to the antibody. Cells expressing FcRs, including FcγRIII (CD16), FcγRII (CD32), and FcγRIII (CD64) on the membrane, can be used as effector cells for killing IgG-bound cells. These effector cells include monocytes, macrophages, natural killer cells, neutrophils, and eosinophils. Engagement of FcγR by IgG can activate antibody-dependent cellular cytotoxicity (ADCC) or antibody-dependent cellular phagocytosis (ADCP). ADCC is mediated by the CD16.sup.+ effector cells through secretion of membrane pore-forming proteins and proteases, while phagocytosis is mediated by CD32.sup.+ and CD64.sup.+ effector cells (see Fundamental Immunology, 4th Edition, Paul ed., Lippincott-Raven, New York, 1997, Chapters 3, 17, and 30; Uchida et al., 2004, J. Exp. Med. 199:1659-69; Akewanlop et al., 2001, Cancer Res. 61:4061-65; Watanabe et al., 1999, Breast Cancer Res. Treat. 53:199-207). In addition to ADCC and ADCP, the Fc region of a cell-bound antibody can also activate the complement classical pathway to elicit complement-dependent cytotoxicity (CDC). When an antibody forms a complex with an antigen, C1q of the complement system binds to the Fc region of the antibody. When C1q binds to a cell-bound antibody, a cascade of reactions, including proteolytic activation of C4 and C2, can be initiated to produce C3 convertase. Cleavage of C3 to C3b by the C3 convertase can activate terminal complement components, including C5b, C6, C7, C8, and C9. In general, these proteins form membrane-attack complex pores on antibody-coated cells. These pores disrupt the integrity of the cell membrane, thereby killing target cells (see Immunobiology, 6th Edition, Janeway et al., Garland Science, New York, 2005, Chapter 2).

The term "antibody-dependent cytotoxicity" or ADCC is a mechanism for inducing cell death, which depends on the interaction of antibody-coated target cells with immune cells having lytic activity (also known as effector cells). These effector cells include natural killer cells, monocytes/macrophages, and neutrophils. The effector cells are attached to the Fc effector domain of the Ig, while IgG binds to target cells through the antigen-binding site. The activity of effector cells results in the death of antibody-coated target cells.

The term "antibody-dependent cellular phagocytosis" or ADCP refers to the process of total or partial internalization of antibody-coated cells by phagocytic immune cells (e.g., macrophages, neutrophils, and dendritic cells) that bind to the Fc effector domain of the Ig.

The term "complement-dependent cytotoxicity" or CDC refers to a mechanism for inducing cell death in which the Fc effector domain of an antibody that binds to a target cell activates a series of enzymatic reactions, eventually forming pores in the target cell membrane. Generally, an antigen-antibody complex, such as an antigen-antibody complex formed by coating a target cell with an antibody, binds to and activates the complement component C1q, thereby activating the complement cascade reaction and causing the death of the target cell. Activation of a complement may also result in the deposition of complement components on the surface of target cells, which promote ADCC by binding to complement receptors (e.g., CR3) on leukocytes.

"Cytotoxic effect" refers to the depletion, elimination, and/or killing of target cells. "Cytotoxic formulation" refers to a formulation that has a cytotoxic effect on cells. The cytotoxic formulation may be conjugated to an antibody or administered in combination with the antibody.

"Cytostatic effect" refers to the inhibition of cell proliferation. "Cytostatic agent" refers to an agent that has a cytostatic effect on cells and thus inhibits the growth and/or expansion of a particular cell subpopulation. The cytostatic agent may be conjugated to an antibody or administered in combination with the antibody.

The term "pharmaceutically acceptable" means approved or approvable by a regulatory agency or listed in a pharmacopeia or other commonly-recognized pharmacopeias for use in animals, particularly in humans. The term "pharmaceutically compatible ingredient" refers to a pharmaceutically acceptable diluent, adjuvant, excipient, or carrier combined with the anti-ROR1 antibody or the anti-ROR1 antibody-drug conjugate.

The term "pharmaceutically acceptable salt" refers to a pharmaceutically acceptable organic or inorganic salt of the anti-ROR1 antibody or a conjugate thereof or a formulation for use in combination with the anti-ROR1 antibody. Exemplary salts include sulfate, citrate, acetate, oxalate, chloride, bromide, iodide, nitrate, bisulfate, phosphate, acidic phosphate, isonicotinate, lactate, salicylate, citrate, tartrate, oleate, tannate, pantothenate, bitartrate, ascorbate, succinate, maleate, gentisate, fumarate, gluconate, glucuronate, saccharate, formate, benzoate, glutamate, methanesulfonate, ethanesulfonate, benzenesulfonate, p-toluenesulfonate, and pamoate (i.e., 1,1'-methylenebis(2-hydroxy-3-naphthoate)). The pharmaceutically acceptable salt may comprise another molecule, such as an acetate ion, a succinate ion, or other counterions. The counterion may be any organic or inorganic moiety to stabilize the charge of the parent compound. In addition, the pharmaceutically acceptable salt may have more than one charged atom in its structure. Examples where a plurality of charged atoms are part of the pharmaceutically acceptable salt may have a plurality of counterions. Thus, the pharmaceutically acceptable salt may have one or more charged atoms and/or one or more counterions.

The terms "host cell", "host cell line", and "host cell culture" are used interchangeably and refer to cells into which exogenous nucleic acids have been introduced, including progeny of such cells. Host cells include "transformants" and "transformed cells", which include primary transformed cells and progeny derived therefrom, regardless of the number of passages. Progeny may not be completely identical to parent cells in terms of nucleic acid content and may contain mutations. As used herein, the term includes mutant progeny that have the same function or biological activity as the cells screened or selected from the primary transformed cells. Host cells include prokaryotic and eukaryotic host cells, wherein

the eukaryotic host cells include, but are not limited to, mammalian cells, insect cell lines, plant cells, and fungal cells. Mammalian host cells include human, mouse, rat, canine, monkey, porcine, goat, bovine, equine, and hamster cells, including but not limited to, Chinese hamster ovary (CHO) cells, NSO, SP2 cells, HeLa cells, baby hamster kidney (BHK) cells, monkey kidney cells (COS), human hepatocellular carcinoma cells (e.g., Hep G2), A549 cells, 3T3 cells, and HEK-293 cells. Fungal cells include yeast and filamentous fungal cells, including, for example, *Pichiapastoris, Pichia finlandica, Pichia trehalophila, Pichia koclamae, Pichia membranaefaciens, Pichia minuta (Ogataea minuta, Pichia lindneri), Pichiaopuntiae, Pichia thermotolerans, Pichia salictaria, Pichia guercuum, Pichia pijperi, Pichia stiptis, Pichia methanolica,* Pichia, *Saccharomycescerevisiae,* Saccharomyces, *Hansenula polymorpha,* Kluyveromyces, *Kluyveromyces lactis, Candida albicans, Aspergillus nidulans, Aspergillus niger, Aspergillus oryzae, Trichoderma reesei, Chrysosporium lucknowense, Fusarium* sp., *Fusarium gramineum, Fusarium venenatum, Physcomitrella patens, and Neurospora crassa.* Pichia, any Saccharomyces, *Hansenula polymorpha,* any Kluyveromyces, *Candida albicans,* any Aspergillus, *Trichoderma reesei, Chrysosporium lucknowense,* any Fusarium, *Yarrowia lipolytica,* and *Neurospora crassa.* The host cell of the present patent does not include objects not authorized by the Patent Laws.

In one aspect, the present disclosure provides an anti-ROR1 antibody.

In one embodiment of the present disclosure, the anti-ROR1 antibody targets an epitope located at amino acid positions 70-130 of an ROR1 protein (having an amino acid sequence as set forth in SEQ ID NO: 87);

specifically, the antibody comprises an HCDR1 set forth in SEQ ID NO: 61, an HCDR2 set forth in SEQ ID NO: 46, and an HCDR3 set forth in SEQ ID NO: 65, and comprises an LCDR1 set forth in SEQ ID NO: 63, an LCDR2 set forth in SEQ ID NO: 64, and an LCDR3 set forth in SEQ ID NO: 50;

preferably, the antibody comprises a heavy chain variable region of the amino acid sequence set forth in SEQ ID NO: 72 or a variant thereof and a light chain variable region of the amino acid sequence set forth in SEQ ID NO: 71 or a variant thereof;

alternatively, the antibody comprises an HCDR1 set forth in SEQ ID NO: 45, an HCDR2 set forth in SEQ ID NO: 46, and an HCDR3 set forth in SEQ ID NO: 66, and comprises an LCDR1 set forth in SEQ ID NO: 63, an LCDR2 set forth in SEQ ID NO: 64, and an LCDR3 set forth in SEQ ID NO: 50;

preferably, the antibody comprises a heavy chain variable region of the amino acid sequence set forth in SEQ ID NO: 73 or a variant thereof and a light chain variable region of the amino acid sequence set forth in SEQ ID NO: 71 or a variant thereof;

alternatively, the antibody comprises an HCDR1 set forth in SEQ ID NO: 45, an HCDR2 set forth in SEQ ID NO: 46, and an HCDR3 set forth in SEQ ID NO: 65, and comprises an LCDR1 set forth in SEQ ID NO: 67, an LCDR2 set forth in SEQ ID NO: 68, and an LCDR3 set forth in SEQ ID NO: 50;

preferably, the antibody comprises a heavy chain variable region of the amino acid sequence set forth in SEQ ID NO: 74 or a variant thereof and a light chain variable region of the amino acid sequence set forth in SEQ ID NO: 75 or a variant thereof;

alternatively, the antibody comprises an HCDR1 set forth in SEQ ID NO: 45, an HCDR2 set forth in SEQ ID NO: 46, and an HCDR3 set forth in SEQ ID NO: 65, and comprises an LCDR1 set forth in SEQ ID NO: 63, an LCDR2 set forth in SEQ ID NO: 69, and an LCDR3 set forth in SEQ ID NO: 50;

preferably, the antibody comprises a heavy chain variable region of the amino acid sequence set forth in SEQ ID NO: 74 or a variant thereof and a light chain variable region of the amino acid sequence set forth in SEQ ID NO: 76 or a variant thereof;

alternatively, the antibody comprises an HCDR1 set forth in SEQ ID NO: 45, an HCDR2 set forth in SEQ ID NO: 46, and an HCDR3 set forth in SEQ ID NO: 65, and comprises an LCDR1 set forth in SEQ ID NO: 63, an LCDR2 set forth in SEQ ID NO: 64, and an LCDR3 set forth in SEQ ID NO: 50;

preferably, the antibody comprises a heavy chain variable region of the amino acid sequence set forth in SEQ ID NO: 74 or a variant thereof and a light chain variable region of the amino acid sequence set forth in SEQ ID NO: 71 or a variant thereof.

In one specific embodiment of the present disclosure, the antibody comprises a heavy chain having an amino

acid sequence as set forth in SEQ ID NO: 77 and a light chain having an amino acid sequence as set forth in SEQ ID NO: 78.

In another embodiment of the present disclosure, the anti-ROR1 antibody targets an epitope located at amino acid positions 130-165 of an ROR1 protein (having an amino acid sequence as set forth in SEQ ID NO: 87);

specifically, the antibody comprises an HCDR1 set forth in SEQ ID NO: 17, an HCDR2 set forth in SEQ ID NO: 32, and an HCDR3 set forth in SEQ ID NO: 19, and comprises an LCDR1 set forth in SEQ ID NO: 20, an LCDR2 set forth in SEQ ID NO: 21, and an LCDR3 set forth in SEQ ID NO: 10;

preferably, the antibody comprises a heavy chain variable region of the amino acid sequence set forth in SEQ ID NO: 38 or a variant thereof and a light chain variable region of the amino acid sequence set forth in SEQ ID NO: 23 or a variant thereof;

alternatively, the antibody comprises an HCDR1 set forth in SEQ ID NO: 17, an HCDR2 set forth in SEQ ID NO: 18, and an HCDR3 set forth in SEQ ID NO: 19, and comprises an LCDR1 set forth in SEQ ID NO: 20, an LCDR2 set forth in SEQ ID NO: 21, and an LCDR3 set forth in SEQ ID NO: 10;

preferably, the antibody comprises a heavy chain variable region of the amino acid sequence set forth in SEQ ID NO: 22 or a variant thereof and a light chain variable region of the amino acid sequence set forth in SEQ ID NO: 23 or a variant thereof.

In one specific embodiment of the present disclosure, the antibody comprises a heavy chain having an amino acid sequence as set forth in SEQ ID NO: 44 and a light chain having an amino acid sequence as set forth in SEQ ID NO: 31; or

a heavy chain having an amino acid sequence as set forth in SEQ ID NO: 30 and a light chain having an amino acid sequence as set forth in SEQ ID NO: 31.

The CDR sequences of the antibodies described in the present disclosure are obtained according to the Kabat numbering scheme. However, the CDR sequences of the antibodies of the present disclosure can also be obtained by those skilled in the art according to other known numbering schemes (e.g., IMGT and Chothia).

In another aspect, the present disclosure provides a multispecific antibody comprising the anti-ROR1 antibody described in the present disclosure.

In one embodiment of the present disclosure, the multispecific antibody targets an ROR1 protein and a non-ROR1 protein simultaneously, wherein the non-ROR1 protein includes tumor-associated antigens. The multispecific antibody comprises any anti-ROR1 antibody described in the present disclosure as an antibody moiety that targets the ROR1 protein. Preferably, the multispecific antibody comprises the following anti-ROR1 antibody: the anti-ROR1 antibody comprises an HCDR1 set forth in SEQ ID NO: 61, an HCDR2 set forth in SEQ ID NO: 46, and an HCDR3 set forth in SEQ ID NO: 65, and comprises an LCDR1 set forth in SEQ ID NO: 63, an LCDR2 set forth in SEQ ID NO: 64, and an LCDR3 set forth in SEQ ID NO: 50; alternatively, the anti-ROR1 antibody comprises an HCDR1 set forth in SEQ ID NO: 17, an HCDR2 set forth in SEQ ID NO: 32, and an HCDR3 set forth in SEQ ID NO: 19, and comprises an LCDR1 set forth in SEQ ID NO: 20, an LCDR2 set forth in SEQ ID NO: 21, and an LCDR3 set forth in SEQ ID NO: 10.

In another embodiment of the present disclosure, the multispecific antibody is a bispecific antibody (biepitopic antibody) that targets an ROR1 protein, and the bispecific antibody comprises a first antigen-binding domain that targets a first epitope (located at amino acid positions 130-165 of the ROR1 protein) and a second antigen-binding domain that targets a second epitope (located at amino acid positions 70-130 of the ROR1 protein), wherein the amino acid sequence of the ROR1 protein is set forth in SEQ ID NO: 87. Preferably, the first antigen-binding domain comprises an HCDR1 set forth in SEQ ID NO: 17, an HCDR2 set forth in SEQ ID NO: 32, and an HCDR3 set forth in SEQ ID NO: 19, and comprises an LCDR1 set forth in SEQ ID NO: 20, an LCDR2 set forth in SEQ ID NO: 21, and an LCDR3 set forth in SEQ ID NO: 10, and the second antigen-binding domain comprises an HCDR1 set forth in SEQ ID NO: 61, an HCDR2 set forth in SEQ ID NO: 46, and an HCDR3 set forth in SEQ ID NO: 65, and comprises an LCDR1 set forth in SEQ ID NO: 63, an LCDR2 set forth in SEQ ID NO: 64, and an LCDR3 set forth in SEQ ID NO: 50.

The bispecific antibody of the present disclosure is a heterodimer formed by combining a first Fc variant (Fc1) and a second Fc variant (Fc2) that are obtained after an Fc mutation design, in the "knob-in-hole" form. Techniques for designing mutations for Fc variants have been widely used in the art to produce bispecific antibodies or heterodimeric Fc fusion protein forms. Representative methods include the "knob-in-hole" form proposed by Cater et al. (Protein Engineering vol. 9 no. 7 pp617-621, 1996); the Fc-containing heterodimeric form formed by Amgen's technicians using electrostatic steering (US2010286374A1); the heterodimeric form (SEED bodies) formed using IgG/IgA chain exchange proposed by Jonathan H. Davis et al. (Protein Engineering, Design & Selection pp.1-8, 2010); the bispecific molecule formed using Genmab's DuoBody (Science, 2007. 317 (5844)) platform technology; the heterodimeric protein form formed by Xencor's technicians combining structural calculation and Fc amino acid mutation and different modes of action

(mAbs3:6,546-557; November/December 2011); the heterodimeric protein form obtained using the charge network-based Fc engineering method of Suzhou Alphamab Oncology (CN201110459100.7); and other genetic engineering methods for forming heterodimeric functional proteins by means of Fc amino acid alteration or functional engineering. The selection of a particular immunoglobulin Fc region from a particular immunoglobulin class and subclass is within the knowledge of those skilled in the art. The Fc regions of human IgG1, IgG2, IgG3, and IgG4 antibodies are preferred, and the Fc regions of human IgG1 and IgG4 antibodies are more preferred. One of the first Fc variant or the second Fc variant is randomly selected for a knob mutation and the other for a hole mutation. In an example, the first Fc variant is subjected to a knob mutation, and the second Fc variant is subjected to a hole mutation.

In one specific embodiment of the present disclosure, the bispecific antibody comprises four polypeptide chains: a chain 1 comprising the amino acid sequence set forth in SEQ ID NO: 81, a chain 2 comprising the amino acid sequence set forth in SEQ ID NO: 82, a chain 3 comprising the amino acid sequence set forth in SEQ ID NO: 83, and a chain 4 comprising the amino acid sequence set forth in SEQ ID NO: 84, wherein the chain 1 comprises an Fc knob, and the chain 3 comprises an Fc hole; the chain 1 and chain 3 are combined in the "knob-in-hole" form to form a heterodimer.

The present disclosure further provides an antibody-drug conjugate (ADC) based on the antibody of the present disclosure.

The term antibody-drug conjugate (ADC) means that a monoclonal antibody or an antibody fragment is linked to a biologically active toxic drug by a linker unit. The antibody or the antibody fragment described in the present disclosure may be conjugated to the effector molecule by any means. For example, the antibody or the antibody fragment may be chemically or recombinantly attached to the toxic drug. Chemical means for preparing fusions or conjugates are known in the art. The method for conjugating the antibody or the antibody fragment to the drug must be capable of linking the antibody to the toxic drug without interfering with the ability of the antibody or the antibody fragment to bind to the target molecule.

The drug may be any cytotoxic, cell growth inhibition or immunosuppressive drug. In embodiments, the linker links the antibody and the drug, and the drug has a functional group that can form a bond with the linker. For example, the drug may have an amino, carboxyl, thiol, hydroxyl, or keto group that can form a bond with the linker. In the case of the drug attached directly to the linker, the drug has a reactive group prior to attachment to the antibody. Useful classes of drugs include, for example, anti-tubulin drugs, DNA minor groove binding reagents, DNA replication inhibitors, alkylating reagents, antibiotics, folate antagonists, antimetabolites, chemosensitizers, topoisomerase inhibitors, and vinca alkaloids.

The cytotoxic drug refers to a substance that inhibits or prevents cell functions and/or causes cell death or cell destruction. The cytotoxic drug can kill tumor cells in principle at a sufficiently high concentration; however, due to a lack of specificity, the cytotoxic drug can cause apoptosis of normal cells while killing tumor cells, resulting in serious side effects. The cytotoxic drug includes toxins, such as small molecule toxins or enzymatically active toxins of bacterial, fungal, plant, or animal origin, radioisotopes (e.g., $At^{211}$, $I^{131}$, $I^{125}$, $Y^{90}$, $Re^{186}$ , $Re^{188}$, $Sm^{153}$, $Bi^{212}$, $P^{32}$, and radioisotopes of Lu), chemotherapeutic drugs, antibiotics, and nucleolytic enzymes.

The antibody of the present disclosure may be conjugated to the cytotoxic drug by a conjugating agent. Examples of the conjugating agent may be any one or more of a non-selective conjugating agent, a conjugating agent using a carboxyl group, a peptide chain, and a conjugating agent using a disulfide bond. The non-selective conjugating agent refers to a compound that enables an effector molecule and an antibody to form a covalent bond, such as glutaraldehyde. The conjugating agent using a carboxyl group may be any one or more of a cis-aconitic anhydride conjugating agent (such as cis-aconitic anhydride) and an acylhydrazone conjugating agent (conjugating site is acylhydrazone).

Certain residues on an antibody (such as Cys or Lys) are used to link to a variety of functional groups, including imaging agents (e.g., chromophores and fluorophores), diagnostic agents (e.g., MRI contrast agents and radioisotopes), stabilizing agents (e.g., ethylene glycol polymers), and therapeutic agents. An antibody can be conjugated to a functional agent to form an antibody-functional agent conjugate. The functional agent (e.g., a drug, a detection reagent, a stabilizing agent) is conjugated (covalently linked) to the antibody. The functional agent can be linked to the antibody directly, or indirectly by a linker.

Antibodies can be conjugated to drugs to form antibody-drug conjugates (ADCs). Typically, an ADC comprises a linker between the drug and the antibody. The term "linker unit" or "linking fragment" or "linking unit" refers to a chemical structure fragment or bond that is linked to an antibody or an antigen-binding fragment thereof at one end and to a drug at the other end, and it may also be linked to a drug after being linked to another linker. The linker may be a degradable or non-degradable linker. A degradable linker is typically susceptible to degradation in the intracellular environment. For example, a linker is degraded at the target site, thereby releasing a drug from an antibody. A suitable degradable linker includes, for example, enzymatically degradable linkers including peptidyl-containing linkers that can be degraded by intracellular proteases (e.g., lysosomal proteases or endosomal proteases), or sugar linkers, e.g., glucuronide-containing linkers that can be degraded by glucuronidase. Peptidyl linkers may include, for example, dipeptides such as valine-citrulline, phenylalanine-lysine, or valine-alanine; or tripeptides such as glycine-phenylalanine-glycine; or tetrapeptides such as glycine-glycine-phenylalanine-glycine. Other suitable degradable linkers include, for example, pH-sensitive linkers (e.g.,

linkers that hydrolyze at a pH of less than 5.5, such as hydrazone linkers) and linkers that degrade under reducing conditions (e.g., disulfide linkers). A non-degradable linker typically releases a drug under conditions in which an antibody is hydrolyzed by a protease.

Prior to attachment to the antibody, the linker has a reactive group capable of reacting with certain amino acid residues, and the attachment is achieved by the reactive group. The thiol-specific reactive group is preferred, and includes: such as maleimide compounds, haloamides (e.g., iodo, bromo, or chloro); halogenated esters (e.g., iodo, bromo, or chloro); halomethyl ketones (e.g., iodo, bromo, or chloro), benzyl halides (e.g., iodo, bromo, or chloro); vinyl sulfones, pyridyl disulfides; mercury derivatives such as 3,6-bis-(mercuric methyl)dioxane, and counter ions are acetate, chloride, or nitrate; and polymethylene dimethyl sulfide thiosulfonates. A linker may comprise, for example, a maleimide linked to an antibody via a thiosuccinimide.

In the present disclosure, the drug-linker compound can be used to form an ADC in one simple step. In other embodiments, the bifunctional linker compound may be used to form an ADC in a two-step or multi-step process. For example, a cysteine residue is reacted with a reactive moiety of a linker in the first step, and in a subsequent step, a functional group on the linker is reacted with a drug, thereby forming an ADC.

Generally, the functional group on a linker is selected to facilitate a specific reaction with a suitable reactive group on a drug moiety. As a non-limiting example, an azide-based moiety may be used to specifically react with the reactive alkynyl group on the drug moiety. The drug is covalently bound to the linker through a 1,3-dipolar cycloaddition between the azide and the alkynyl group. Other useful functional groups include, for example, ketones and aldehydes (suitable for reaction with hydrazides and alkoxyamines), phosphines (suitable for reaction with azides); isocyanates and isothiocyanates (suitable for reaction with amines and alcohols); and activated esters, such as N-hydroxysuccinimide esters (suitable for reaction with amines and alcohols). These and other ligation strategies, such as those described in Bioconjugation Technology, 2nd Edition (Elsevier), are well known to those skilled in the art. It will be appreciated by those skilled in the art that for the selective reaction of a drug moiety and a linker, each member of a complementary pair may be used for both the linker and the drug when the reactive functional group of the complementary pair is selected.

The present disclosure further provides a method for preparing an ADC, and the method may further comprise: combining an antibody and a linker-drug compound (LD, e.g., the LD-1 to LD-17 shown in the present disclosure) under conditions sufficient to form an antibody conjugate (ADC).

In certain embodiments, the method of the present disclosure comprises: binding an antibody to a linker compound under conditions sufficient to form an antibody-linker conjugate. In these embodiments, the method of the present disclosure further comprises: binding the antibody-linker conjugate to a drug moiety under conditions sufficient to covalently link the drug moiety to the antibody by a linker.

Drug loading, also referred to as the drug-to-antibody ratio (DAR), refers to the average number of drugs conjugated per antibody in an ADC. The drug loading may be within, for example, a range of about 1 to about 10 drugs conjugated per antibody, and, in certain examples, a range of about 1 to about 8 drugs conjugated per antibody; preferably, the drug loading is selected from the group consisting of the following ranges: 2-8, 2-7, 2-6, 2-5, 2-4, 3-4, 3-5, 5-6, 5-7, 5-8, and 6-8. Illustratively, the drug loading may be an average of 1, 2, 3, 4, 5, 6, 7, 8, 9, or 10. The ADC general formulas of the present disclosure include a set of antibody-drug conjugates within a certain range as described above. In an embodiment of the present disclosure, drug loading may be represented by n and is a decimal or an integer. Drug loading can be determined by conventional methods such as UV/visible spectroscopy, mass spectrometry, ELISA assay, and HPLC.

In one embodiment of the present disclosure, the cytotoxic drug is conjugated to the antibody by a linker unit.

The loading of a ligand-drug conjugate can be controlled by the following non-limiting methods, including:

(1) controlling the molar ratio of a linker fragment of the drug to the monoclonal antibody,

(2) controlling reaction time and temperature, and

(3) selecting different reaction reagents.

The present disclosure further provides a composition. Preferably, the composition is a pharmaceutical composition comprising the above antibody or an active fragment thereof or a fusion protein thereof, and a pharmaceutically acceptable carrier. Generally, these materials can be formulated in a non-toxic, inert, and pharmaceutically acceptable aqueous carrier medium, typically having a pH of about 5-8, preferably a pH of about 6-8, although the pH value may vary depending on the nature of the materials being formulated and conditions being treated. The formulated pharmaceutical composition can be administered via conventional routes, including (but not limited to) intravenous injection, intravenous drip, subcutaneous injection, topical injection, intramuscular injection, intratumoral injection, intraperitoneal injection (e.g., intraperitoneal), intracranial injection, or intracavity injection. In the present disclosure, the term "pharmaceutical composition" means that the bispecific antibody of the present disclosure can be combined with a pharmaceutically acceptable carrier to form a pharmaceutical formulation composition, so that the antibody can more

stably exert its therapeutic effect. These formulations can ensure the conformational integrity of the core sequence of amino acids of the bispecific antibody disclosed herein while protecting the multifunctional groups of the protein to prevent it from degradation (including but not limited to aggregation, deamination, or oxidation). The pharmaceutical composition of the present disclosure contains a safe and effective amount (such as 0.001-99 wt%, preferably 0.01-90 wt%, and more preferably 0.1-80 wt%) of the above anti-ROR1 antibody, bispecific antibody, or antibody-drug conjugate of the present disclosure and a pharmaceutically acceptable carrier or excipient. Such vectors include (but are not limited to): saline, buffer, glucose, water, glycerol, ethanol, and combinations thereof. The pharmaceutical formulation should be compatible with the mode of administration. The pharmaceutical composition of the present disclosure can be prepared in the form of an injection, for example, by a conventional method using normal saline or an aqueous solution containing glucose and other adjuvants. Pharmaceutical compositions (such as injections and solutions) are preferably manufactured under sterile conditions. The amount of active ingredient administered is a therapeutically effective amount, for example, about 10 μg/kg body weight to about 50 mg/kg body weight per day. In addition, the pharmaceutical composition of the present disclosure may also be used together with other therapeutic agents.

When the pharmaceutical composition is used, a safe and effective amount of the antibody or multispecific antibody or an immunoconjugate thereof is administered to a mammal, wherein the safe and effective amount is generally at least about 10 μg/kg body weight, and in most cases no more than about 50 mg/kg body weight. Preferably, the dosage is about 10 μg/kg body weight to about 10 mg/kg body weight. In determining a specific dose, such factors as the route of administration, the health condition of the patient, and the like will also be considered, which are well-known to skilled physicians.

The term "bond" means that the group is absent and the groups on both sides are directly linked to form a bond.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$ alkyl). Preferably, the alkyl is an alkyl group having 1 to 12 carbon atoms (i.e., $C_{1-12}$ alkyl); more preferably, the alkyl is an alkyl group having 1 to 6 carbon atoms (i.e., $C_{1-6}$ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, and the like. Alkyl may be substituted or unsubstituted, and when it is substituted, it may be substituted at any accessible point of attachment, and the substituent is preferably selected from one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples include: methoxy, ethoxy, propoxy, butoxy, and the like. Alkoxy may be substituted or unsubstituted, and when it is substituted, it may be substituted at any accessible point of attachment, and the substituent is preferably selected from one or more of a D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "cycloalkyl" refers to a saturated or partially unsaturated monocyclic all-carbon ring (i.e., monocyclic cycloalkyl) or polycyclic system (i.e., polycyclic cycloalkyl), and it has 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). Preferably, the cycloalkyl is a cycloalkyl group having 3 to 12 ring atoms (i.e., 3- to 12-membered cycloalkyl); more preferably, the cycloalkyl is a cycloalkyl group having 3 to 8 ring atoms (i.e., 3- to 8-membered cycloalkyl, e.g., $C_{3-7}$ cycloalkyl); most preferably, the cycloalkyl is a cycloalkyl group having 3 to 6 ring atoms (i.e., 3- to 6-membered cycloalkyl, e.g., $C_{3-6}$ cycloalkyl).

The term "aryl" refers to a monocyclic all-carbon aromatic ring (i.e., monocyclic aryl) or polycyclic aromatic ring system (i.e., polycyclic aryl) having a conjugated π-electron system, and it has 6 to 14 (e.g., 6, 7, 8, 9, 10, 11, 12, 13, or 14) ring atoms (i.e., 6- to 14-membered aryl). The aryl is preferably an aryl group having 6 to 10 ring atoms (i.e., 6- to 10-membered aryl). The monocyclic aryl is, for example, phenyl. Non-limiting examples of the polycyclic aryl include: naphthyl, anthryl, phenanthryl, and the like. The polycyclic aryl also includes those that are formed by fusing a phenyl group with one or more of a heterocyclyl group or a cycloalkyl group or fusing a naphthyl group with one or more of a heterocyclyl group or a cycloalkyl group, wherein the point of attachment is on the phenyl group or the naphthyl group, and in the circumstances the number of ring atoms continues to represent the number of ring atoms in the polycyclic aromatic ring system; non-limiting examples include:

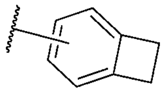, 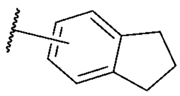, 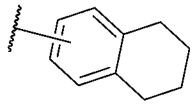, 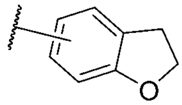, 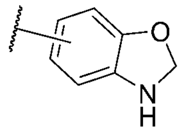,

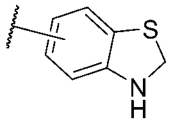,

and the like.

Aryl may be substituted or unsubstituted, and when it is substituted, it may be substituted at any accessible point of attachment, and the substituent is preferably selected from one or more of a D atom, halogen, alkyl, alkoxy, haloalkyl, haloalkoxy, cycloalkyloxy, heterocyclyloxy, hydroxyl, hydroxyalkyl, oxo, cyano, amino, nitro, cycloalkyl, heterocyclyl, aryl, and heteroaryl.

The term "heteroaryl" refers to a monocyclic heteroaromatic ring (i.e., monocyclic heteroaryl) or polycyclic heteroaromatic ring system (i.e., polycyclic heteroaryl) having a conjugated $\pi$-electron system, and it contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, but -O-O-, -O-S-, or -S-S- is excluded) and has 5 to 14 (e.g., 5, 6, 7, 8, 9, 10, 11, 12, 13, or 14) ring atoms (i.e., 5- to 14-membered heteroaryl). Preferably, the heteroaryl is a heteroaryl group having 5 to 10 ring atoms (i.e., 5- to 10-membered heteroaryl); more preferably, the heteroaryl is a heteroaryl group having 5 or 6 ring atoms (i.e., 5- or 6-membered heteroaryl).

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to alkyl substituted with one or more halogens, wherein the alkyl is as defined above.

The term "deuterated alkyl" refers to alkyl substituted with one or more deuterium atoms, wherein the alkyl is as defined above.

The term "haloalkoxy" refers to alkoxy substituted with one or more halogens, wherein the alkoxy is as defined above.

The term "hydroxyalkyl" refers to alkyl substituted with one or more hydroxyl groups, wherein the alkyl is as defined above.

The term "halogen" refers to fluorine, chlorine, bromine, or iodine.

The term "hydroxyl" refers to -OH.

The term "amino" refers to $-NH_2$.

The term "cyano" refers to -CN.

The term "oxo" refers to =O.

N-Ethyldiisopropylamine is abbreviated as DIEA.

N,N-Dimethylformamide is abbreviated as DMF.

O-(7-Azabenzotriazol-1-yl)-N,N,N',N'-tetramethyluronium hexafluorophosphate is abbreviated as HATU.

1-Hydroxybenzotriazole is abbreviated as HOBt.

"Substituted" means that one or more, preferably 1-6, and more preferably 1-3, hydrogen atoms in the group are independently substituted with a corresponding number of substituents. Those skilled in the art can determine (experimentally or theoretically) possible or impossible substitutions without undue effort. For example, it may be unstable when an amino or hydroxyl group having free hydrogen is bound to a carbon atom having an unsaturated (e.g., olefinic) bond.

The present disclosure is illustrated with reference to the following specific examples. It will be appreciated by those skilled in the art that these examples are merely illustrative of the present disclosure and are not intended to limit the scope of the present disclosure in any way.

Unless otherwise specified, the experimental methods in the following examples are all conventional methods. The starting materials, reagent materials, and the like used in the following examples are all commercially available products unless otherwise specified.

**Example 1: Humanization and Expression of Antibody D10**

Humanization engineering of murine anti-ROR1 monoclonal antibody D10:

The murine antibody was subjected to humanization engineering using CDR grafting. The CDRs were grafted to framework sequences of matching heavy chain and light chain variable regions to obtain a heavy chain variable region sequence (set forth in SEQ ID NO: 1) and a light chain variable region sequence (set forth in SEQ ID NO: 2) of humanized antibody BRHu-3.

BRHu-3-VH (SEQ ID NO: 1):

QVQLQESGPGLVKPSETLSLTCTVSGFSLTSYGVHWIRQPPGKGLEWLGVIWAGGFTNYNSALKSRLTISKDNSKNQVSLKLSSVTAADTAVYYCARRGSSYSMDYWGQGTLVTVSS

BRHu-3-VL (SEQ ID NO: 2):

EIVLTQSPATLSLSPGERATLSCSASSNVSYIHWYQQKPGQAPRPWIYEISKLASGIPARFSGSGSGTDYTLTISSLEPEDFAVYYCQQWNYPLITFGQGTKLEIK

D10-VH (SEQ ID NO: 3):

QVQLKESGPGLVAPSQTLSITCTVSGFSLTSYGVHWVRQPPGKGLEWLGVIWAGGFTNYNSALKSRLSISKDNSKSQVLLKMTSLQTDDTAMYYCARRGSSYSMDYWGQGTSVTVSS

D10-VL (SEQ ID NO: 4):

EIVLSQSPAITAASLGQKVTITCSASSNVSYIHWYQQRSGTSPRPWIYEISKLASGVPVRFSGSGSGTSYSLTISSMEAEDAAIYYCQQWNYPLITFGSGTKLEIQ

Table 1. The CDRs of antibody D10/BRHu-3

| Antibody | Sequence | No. |
|---|---|---|
| HCDR1 | SYGVH | SEQ ID NO: 5 |
| HCDR2 | VIWAGGFTNYNSALKS | SEQ ID NO: 6 |
| HCDR3 | RGSSYSMDY | SEQ ID NO: 7 |
| LCDR1 | SASSNVSYIH | SEQ ID NO: 8 |
| LCDR2 | EISKLAS | SEQ ID NO: 9 |
| LCDR3 | QQWNYPLIT | SEQ ID NO: 10 |

The VH and VL of the above antibody BRHu-3 were combined with the IgG1 human heavy chain constant region and the κ light chain constant region, respectively, to obtain humanized antibody BRHu-3, and the VH and VL of monoclonal antibody D10 were combined with these constant regions, respectively, to obtain chimeric antibody ch-D10.

IgG1 heavy chain constant region (SEQ ID NO: 11):

ASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKVEPKSCDKTHTCP PCPAPELLGGPSVFLFPPKPKDTLMISRTPEVTCVVVDVSHEDPEVKFNWYVDGVEV HNAKTKPREEQYNSTYRVVSVLTVLHQDWLNGKEYKCKVSNKALPAPIEKTISKAK GQPREPQVYTLPPSREEMTKNQVSLTCLVKGFYPSDIAVEWESNGQPENNYKTTPPVL DSDGSFFLYSKLTVDKSRWQQGNVFSCSVMHEALHNHYTQKSLSLSPGK

κ light chain constant region (SEQ ID NO: 12):

RTVAAPSVFIFPPSDEQLKSGTASVVCLLNNFYPREAKVQWKVDNALQSGNS QESVTEQDSKDSTYSLSSTLTLSKADYEKHKVYACEVTHQGLSSPVTKSFNRGEC

Chimeric antibody ch-D10 (whose heavy chain amino acid sequence is set forth in SEQ ID NO: 15 and light chain amino acid sequence is set forth in SEQ ID NO: 16) and humanized antibody BRHu-3 (whose heavy chain amino acid sequence is set forth in SEQ ID NO: 13 and light chain amino acid sequence is set forth in SEQ ID NO: 14) were expressed in CHO-K1 cells and purified. Firstly, vectors containing sequences encoding the heavy and light chains of the antibodies were transfected into CHO-K1 cells by electroporation. After the cells were cultured at 37 °C with 5% $CO_2$ for 4 days, the cell culture supernatants were centrifuged at 3000 rpm for 10 min. The supernatants were purified by Protein A affinity chromatography to obtain the antibodies with purity >95%.

Heavy chain sequence of BRHu-3 (SEQ ID NO: 13):

QVQLQESGPGLVKPSETLSLTCTVSGFSLTSYGVHWIRQPPGKGLEWLGVIW AGGFTNYNSALKSRLTISKDNSKNQVSLKLSSVTAADTAVYYCARRGSSYSMDYWG QGTLVTVSSASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEPKSCDKTH TCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKA KGQPREPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK

Light chain sequence of BRHu-3 (SEQ ID NO: 14):

EIVLTQSPATLSLSPGERATLSCSASSNVSYIHWYQQKPGQAPRPWIYEISKLA SGIPARFSGSGSGTDYTLTISSLEPEDFAVYYCQQWNYPLITFGQGTKLEIKRTVAAPSV FIFPPSDEQLKSGTASVVCLLNNFYPREAKVQWKVDNALQSGNSQESVTEQDSKDST YSLSSTLTLSKADYEKHKVYACEVTHQGLSSPVTKSFNRGEC

Heavy chain sequence of chimeric antibody ch-D10 (SEQ ID NO: 15):

QVQLKESGPGLVAPSQTLSITCTVSGFSLTSYGVHWVRQPPGKGLEWLGVIW AGGFTNYNSALKSRLSISKDNSKSQVLLKMTSLQTDDTAMYYCARRGSSYSMDYW GQGTSVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISK AKGQPREPQVYTLPPSREEMTKNQVSLTCLVKGFYPSDIAVEWESNGQPENNYKTTP PVLDSDGSFFLYSKLTVDKSRWQQGNVFSCSVMHEALHNHYTQKSLSLSPGK

Light chain sequence of chimeric antibody ch-D10 (SEQ ID NO: 16):

EIVLSQSPAITAASLGQKVTITCSASSNVSYIHWYQQRSGTSPRPWIYEISKLA SGVPVRFSGSGSGTSYSLTISSMEAEDAAIYYCQQWNYPLITFGSGTKLEIQRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGEC

**Example 2: Affinity Maturation of Humanized Antibody BRHu-3**

Humanized antibody BRHu-3 was subjected to affinity maturation as follows:

With humanized antibody BRHu-3 as a mutation template, a primer was designed. Random mutations were introduced into the 6 CDRs of BRHu-3 by PCR, and the mutant DNA was electroporated into SU320+Helper phage supercompetent cells. The cells were then cultured overnight at 37 °C for expansion. Separation and purification were performed using the PEG8000/NaCl precipitation method to obtain a phage display mutant library.

The phage display mutant library was subjected to biopanning to enrich positive monoclones. A Maxi-sorp plate was coated overnight at 4 °C with antigen ROR2 at 500 ng/well. The Maxi-sorp plate was blocked with 1% PVA at room temperature for 2 h and subsequently incubated with phages from the mutant library at 4 °C for 2 h. The supernatant was collected and incubated with negative CHO-K1 cells at 4 °C for 2 h. The mixture was centrifuged, and the supernatant was collected and incubated with CHO-K1-hROR1 cells (a stable cell line that overexpresses human ROR1; the sequence of human ROR1 is set forth in SEQ ID NO: 87) at 4 °C for 2 h. A PBS buffer containing 0.05% Tween20 (PBST) pre-cooled at 4 °C was added. After 6-8 washes, 1 mL of NEBalpha5F' cells (OD600 = 0.8, purchased from NEB) was infected with phages, and the bacteria were shaken at 37 °C for 1 h. Subsequently, M13K07 helper phages (purchased from NEB) were added, and the bacteria were shaken at 37 °C for 1 h, transferred to 40 mL of 2YT/Carb/Kan culture medium, and cultured overnight at 37 °C for expansion. The culture was applied to a plate. On day 3, the degree of enrichment was determined. Phages were isolated and purified using the PEG 8000/NaCl precipitation method and subjected to the next round of panning.

According to the above steps, 5 rounds of biopanning were performed, yielding successfully enriched phage clones.

From the enriched phage clones, monoclonal phages were randomly picked for a phage ELISA experiment. ELISA plates were coated overnight at 4 °C with recombinant proteins ROR1 (purchased from Kactus Biosystems) and ROR2 (purchased from Kactus Biosystems) at 100 ng/well and then blocked with 1% PVA at room temperature for 2 h. On the day of coating, monoclonal phages were picked, inoculated into a deep-well plate, and then cultured at 37 °C overnight with shaking. The deep-well plate was centrifuged, and 50 μL of the supernatant was transferred to the ELISA plates. The plates were then incubated at room temperature for 2 h. The ELISA plates were washed with PBST, and 100 μL of anti-M13 HRP antibody (purchased from Sino Biological) was added. The plates were then incubated at room temperature for 1 h. Subsequently, the ELISA plates were washed with PBST and then with PBS, and 100 μL of TMB was added. The plates were then left at 37 °C for 5-10 min. 50 μL of 1 M phosphoric acid was added to stop the reaction. The absorbance at 450 nm was measured using a microplate reader. Five clones with high affinity were sequenced. The 5 clones were YR-4, YR-8, YR-10, YR-11, and YR-21. The variable regions and CDRs of the antibodies are shown below:

Table 2. The CDRs of antibody YR-8

| Antibody YR-8 | Sequence | No. |
|---|---|---|
| HCDR1 | RYGVH | SEQ ID NO: 17 |
| HCDR2 | VIWAGGFTNYNSDLKS | SEQ ID NO: 18 |
| HCDR3 | RGDSYSMKY | SEQ ID NO: 19 |
| LCDR1 | SASSQVSYIH | SEQ ID NO: 20 |
| LCDR2 | ETSKLAS | SEQ ID NO: 21 |
| LCDR3 | QQWNYPLIT | SEQ ID NO: 10 |

The variable region sequences of the antibodies obtained by affinity maturation:

YR-8-VH (SEQ ID NO: 22):

QVQLQESGPGLVKPSETLSLTCTVSGFSLTRYGVHWIRQPPGKGLEWLGVIWAGGFTNYNSDLKSRLTISKDNSKNQVSLKLSSVTAADTAVYYCARRGDSYSMKYWGQGTLVTVSS

YR-8-VL (SEQ ID NO: 23):

EIVLTQSPATLSLSPGERATLSCSASSQVSYIHWYQQKPGQAPRPWIYETSKLASGIPARFSGSGSGTDYTLTISSLEPEDFAVYYCQQWNYPLITFGQGTKLEIK

YR-4-VH (SEQ ID NO: 24):

QVQLQESGPGLVKPSETLSLTCTVSGFSLTRYGVHWIRQPPGKGLEWIGVIWAGGFTNYNSSLKSRVTISKDNSKNQVSLKLSSVTAADTAVYYCARRGDGYSMQYWGQGTLVTVSS

YR-4-VL (SEQ ID NO: 25):

EIVLTQSPATLSLSPGERATLSCSASSQVSYIHWYQQKPGQAPRPWIYEISKLASGIPARFSGSGSGTDYTLTISSLEPEDFAVYYCQQWNYPLITFGQGTKLEIK

YR-10-VH (SEQ ID NO: 26):

QVQLQESGPGLVKPSETLSLTCTVSGFSLTRYGVHWIRQPPGKGLEWIGVIWAGGFTNYNSTLKSRVTISKDNSKNQVSLKLSSVTAADTAVYYCARRGNQYSMKYWGQGTLVTVSS

YR-10-VL (SEQ ID NO: 27):

EIVLTQSPATLSLSPGERATLSCSASSQVSYIHWYQQKPGQAPRPWIYETSKLASGIPARFSGSGSGTDYTLTISSLEPEDFAVYYCQQWNYPLISFGQGTKLEIK

YR-11-VH (SEQ ID NO: 28):

QVQLQESGPGLVKPSETLSLTCTVSGFSLTRYGVHWIRQPPGKGLEWIGVIWAGGFTNYNSALKSRVTISKDNSKNQVSLKLSSVTAADTAVYYCARRGNEYSMSYWGQGTLVTVSS

YR-11-VL (SEQ ID NO: 23):

EIVLTQSPATLSLSPGERATLSCSASSQVSYIHWYQQKPGQAPRPWIYETSKLASGIPARFSGSGSGTDYTLTISSLEPEDFAVYYCQQWNYPLITFGQGTKLEIK

YR-21-VH (SEQ ID NO: 29):

QVQLQESGPGLVKPSETLSLTCTVSGFSLTRYGVHWIRQPPGKGLEWLGVIWAGGFTNYNSGLKSRLTISKDNSKNQVSLKLSSVTAADTAVYYCARRGDQYSMGYWGQGTLVTVSS

YR-21-VL (SEQ ID NO: 23):

EIVLTQSPATLSLSPGERATLSCSASSQVSYIHWYQQKPGQAPRPWIYETSKLASGIPARFSGSGSGTDYTLTISSLEPEDFAVYYCQQWNYPLITFGQGTKLEIK

The above VH and VL sequences were combined with the IgG1 human heavy chain constant region and the κ light chain constant region (as shown in Example 1), respectively, to obtain the full-length sequences of the antibodies. The above antibodies were expressed and purified using the method described in Example 1. The heavy and light chains of the exemplary antibody YR-8 are set forth in SEQ ID NO: 30 and SEQ ID NO: 31, respectively.

Heavy chain sequence of YR-8 (SEQ ID NO: 30):

QVQLQESGPGLVKPSETLSLTCTVSGFSLTRYGVHWIRQPPGKGLEWLGVIWAGGFTNYNSDLKSRLTISKDNSKNQVSLKLSSVTAADTAVYYCARRGDSYSMKYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSLTCLVKGFYPSDIAVEWESNGQPENNYKTTPPVLDSDGSFFLYSKLTVDKSRWQQGNVFSCSVMHEALHNHYTQKSLSLSPGK

Light chain sequence of YR-8 (SEQ ID NO: 31):

EIVLTQSPATLSLSPGERATLSCSASSQVSYIHWYQQKPGQAPRPWIYETSKLASGIPARFSGSGSGTDYTLTISSLEPEDFAVYYCQQWNYPLITFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

Example 3: Engineering of Affinity-Matured Antibody Molecule YR-8

The N at position 60 and the S at position 61 of the heavy chain of molecule YR-8 (amino acid positions obtained according to Kabat numbering) were subjected to amino acid mutation. The mutation scheme included: mutating the N at position 60 to E, or mutating the S at position 61 to A, V, T, L, and I. The mutant molecules were designated YR-8-ES, YR-8-NA, YR-8-NV, YR-8-NT, YR-8-NL, and YR-8-NI, respectively. The HCDR2 and VH sequences of the mutant antibodies are shown below:

Table 3. The HCDR2 sequences of the engineered antibodies of YR-8

| Antibody | HCDR2 | SEQ ID NO |
|---|---|---|
| YR-8-ES | VIWAGGFTNYESDLKS | 32 |
| YR-8-NA | VIWAGGFTNYNADLKS | 33 |
| YR-8-NV | VIWAGGFTNYNVDLKS | 34 |
| YR-8-NT | VIWAGGFTNYNTDLKS | 35 |
| YR-8-NL | VIWAGGFTNYNLDLKS | 36 |
| YR-8-NI | VIWAGGFTNYNIDLKS | 37 |

The VH sequences of the mutant antibodies are shown below:

YR-8-ES-VH (SEQ ID NO: 38):

QVQLQESGPGLVKPSETLSLTCTVSGFSLTRYGVHWIRQPPGKGLEWLGVIWAGGFTNYESDLKSRLTISKDNSKNQVSLKLSSVTAADTAVYYCARRGDSYSMKYWGQGTLVTVSS

YR-8-NA-VH (SEQ ID NO: 39):

QVQLQESGPGLVKPSETLSLTCTVSGFSLTRYGVHWIRQPPGKGLEWLGVIWAGGFTNYNADLKSRLTISKDNSKNQVSLKLSSVTAADTAVYYCARRGDSYSMKYWGQGTLVTVSS

YR-8-NV-VH (SEQ ID NO: 40):

QVQLQESGPGLVKPSETLSLTCTVSGFSLTRYGVHWIRQPPGKGLEWLGVIWAGGFTNYNVDLKSRLTISKDNSKNQVSLKLSSVTAADTAVYYCARRGDSYSMKYWGQGTLVTVSS

YR-8-NT-VH (SEQ ID NO: 41):

QVQLQESGPGLVKPSETLSLTCTVSGFSLTRYGVHWIRQPPGKGLEWLGVIWAGGFTNYNTDLKSRLTISKDNSKNQVSLKLSSVTAADTAVYYCARRGDSYSMKYWGQGTLVTVSS

YR-8-NL-VH (SEQ ID NO: 42):

QVQLQESGPGLVKPSETLSLTCTVSGFSLTRYGVHWIRQPPGKGLEWLGVIW AGGFTNYNLDLKSRLTISKDNSKNQVSLKLSSVTAADTAVYYCARRGDSYSMKYWG QGTLVTVSS

**[0337]** YR-8-NI-VH (SEQ ID NO: 43):

QVQLQESGPGLVKPSETLSLTCTVSGFSLTRYGVHWIRQPPGKGLEWLGVIW AGGFTNYNIDLKSRLTISKDNSKNQVSLKLSSVTAADTAVYYCARRGDSYSMKYWG QGTLVTVSS

The mutated VH sequences described above and YR-8-VL (SEQ ID NO: 23) were combined with the human IgG1 heavy chain constant region and the κ light chain constant region (as shown in Example 1) to obtain complete antibodies. The above mutants were expressed and purified using the method described in Example 1. The heavy and light chains of the exemplary antibody YR-8-ES obtained by the present disclosure are set forth in SEQ ID NO: 44 and SEQ ID NO: 31, respectively.

Heavy chain sequence of YR-8-ES (SEQ ID NO: 44):

QVQLQESGPGLVKPSETLSLTCTVSGFSLTRYGVHWIRQPPGKGLEWLGVIW AGGFTNYESDLKSRLTISKDNSKNQVSLKLSSVTAADTAVYYCARRGDSYSMKYWG QGTLVTVSSASTKGPSVFPLAPSSKSTSGGTAALGCLVKDYFPEPVTVSWNSGALTSG

VHTFPAVLQSSGLYSLSSVVTVPSSSLGTQTYICNVNHKPSNTKVDKKVEPKSCDKTH TCPPCPAPELLGGPSVFLFPPKPKDTLMISRTPEVTCVVVDVSHEDPEVKFNWYVDGV EVHNAKTKPREEQYNSTYRVVSVLTVLHQDWLNGKEYKCKVSNKALPAPIEKTISKA KGQPREPQVYTLPPSREEMTKNQVSLTCLVKGFYPSDIAVEWESNGQPENNYKTTPP VLDSDGSFFLYSKLTVDKSRWQQGNVFSCSVMHEALHNHYTQKSLSLSPGK

Light chain sequence of YR-8-ES (SEQ ID NO: 31):

EIVLTQSPATLSLSPGERATLSCSASSQVSYIHWYQQKPGQAPRPWIYETSKLA SGIPARFSGSGSGTDYTLTISSLEPEDFAVYYCQQWNYPLITFGQGTKLEIKRTVAAPSV FIFPPSDEQLKSGTASVVCLLNNFYPREAKVQWKVDNALQSGNSQESVTEQDSKDST YSLSSTLTLSKADYEKHKVYACEVTHQGLSSPVTKSFNRGEC

Example 4: **Humanization of Antibody H10**

The anti-ROR1 murine monoclonal antibody H10 was subjected to humanization engineering using CDR grafting. The CDRs were grafted to framework sequences of matching heavy chain and the light chain variable regions to obtain 16 humanized molecules with different sequences. The sequences of the variable regions and CDRs of the humanized antibodies are shown below:

Table 4. The CDRs of H10/humanized antibodies of H10

| Antibody H10 | Sequence | No. |
|---|---|---|
| HCDR1 | SYAMS | SEQ ID NO: 45 |

(continued)

| Antibody H10 | Sequence | No. |
|---|---|---|
| HCDR2 | SISTGASAYFPDSVKG | SEQ ID NO: 46 |
| HCDR3 | ITTSTWYFDV | SEQ ID NO: 47 |
| LCDR1 | KASQDINSYLS | SEQ ID NO: 48 |
| LCDR2 | RANRLVD | SEQ ID NO: 49 |
| LCDR3 | LQYDEFPYT | SEQ ID NO: 50 |

H10 VH (SEQ ID NO: 51):

EVKLVESGGGLVKPGGSLKLSCAASGFTFSSYAMSWVRQTPEKRLEWVASISTGASAYFPDSVKGRFTISRDNARNILYLQMSSLRSEDTAMYYCARITTSTWYFDVWGAGTTVTVSS

H10 VL (SEQ ID NO: 52):

DIKMTQSPSSMYASLGERVTITCKASQDINSYLSWFQQKPGKSPKTLIYRANRLVDGVPSRFSGSGSGQDYSLTISSLEYEDMGIYYCLQYDEFPYTFGGGTKLEIK

H10-hVH1 (SEQ ID NO: 53):

EVQLVESGGGLVKPGGSLRLSCAASGFTFSSYAMSWVRQAPGKGLEWVSSISTGASAYFPDSVKGRFTISRDNAKNSLYLQMNSLRAEDTAVYYCARITTSTWYFDVWGQGTTVTVSS

H10-hVH2 (SEQ ID NO: 54):

EVQLVESGGGLVQPGGSLRLSCAASGFTFSSYAMSWVRQAPGKGLEWVSSISTGASAYFPDSVKGRFTISRDNSKNTLYLQMNSLRAEDTAVYYCARITTSTWYFDVWGQGTTVTVSS

H10-hVH3 (SEQ ID NO: 55):

EVQLVESGGGLVQPGGSLRLSCAASGFTFSSYAMSWVRQAPGKGLEWVASISTGASAYFPDSVKGRFTISRDNAKNSLYLQMNSLRAEDTAVYYCARITTSTWYFDVWGQGTTVTVSS

H10-hVH4 (SEQ ID NO: 56):

EVQLVESGGGLVQPGGSLRLSCAASGFTFSSYAMSWVRQAPGKGLEWVSSISTGASAYFPDSVKGRFTISRDNAKNSLYLQMNSLRAEDTAVYYCARITTSTWYFDVWGQGTTVTVSS

H10-hVL1 (SEQ ID NO: 57):

DIQMTQSPSSLSASVGDRVTITCKASQDINSYLSWFQQKPGKAPKTLIYRANR
LVDGVPSRFSGSGSGQDYTLTISSLQPEDMATYYCLQYDEFPYTFGQGTKLEIK

H10-hVL2 (SEQ ID NO: 58):

DIQMTQSPSSLSASVGDRVTITCKASQDINSYLSWFQQKPGKAPKTLIYRANR
LVDGVPSRFSGSGSGQDYTFTISSLQPEDMATYYCLQYDEFPYTFGQGTKLEIK

H10-hVL4 (SEQ ID NO: 59):

DIQMTQSPSAMSASVGDRVTITCKASQDINSYLSWFQQKPGKVPKTLIYRAN
RLVDGVPSRFSGSGSGQEYTLTISSLQPEDMATYYCLQYDEFPYTFGQGTKLEIK

H10-hVL5 (SEQ ID NO: 60):

DIQMTQSPSSLSASVGDRVTITCKASQDINSYLSWFQQKPGKVPKTLIYRANR

LVDGVPSRFSGSGSGQDYTLTISSLQPEDMATYYCLQYDEFPYTFGQGTKLEIK

The variable regions of the humanized antibodies of **H10** were combined with the human IgG1 heavy chain constant region and the κ light chain constant region (as shown in Example 1) to obtain complete antibodies. The specific antibodies are shown below:

Table 5. The humanized antibodies of H10

| Variable region | H10-hVH1 | H10-hVH2 | H10-hVH3 | H10-hVH4 |
|---|---|---|---|---|
| H10-hVL1 | H10-hVH1-hVL1 | H10-hVH2-hVL1 | H10-hVH3-hVL1 | H10-hVH4-hVL1 |
| H10-hVL2 | H10-hVH1-hVL2 | H10-hVH2-hVL2 | H10-hVH3-hVL2 | H10-hVH4-hVL2 |
| H10-hVL4 | H10-hVH1-hVL4 | H10-hVH2-hVL4 | H10-hVH3-hVL4 | H10-hVH4-hVL4 |
| H10-hVL5 | H10-hVH1-hVL5 | H10-hVH2-hVL5 | H10-hVH3-hVL5 | H10-hVH4-hVL5 |

**Example 5: Affinity Maturation of Humanized Antibodies of H10**

The 6 CDRs of the H10-hVH4-hVL4 described above were subjected to saturation mutagenesis. Vectors containing sequences encoding the heavy and light chains of the mutated antibodies were transfected into CHO-K1 cells by electroporation. The cells were cultured at 37 °C with 5% $CO_2$ for 4 days. The supernatant was collected and added to a microplate coated with ROR1 (manufacturer: Kactus Biosystems, Cat. No. ROR-HM401), and the plate was incubated at 37 °C for 1 h. The plate was washed, and HRP-labeled goat anti-human IgG (manufacturer: Jackson, 109-005-008) was added. The plate was then incubated at 37 °C for 1 h. After the plate was washed, the TMB solution was added. The plate was incubated in a dark place at room temperature for 15 min, and the ELISA stopping solution was then added to stop the reaction. The plate was placed on a microplate reader, and the absorbance at a wavelength of 450 nm was measured. Six clones with high affinity were sequenced. The 6 clones obtained were named AM3-ZH1, AM3-ZH3, AM3-ZH4, AM3-ZH5, AM3-ZH6, and AM3-ZH7. The related antibody sequences are shown below:

Table 6. The CDR sequences of the affinity-matured antibodies of H10

| Antibody | CDR | Sequence | No. |
|---|---|---|---|
| AM3-ZH1 | HCDR1 | SYPMS | SEQ ID NO: 61 |
| | HCDR2 | SISTGASAYFPDSVKG | SEQ ID NO: 46 |
| | HCDR3 | YTTSTWYFDV | SEQ ID NO: 62 |
| | LCDR1 | KASQDIYSYLS | SEQ ID NO: 63 |
| | LCDR2 | RANRLVR | SEQ ID NO: 64 |
| | LCDR3 | LQYDEFPYT | SEQ ID NO: 50 |
| AM3-ZH3 | HCDR1 | SYPMS | SEQ ID NO: 61 |
| | HCDR2 | SISTGASAYFPDSVKG | SEQ ID NO: 46 |
| | HCDR3 | ITTSTWYMDV | SEQ ID NO: 65 |
| | LCDR1 | KASQDIYSYLS | SEQ ID NO: 63 |
| | LCDR2 | RANRLVR | SEQ ID NO: 64 |
| | LCDR3 | LQYDEFPYT | SEQ ID NO: 50 |
| AM3-ZH4 | HCDR1 | SYAMS | SEQ ID NO: 45 |
| | HCDR2 | SISTGASAYFPDSVKG | SEQ ID NO: 46 |
| | HCDR3 | YTTSTWYHDV | SEQ ID NO: 66 |
| | LCDR1 | KASQDIYSYLS | SEQ ID NO: 63 |
| | LCDR2 | RANRLVR | SEQ ID NO: 64 |
| | LCDR3 | LQYDEFPYT | SEQ ID NO: 50 |
| AM3-ZH5 | HCDR1 | SYAMS | SEQ ID NO: 45 |
| | HCDR2 | SISTGASAYFPDSVKG | SEQ ID NO: 46 |
| | HCDR3 | ITTSTWYMDV | SEQ ID NO: 65 |
| | LCDR1 | KASQDIFSYLS | SEQ ID NO: 67 |
| | LCDR2 | RANRLVS | SEQ ID NO: 68 |
| | LCDR3 | LQYDEFPYT | SEQ ID NO: 50 |
| AM3-ZH6 | HCDR1 | SYAMS | SEQ ID NO: 45 |
| | HCDR2 | SISTGASAYFPDSVKG | SEQ ID NO: 46 |
| | HCDR3 | ITTSTWYMDV | SEQ ID NO: 65 |
| | LCDR1 | KASQDIYSYLS | SEQ ID NO: 63 |
| | LCDR2 | RANRLVG | SEQ ID NO: 69 |
| | LCDR3 | LQYDEFPYT | SEQ ID NO: 50 |
| AM3-ZH7 | HCDR1 | SYAMS | SEQ ID NO: 45 |
| | HCDR2 | SISTGASAYFPDSVKG | SEQ ID NO: 46 |
| | HCDR3 | ITTSTWYMDV | SEQ ID NO: 65 |
| | LCDR1 | KASQDIYSYLS | SEQ ID NO: 63 |
| | LCDR2 | RANRLVR | SEQ ID NO: 64 |
| | LCDR3 | LQYDEFPYT | SEQ ID NO: 50 |

AM3-ZH1-VH (SEQ ID NO: 70):

EVQLVESGGGLVQPGGSLRLSCAASGFTFSSYPMSWVRQAPGKGLEWVSSISTGASAYFPDSVKGRFTISRDNAKNSLYLQMNSLRAEDTAVYYCARYTTSTWYFDVWGQGTTVTVSS

AM3-ZH1-VL (SEQ ID NO: 71):

DIQMTQSPSAMSASVGDRVTITCKASQDIYSYLSWFQQKPGKVPKTLIYRANRLVRGVPSRFSGSGSGQEYTLTISSLQPEDMATYYCLQYDEFPYTFGQGTKLEIK

AM3-ZH3-VH (SEQ ID NO: 72):

EVQLVESGGGLVQPGGSLRLSCAASGFTFSSYPMSWVRQAPGKGLEWVSSISTGASAYFPDSVKGRFTISRDNAKNSLYLQMNSLRAEDTAVYYCARITTSTWYMDVWGQGTTVTVSS

AM3-ZH3-VL (SEQ ID NO: 71):

DIQMTQSPSAMSASVGDRVTITCKASQDIYSYLSWFQQKPGKVPKTLIYRANRLVRGVPSRFSGSGSGQEYTLTISSLQPEDMATYYCLQYDEFPYTFGQGTKLEIK

AM3-ZH4-VH (SEQ ID NO: 73):

EVQLVESGGGLVQPGGSLRLSCAASGFTFSSYAMSWVRQAPGKGLEWVSSISTGASAYFPDSVKGRFTISRDNAKNSLYLQMNSLRAEDTAVYYCARYTTSTWYHDVWGQGTTVTVSS

AM3-ZH4-VL (SEQ ID NO: 71):

DIQMTQSPSAMSASVGDRVTITCKASQDIYSYLSWFQQKPGKVPKTLIYRANRLVRGVPSRFSGSGSGQEYTLTISSLQPEDMATYYCLQYDEFPYTFGQGTKLEIK

AM3-ZH5-VH (SEQ ID NO: 74):

EVQLVESGGGLVQPGGSLRLSCAASGFTFSSYAMSWVRQAPGKGLEWVSSISTGASAYFPDSVKGRFTISRDNAKNSLYLQMNSLRAEDTAVYYCARITTSTWYMDVWGQGTTVTVSS

AM3-ZH5-VL (SEQ ID NO: 75):

DIQMTQSPSAMSASVGDRVTITCKASQDIFSYLSWFQQKPGKVPKTLIYRANRLVSGVPSRFSGSGSGQEYTLTISSLQPEDMATYYCLQYDEFPYTFGQGTKLEIK

AM3-ZH6-VH (SEQ ID NO: 74):

EVQLVESGGGLVQPGGSLRLSCAASGFTFSSYAMSWVRQAPGKGLEWVSSIS TGASAYFPDSVKGRFTISRDNAKNSLYLQMNSLRAEDTAVYYCARITTSTWYMDVW GQGTTVTVSS

AM3-ZH6-VL (SEQ ID NO: 76):

DIQMTQSPSAMSASVGDRVTITCKASQDIYSYLSWFQQKPGKVPKTLIYRAN RLVGGVPSRFSGSGSGQEYTLTISSLQPEDMATYYCLQYDEFPYTFGQGTKLEIK

AM3-ZH7-VH (SEQ ID NO: 74):

EVQLVESGGGLVQPGGSLRLSCAASGFTFSSYAMSWVRQAPGKGLEWVSSIS TGASAYFPDSVKGRFTISRDNAKNSLYLQMNSLRAEDTAVYYCARITTSTWYMDVW GQGTTVTVSS

AM3-ZH7-VL (SEQ ID NO: 71):

DIQMTQSPSAMSASVGDRVTITCKASQDIYSYLSWFQQKPGKVPKTLIYRAN RLVRGVPSRFSGSGSGQEYTLTISSLQPEDMATYYCLQYDEFPYTFGQGTKLEIK

The above variable regions were combined with the human IgG1 heavy chain constant region and the κ light chain constant region (as shown in Example 1) to obtain complete antibodies. The heavy and light chain sequences of the exemplary antibody AM3-ZH-3 obtained by the present disclosure are set forth in SEQ ID NO: 77 and SEQ ID NO: 78, respectively.

Heavy chain sequence of AM3-ZH3 (SEQ ID NO: 77):

EVQLVESGGGLVQPGGSLRLSCAASGFTFSSYPMSWVRQAPGKGLEWVSSIS TGASAYFPDSVKGRFTISRDNAKNSLYLQMNSLRAEDTAVYYCARITTSTWYMDVW GQGTTVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISK AKGQPREPQVYTLPPSREEMTKNQVSLTCLVKGFYPSDIAVEWESNGQPENNYKTTP PVLDSDGSFFLYSKLTVDKSRWQQGNVFSCSVMHEALHNHYTQKSLSLSPGK

Light chain sequence of AM3-ZH3 (SEQ ID NO: 78):

DIQMTQSPSAMSASVGDRVTITCKASQDIYSYLSWFQQKPGKVPKTLIYRAN RLVRGVPSRFSGSGSGQEYTLTISSLQPEDMATYYCLQYDEFPYTFGQGTKLEIKRTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC

**Example** 6: Construction of ROR1-Targeting Biepitopic Antibodies

In this example, ROR1-targeting bispecific antibodies were constructed using a D10-derived ROR1 antibody and an H10-derived ROR1 antibody. The D10-derived ROR1 antibody and the H10-derived ROR1 antibody bind to different epitopes of ROR1, respectively.

In this example, biepitopic antibodies of KIH structures were constructed. Exemplary structures are shown below:

Structure 1 contained the following 4 chains:

chain 1: YR8VH-CH1-Fcknob;

chain 2: YR8VL-CL;

chain 3: ZH3VH-CH1-FcHole; and

chain 4: ZH3VL-CL.

Structure 2 contained the following 4 chains:

chain 1: YR8VH-CH1-Fchole;

chain 2: YR8VL-CL;

chain 3: ZH3VH-CH1-Fcknob; and

chain 4: ZH3VL-CL.

CH1 (SEQ ID NO: 88)

ASTKGPSVFPLAPSSKSTSGGTAALGCLVKDYFPEPVTVSWNSGALTSGVHT FPAVLQSSGLYSLSSVVTVPSSSLGTQTYICNVNHKPSNTKVDKKV

Sequence of Fcknob (T366W) (SEQ ID NO: 79):

EPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCK VSNKALPAPIEKTISKAKGQPREPQVYTLPPSREEMTKNQVSL**W**CLVKGFYPSDIAVE WESNGQPENNYKTTPPVLDSDGSFFLYSKLTVDKSRWQQGNVFSCSVMHEALHNHY TQKSLSLSPGK

Sequence of FcHole (T366S, L368A, and Y407V) (SEQ ID NO: 80):

EPKSCDKTHTCPPCPAPELLGGPSVFLFPPKPKDTLMISRTPEVTCVVVDVSH EDPEVKFNWYVDGVEVHNAKTKPREEQYNSTYRVVSVLTVLHQDWLNGKEYKCK VSNKALPAPIEKTISKAKGQPREPQVYTLPPSREEMTKNQVSL**S**C**A**VKGFYPSDIAVE WESNGQPENNYKTTPPVLDSDGSFFL**V**SKLTVDKSRWQQGNVFSCSVMHEALHNHY TQKSLSLSPGK

To further prepare antibodies that bind to different epitopes of ROR1, the present disclosure also prepared YR-8-ES variant 1, in which the Fc contains only a knob (T366W) mutation, based on antibody YR-8-ES, and prepared AM3-ZH3 variant 1, in which the Fc region contains only hole (T366S, L368A, and Y407V) mutations, based on antibody AM3-ZH3. The sequences are as follows:

Heavy chain of YR-8-ES variant 1 (SEQ ID NO: 81):

QVQLQESGPGLVKPSETLSLTCTVSGFSLTRYGVHWIRQPPGKGLEWLGVIW
AGGFTNYESDLKSRLTISKDNSKNQVSLKLSSVTAADTAVYYCARRGDSYSMKYWG
QGTLVTVSSASTKGPSVFPLAPSSKSTSGGTAALGCLVKDYFPEPVTVSWNSGALTSG
VHTFPAVLQSSGLYSLSSVVTVPSSSLGTQTYICNVNHKPSNTKVDKKVEPKSCDKTH
TCPPCPAPELLGGPSVFLFPPKPKDTLMISRTPEVTCVVVDVSHEDPEVKFNWYVDGV
EVHNAKTKPREEQYNSTYRVVSVLTVLHQDWLNGKEYKCKVSNKALPAPIEKTISKA
KGQPREPQVYTLPPSREEMTKNQVSL**W**CLVKGFYPSDIAVEWESNGQPENNYKTTPP
VLDSDGSFFLYSKLTVDKSRWQQGNVFSCSVMHEALHNHYTQKSLSLSPGK

Light chain of YR-8-ES variant 1 (SEQ ID NO: 31):

EIVLTQSPATLSLSPGERATLSCSASSQVSYIHWYQQKPGQAPRPWIYETSKLA
SGIPARFSGSGSGTDYTLTISSLEPEDFAVYYCQQWNYPLITFGQGTKLEIKRTVAAPSV
FIFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC

Heavy chain of AM3-ZH3 variant 1 (SEQ ID NO: 83):

EVQLVESGGGLVQPGGSLRLSCAASGFTFSSYPMSWVRQAPGKGLEWVSSIS
TGASAYFPDSVKGRFTISRDNAKNSLYLQMNSLRAEDTAVYYCARITTSTWYMDVW
GQGTTVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFNWYVDG
VEVHNAKTKPREEQYNSTYRVVSVLTVLHQDWLNGKEYKCKVSNKALPAPIEKTISK
AKGQPREPQVYTLPPSREEMTKNQVSL**S**C**A**VKGFYPSDIAVEWESNGQPENNYKTTP
PVLDSDGSFFL**V**SKLTVDKSRWQQGNVFSCSVMHEALHNHYTQKSLSLSPGK

Light chain of AM3-ZH3 variant 1 (SEQ ID NO: 78):

DIQMTQSPSAMSASVGDRVTITCKASQDIYSYLSWFQQKPGKVPKTLIYRAN
RLVRGVPSRFSGSGSGQEYTLTISSLQPEDMATYYCLQYDEFPYTFGQGTKLEIKRTVA
APSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC

Illustratively, YR-8-ES antibody variant 1 and AM3-ZH3 antibody variant 1 were separately dissolved in PBS (pH 7.2), then mixed at a molar ratio of 1:1. 2 mM EDTA was added, and the antibody was reduced with tris(2-carboxyethyl) phosphine hydrochloride (the molar ratio of tris(2-carboxyethyl)phosphine hydrochloride to the antibody was 10:1). The mixture was incubated at 25 °C for about 16 h, purified and desalted by elution using G25 resin, and filtered using a 0.2 μm filter under sterile conditions to give KIH YR8/ZH3, an ROR1-targeting biepitopic antibody. Its sequences are shown below:

Chain 1 of KIH YR8/ZH3 (SEQ ID NO: 81):

QVQLQESGPGLVKPSETLSLTCTVSGFSLTRYGVHWIRQPPGKGLEWLGVIWAGGFTNYESDLKSRLTISKDNSKNQVSLKLSSVTAADTAVYYCARRGDSYSMKYWGQGTLVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSL**W**CLVKGFYPSDIAVEWESNGQPENNYKTTPPVLDSDGSFFLYSKLTVDKSRWQQGNVFSCSVMHEALHNHYTQKSLSLSPGK

Chain 2 of KIH YR8/ZH3 (SEQ ID NO: 82):

EIVLTQSPATLSLSPGERATLSCSASSQVSYIHWYQQKPGQAPRPWIYETSKLASGIPARFSGSGSGTDYTLTISSLEPEDFAVYYCQQWNYPLITFGQGTKLEIKRTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC

Chain 3 of KIH YR8/ZH3 (SEQ ID NO: 83):

EVQLVESGGGLVQPGGSLRLSCAASGFTFSSYPMSWVRQAPGKGLEWVSSISTGASAYFPDSVKGRFTISRDNAKNSLYLQMNSLRAEDTAVYYCARITTSTWYMDVWGQGTTVTVSSASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSREEMTKNQVSL**S**C**A**VKGFYPSDIAVEWESNGQPENNYKTTPPVLDSDGSFFL**V**SKLTVDKSRWQQGNVFSCSVMHEALHNHYTQKSLSLSPGK

Chain 4 of KIHYR8/ZH3 (SEQ ID NO: 84):

DIQMTQSPSAMSASVGDRVTITCKASQDIYSYLSWFQQKPGKVPKTLIYRAN RLVRGVPSRFSGSGSGQEYTLTISSLQPEDMATYYCLQYDEFPYTFGQGTKLEIKRTVA APSVFIFPPSDEQLKSGTASVVCLLNNFYPREAKVQWKVDNALQSGNSQESVTEQDS KDSTYSLSSTLTLSKADYEKHKVYACEVTHQGLSSPVTKSFNRGEC

In addition, the present disclosure used UC961 as a positive control. Its sequences are shown below:

Heavy chain of UC961 (SEQ ID NO: 85):

QVQLQESGPGLVKPSQTLSLTCTVSGYAFTAYNIHWVRQAPGQGLEWMGSF DPYDGGSSYNQKFKDRLTISKDTSKNQVVLTMTNMDPVDTATYYCARGWYYFDYW GHGTLVTVSSASTKGPSVFPLAPSSKSTSGGTAALGCLVKDYFPEPVTVSWNSGALTS GVHTFPAVLQSSGLYSLSSVVTVPSSSLGTQTYICNVNHKPSNTKVDKKVEPKSCDKT HTCPPCPAPELLGGPSVFLFPPKPKDTLMISRTPEVTCVVVDVSHEDPEVKFNWYVDG VEVHNAKTKPREEQYNSTYRVVSVLTVLHQDWLNGKEYKCKVSNKALPAPIEKTISK AKGQPREPQVYTLPPSRDELTKNQVSLTCLVKGFYPSDIAVEWESNGQPENNYKTTPP VLDSDGSFFLYSKLTVDKSRWQQGNVFSCSVMHEALHNHYTQKSLSLSPGK

Light chain of UC961 (SEQ ID NO: 86):

DIVMTQTPLSLPVTPGEPASISCRASKSISKYLAWYQQKPGQAPRLLIYSGSTL QSGIPPRFSGSGYGTDFTLTINNIESEDAAYYFCQQHDESPYTFGEGTKVEIKRTVAAPS VFIFPPSDEQLKSGTASVVCLLNNFYPREAKVQWKVDNALQSGNSQESVTEQDSKDS TYSLSSTLTLSKADYEKHKVYACEVTHQGLSSPVTKSFNRGEC

Amino acid sequence of human ROR1 (SEQ ID NO: 87):

MHRPRRRGTRPPLLALLAALLLAARGAAAQETELSVSAELVPTSSWNISSEL NKDSYLTLDEPMNNITTSLGQTAELHCKVSGNPPPTIRWFKNDAPVVQEPRRLSFRST

IYGSRLRIRNLDTTDTGYFQCVATNGKEVVSSTGVLFVKFGPPPTASPGYSDEYEEDG FCQPYRGIACARFIGNRTVYMESLHMQGEIENQITAAFTMIGTSSHLSDKCSQFAIPSL CHYAFPYCDETSSVPKPRDLCRDECEILENVLCQTEYIFARSNPMILMRLKLPNCEDLP QPESPEAANCIRIGIPMADPINKNHKCYNSTGVDYRGTVSVTKSGRQCQPWNSQYPH THTFTALRFPELNGGHSYCRNPGNQKEAPWCFTLDENFKSDLCDIPACDSKDSKEKN KMEILYILVPSVAIPLAIALLFFFICVCRNNQKSSSAPVQRQPKHVRGQNVEMSMLNAY KPKSKAKELPLSAVRFMEELGECAFGKIYKGHLYLPGMDHAQLVAIKTLKDYNNPQQ WTEFQQEASLMAELHHPNIVCLLGAVTQEQPVCMLFEYINQGDLHEFLIMRSPHSDV GCSSDEDGTVKSSLDHGDFLHIAIQIAAGMEYLSSHFFVHKDLAARNILIGEQLHVKIS DLGLSREIYSADYYRVQSKSLLPIRWMPPEAIMYGKFSSDSDIWSFGVVLWEIFSFGL QPYYGFSNQEVIEMVRKRQLLPCSEDCPPRMYSLMTECWNEIPSRRPRFKDIHVRLRS WEGLSSHTSSTTPSGGNATTQTTSLSASPVSNLSNPRYPNYMFPSQGITPQGQIAGFIG PPIPQNQRFIPINGYPIPPGYAAFPAAHYQPTGPPRVIQHCPPPKSRSPSSASGSTSTGHV TSLPSSGSNQEANIPLLPHMSIPNHPGGMGITVFGNKSQKPYKIDSKQASLLGDANIH GHTESMISAEL

**Example** 7: **Preparation of Linker-Drug Compounds**

Toxins (Drug) used for preparing ADCs were conjugated to linkers in advance to prepare linker-drug (LD) compounds LD-1 to LD-17; exemplary toxins were MMAE, eribulin, and exatecan.

The experimental methods without specific conditions indicated in this example were generally conducted under conventional conditions or conditions recommended by the manufacturer of the starting materials or commercial products. The reagents whose sources are not specifically indicated were commercially available conventional reagents.

The structures of the compounds were determined by nuclear magnetic resonance (NMR) spectroscopy or/and mass spectrometry (MS). The NMR shifts ($\delta$) are expressed in $10^{-6}$ (ppm). The $^1$HNMR analyses were performed using a Bruker AVANCE-400 nuclear magnetic resonance instrument, with deuterated dimethyl sulfoxide as a solvent and tetramethylsilane (TMS) as an internal standard.

The MS analyses were performed using a Shimadzu LCMS-2020 Single Quaddrupole liquid chromatography-mass spectrometry system (manufacturer: Shimadzu; MS model: 2020 Single Quadrupole MS); chromatography column: Phenomenex Gemini NX 5 μm, C18, 110 Å, 50 × 4.6 mm; mobile phase: 0.1% formic acid in water/0.1% formic acid in acetonitrile (ACN); flow rate: 1 mL/min.

RP-HPLC used a Shimadzu Nexera preparative liquid chromatography system (chromatography column: Phenomenex Gemini NX 5 μm, C18, 110 Å, 150 × 50 mm; mobile phase: 0.1% trifluoroacetic acid in water/0.1% trifluoroacetic acid in acetonitrile (ACN); flow rate: 50 mL/min).

7.1. Preparation of LD-1

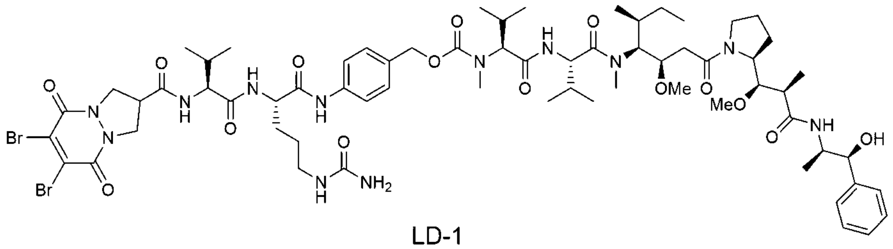

LD-1

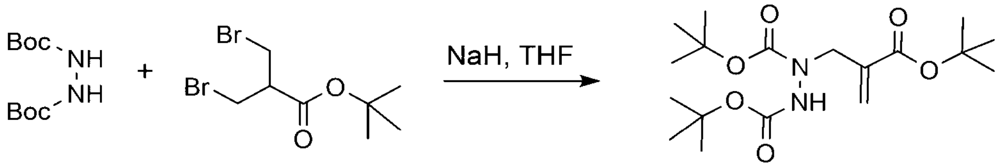


A1

NaH (60%, 80 mg, 2.0 mmol) was added to 3 mL of a solution of 2-tert-butyl hydrazine-1,2-dicarboxylate (76.8 mg, 0.33 mmol) and tert-butyl 3-bromo-2-(bromomethyl)propanoate (200 mg, 0.66 mmol) in anhydrous tetrahydrofuran. The mixture was stirred at room temperature for 15 min, and the reaction was then quenched with 1 mL of water containing 60 μL of AcOH. The mixture was then purified by RP-HPLC. The pure fraction was lyophilized to give A1 (204 mg) as a white solid.

MS m/z: 373.6 [M+H]$^+$.

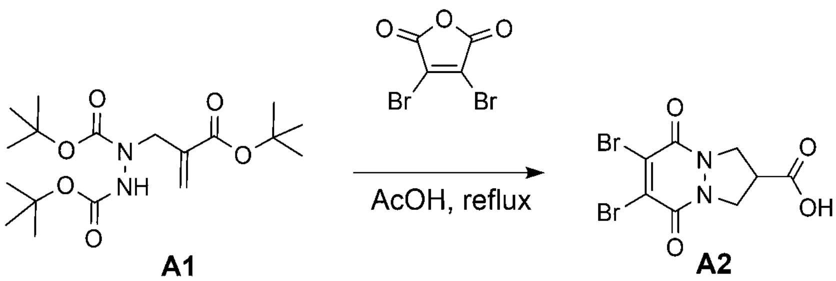


A1 A2

3,4-Dibromofuran-2,5-dione (140 mg, 0.55 mmol) was added to 8.0 mL of a solution of tert-butyl 2-[[tert-butoxycarbonyl-(tert-butoxycarbonylamino)amino]methyl]prop-2-enoate (A1) (204 mg, 0.55 mmol) in acetic acid (AcOH). The mixture was stirred at reflux in an argon atmosphere for 11 days, then concentrated to 3 mL, and purified by RP-HPLC to give A2 (43 mg, yield: 22%) as a white solid.

MS m/z: 354.8 [M+H]$^+$.

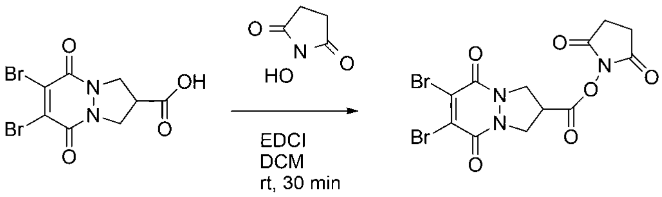


A2 4

N-Hydroxysuccinimide (230 mg) was added to a stirred solution of 6,7-dibromo-5,8-dioxo-2,3,5,8-tetrahydro-1H-pyrazolo[1,2-a]pyridazine-2-carboxylic acid (A2, 350 mg) in anhydrous dichloromethane (10 mL), and N-(3-dimethylaminopropyl)-N'-ethylcarbodiimide hydrochloride (400 mg) was then added. The mixture was stirred at room temperature for 30 min, and the reaction was concentrated to dryness under reduced pressure. The residue was directly purified by RP-HPLC and lyophilized to give compound 4 as a white solid (337 mg).

MS m/z: 452.0 [M+H]$^+$.

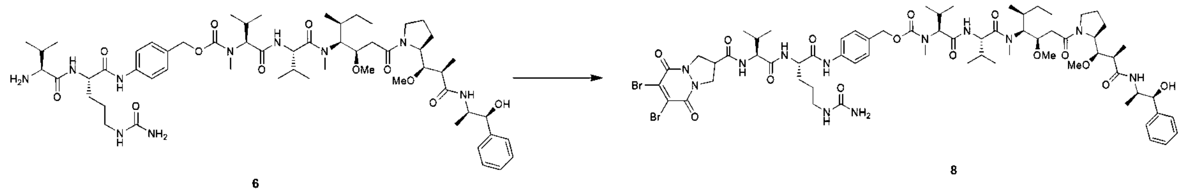

6 8

Compound 4 (12 mg) was added to a solution of compound 6 (31 mg, purchased from MedChemExpress, Cat. No. HY-100374) in DMF (1 mL), and DIEA (0.01 mL) was subsequently added. The reaction mixture was stirred at room temperature (22 °C). After 3 h, the crude reaction mixture was directly purified by RP-HPLC and lyophilized to give compound 8 (17 mg), namely LD-1, as a white solid.

MS m/z: 1243.6 $[M+H]^+$.

7.2. Preparation of LD-2

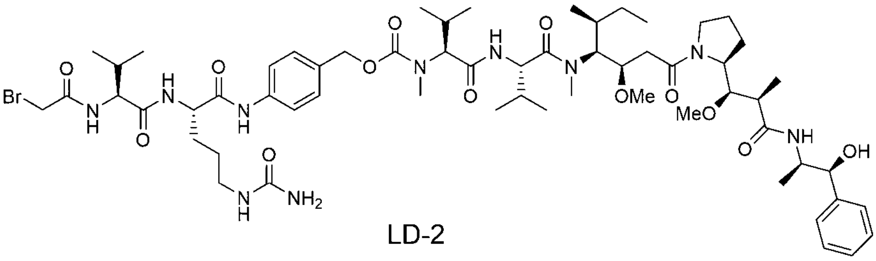

LD-2

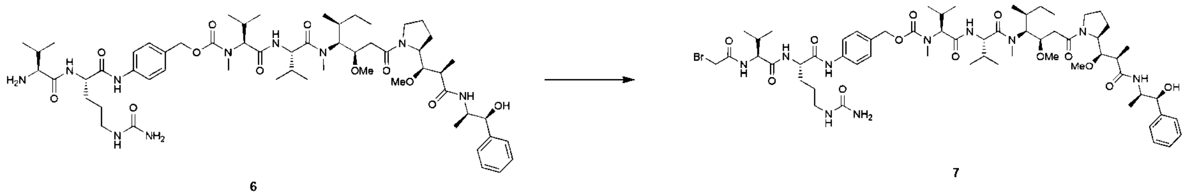

6 7

Saturated $NaHCO_3$ (0.2 mL) and bromoacetic anhydride (9 mg) were added to a solution of compound 6 (20 mg) in acetonitrile/water (6/4, v/v, 2 mL). The reaction mixture was stirred at room temperature (22 °C) for 10 min, then directly purified by RP-HPLC, and lyophilized to give compound 7 (17 mg), namely LD-2, as a white solid.

MS m/z: 1243.6 $[M+H]^+$.

7.3. Preparation of LD-3

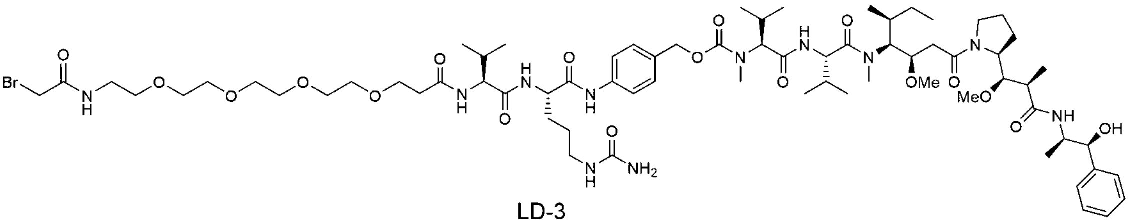

LD-3

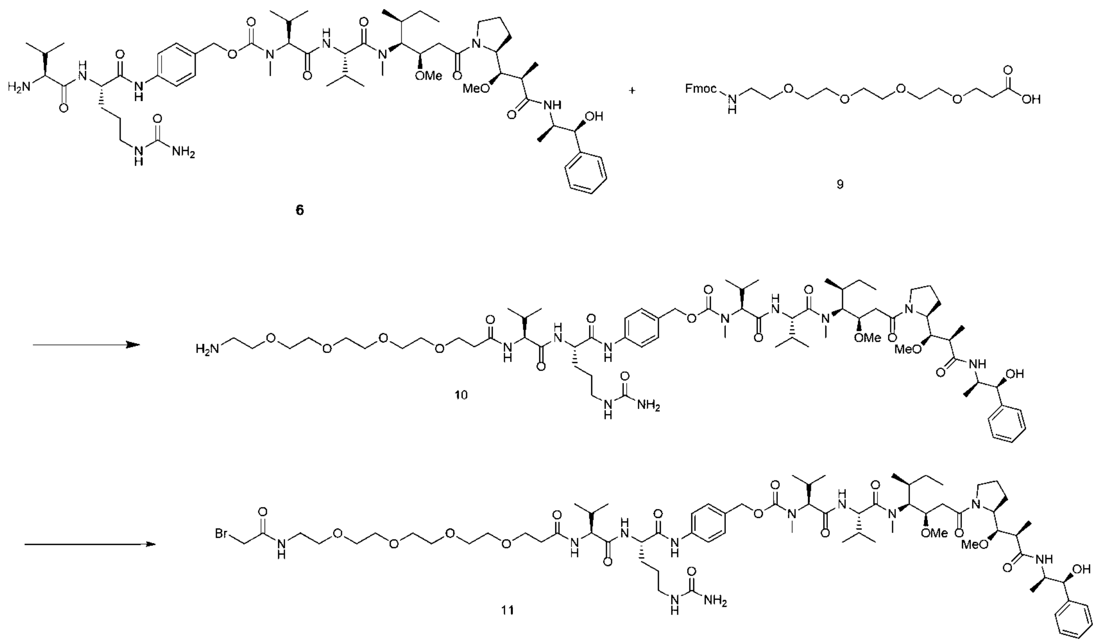

6 + 9 → 10 → 11

DIEA (0.025 mL) was added to a solution of compound 6 (62 mg) and Fmoc-NH-PEG4-COOH (compound 9, 25 mg, purchased from PurePEG, Cat. No. 433704-1H) in anhydrous DMF (1 mL), and HATU (20 mg) was subsequently added. The reaction mixture was stirred at room temperature (22 °C). After 15 min, piperidine (0.1 mL) was added, and the mixture was left to react for another 30 min. The crude reaction mixture was directly purified by RP-HPLC and lyophilized to give compound 10 (61 mg, TFA salt) as a white solid.

Saturated $NaHCO_3$ (0.03 mL) and bromoacetic anhydride (7 mg) were added to a solution of compound 10 (37 mg) in acetonitrile/water (6/4, v/v, 2 mL). After the reaction mixture was stirred at room temperature for 10 min, the crude mixture was purified by RP-HPLC and lyophilized to give compound 11 (32 mg), namely LD-3, as a white solid.

MS m/z: 491.0 $[M+H]^+$.

### 7.4. Preparation of LD-4

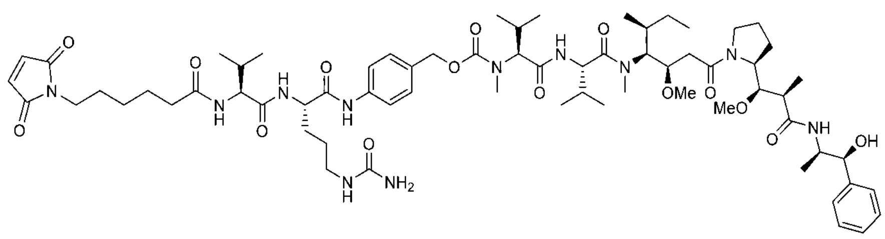

LD-4

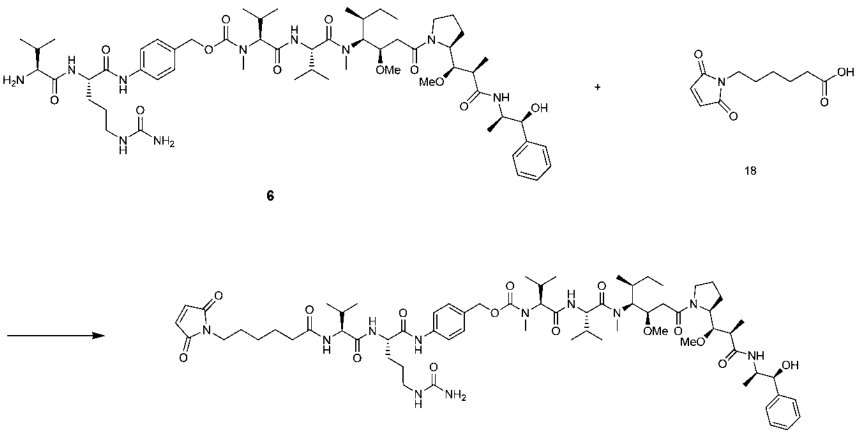

6

18

19

Maleimidohexanoic acid (compound 18, 12 mg) was added to a solution of compound 6 (62 mg, TFA salt) in anhydrous DMF (2 mL), and DIEA (0.02 mL) and HATU (20 mg) were subsequently added. The reaction mixture was stirred at room temperature (22 °C). After 15 min, the crude reaction mixture was directly purified by RP-HPLC and lyophilized to give compound 19 (62 mg, TFA salt), namely LD-4, as a white solid.

MS m/z: 1316.6 $[M+H]^+$.

7.5. Preparation of LD-5

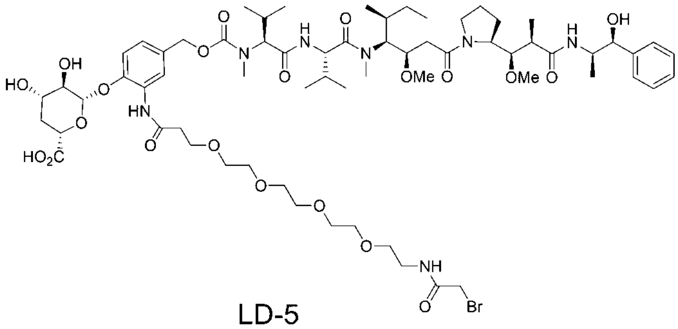

LD-5

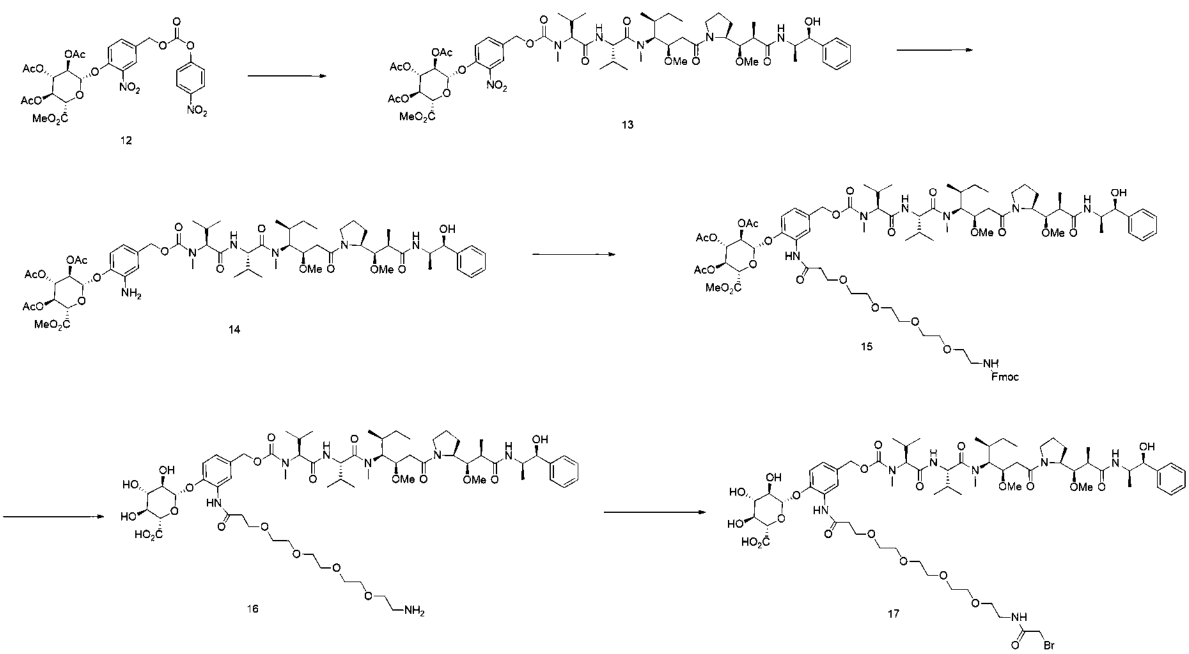

12 13 14 15 16 17

DIEA (0.002 mL) was added to a solution of compound 12 (65 mg, prepared according to reference WO2022026915) and MMAE (72 mg) in anhydrous DMF (2 mL), and HOBt (3 mg) was subsequently added. The reaction mixture was stirred at room temperature (22 °C) for 18 h and then diluted with water (20 mL). The reaction mixture was extracted with diethyl ether (40 mL). The organic phase was dried over $Na_2SO_4$ and concentrated to dryness under reduced pressure to give crude compound 13, which was finally dissolved in methanol (2 mL). Zinc powder (200 mg) was added to the solution of compound 13 in methanol, and formic acid (0.2 mL) was then added. The reaction was stirred at room temperature for 30 min. The solid was removed by filtration, and the filtrate was directly purified by RP-HPLC and lyophilized to give compound 14 (72 mg) as a white solid. DIEA (0.025 mL) was added to a solution of compound 14 (TFA salt, 66 mg) and Fmoc-NH-PEG4-COOH (purchased from PurePEG, Cat. No. 433704, 25 mg) in anhydrous DMF (2 mL), and HATU (20 mg) was then added. The mixture was stirred at room temperature. After 16 h, the crude mixture was purified by RP-HPLC and lyophilized to give compound 15 (72 mg) as a white powder.

Compound 15 (70 mg) was dissolved in a solution of acetonitrile/water (6/4, v/v, 3 mL), and NaOH (aq., 1 M, 0.3 mL) was added. The reaction mixture was stirred at room temperature (22 °C) to give compound 16. After 8 h, hydrochloric acid (1 M, 0.12 mL) was added to the crude product of compound 16, and bromoacetic anhydride (14 mg) was then added. The mixture was left to react for 0.5 h. The crude reaction mixture was directly purified by RP-HPLC and lyophilized to give compound 17 (46 mg), namely LD-5, as a white solid.

MS m/z: 1426.7 $[M+H]^+$.

### 7.6. Preparation of LD-6

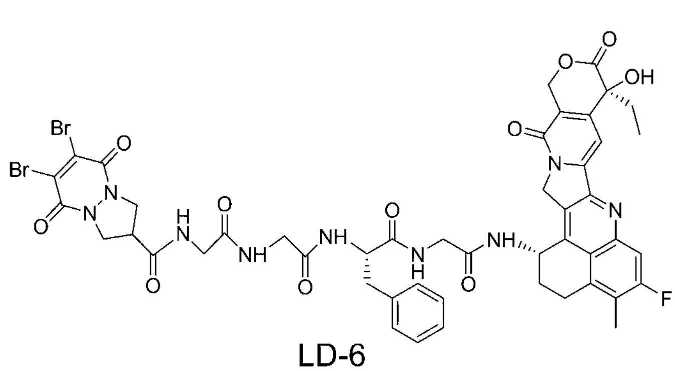

LD-6

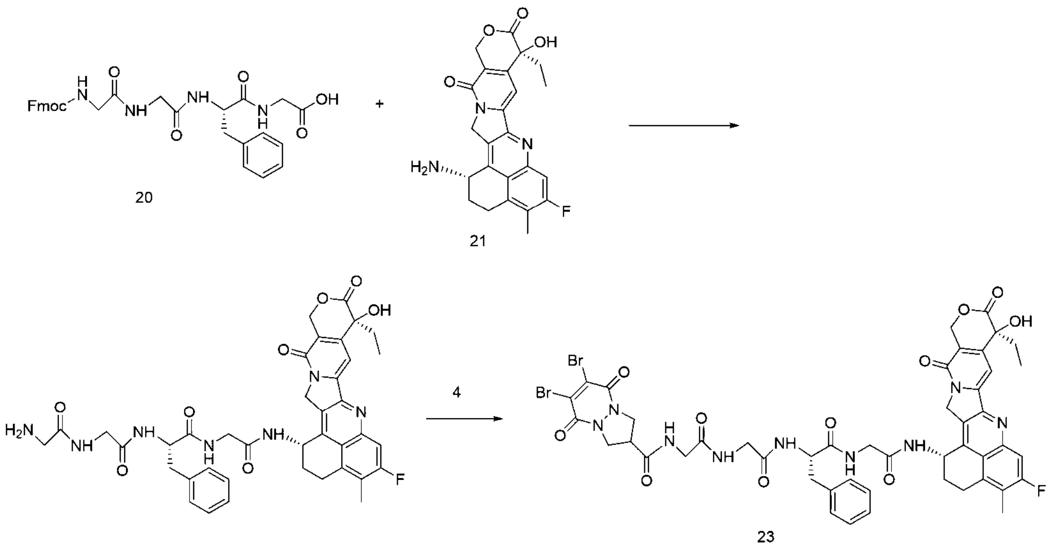


20

21

22

23

N-Ethyldiisopropylamine (0.05 mL) was added to a solution of compound 20 (60 mg, purchased from InnoPep) and exatecan mesylate (compound 21, 53 mg, purchased from Advance ChemBlocks, Cat. No. 10484) in anhydrous N,N-dimethylformamide (2 mL), and O-(7-azabenzotriazol-1-yl)-N,N,N',N'-tetramethyluronium hexafluorophosphate (38 mg) was then added. The mixture was stirred at room temperature for 15 min. Piperidine (0.1 mL) was added. After 30 min, the crude mixture was purified by RP-HPLC and lyophilized to give compound 22 (67 mg, trifluoroacetate) as a yellow powder.

Compound 4 (25 mg, synthesized using the method described in PCT/US2022/078563) was added to a solution of compound 22 (44 mg) in N,N-dimethylformamide (2 mL), and N-ethyldiisopropylamine (0.02 mL) was then added. The reaction mixture was stirred at room temperature. After 1 h, the crude mixture was purified by RP-HPLC and lyophilized to give compound 23, namely LD-6, as a yellow solid (42 mg).

MS m/z: 1090.2 $[M+H]^+$.

7.7. **Preparation of LD-14**

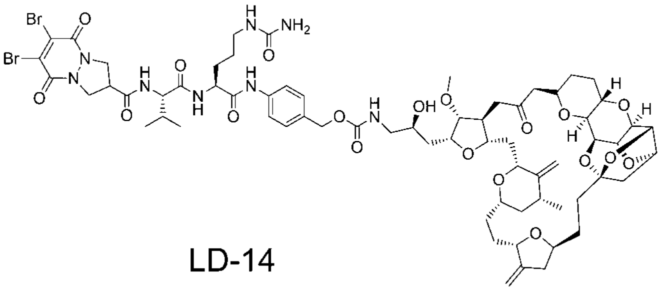

LD-14

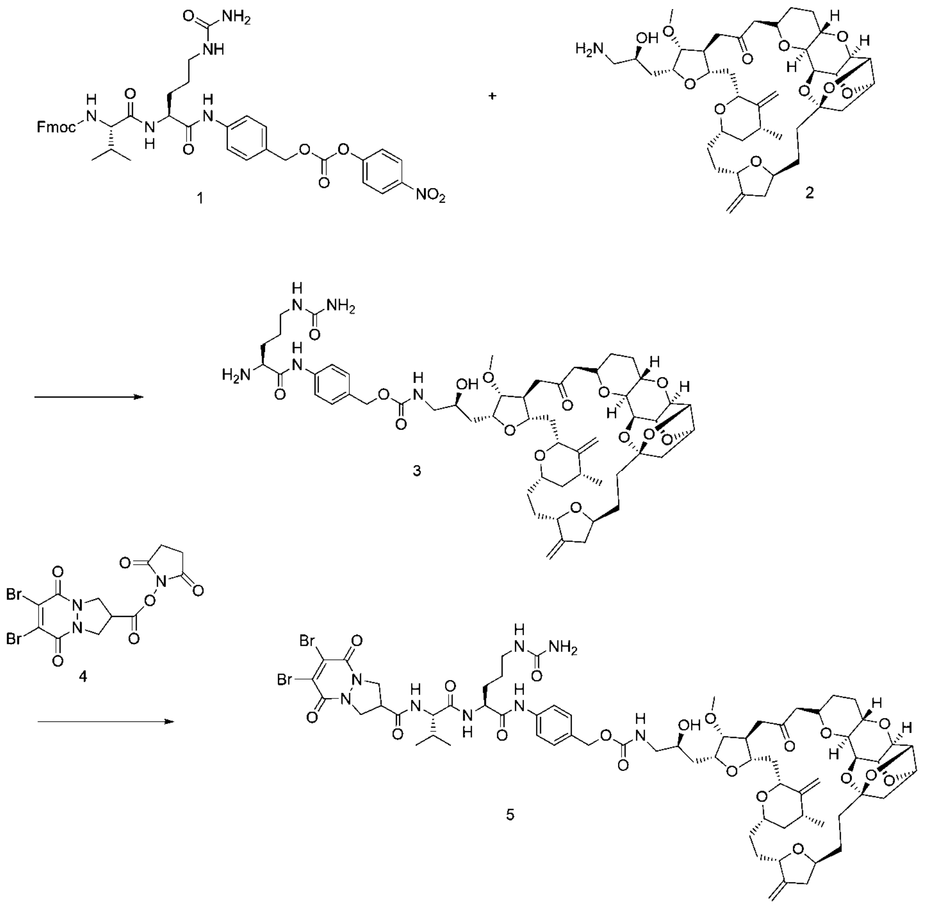

1 + 2 → 3 → 4 → 5

Eribulin (compound 2, mesylate, 82 mg, MedChemExpress, HY-13442) was added to a solution of compound 1 (77 mg, MedChemExpress, HY-41189) in anhydrous N,N-dimethylformamide (2 mL), and N-ethyldiisopropylamine (0.035 mL) was then added. The reaction mixture was stirred at room temperature (22 °C). After 6 h, piperidine (0.1 mL) was added, and the reaction was stirred at room temperature for 15 min. The reaction mixture was directly purified by RP-HPLC and lyophilized to give compound 3 (110 mg, trifluoroacetate) as a white solid.

Compound 4 (20 mg, synthesized using the method described in PCT/US2022/078563) was added to a solution of compound 3 (50 mg) in N,N-dimethylformamide (2 mL), and N-ethyldiisopropylamine (0.01 mL) was then added. The reaction mixture was stirred at room temperature. After 3 h, the crude mixture was purified by RP-HPLC and lyophilized to give compound 5 (43 mg), namely LD-14, as a white solid.

MS m/z: 1471.6 $[M+H]^+$.

$^1$HNMR (400 MHz, DMSO) δ: 10.00 (s, 1H), 8.36 (d, J = 8.8 Hz, 1H), 8.25 (d, J = 7.6 Hz, 1H), 7.57 (d, J = 8.4 Hz, 2H), 7.27 (d, J = 8.4 Hz, 2H), 7.08 (t, J = 6 Hz, 1H), 5.97 (t, J = 5.6 Hz, 1H), 5.41 (s, 2H), 5.05 (s, 1H), 5.00 (s, 1H), 4.97-4.89 (m, 2H), 4.83 (s, 1H), 4.75 (s, 1H), 4.63 (s, 1H), 4.55 (dd, J = 7.2, 4.4 Hz, 2H), 4.38 (dd, J = 13.2 Hz, J = 7.6 Hz, 1H), 4.32-4.21 (m, 4H), 4.21-4.12 (m, 3H), 4.22-4.06 (m, 3H), 4.02 (t, J = 7.6 Hz, 1H), 3.86-3.73 (m, 2H), 3.73-3.62 (m, 2H), 3.58-3.45 (m, 3H), 3.30 (s, 1H), 3.28-3.18 (m, 4H), 3.06-2.90 (m, 4H), 2.84 (d, J = 9.6 Hz, 1H), 2.80-2.63 (m, 2H), 2.61-2.52 (m, 1H), 2.37-2.17 (m, 5H), 2.17-2.08 (m, 1H), 2.06-1.85 (m, 7H), 1.78-1.54 (m, 7H), 1.54-1.39 (m, 4H), 1.39-1.26 (m, 4H), 1.24-1.11 (m, 1H), 1.03 (d, J = 6.4 Hz, 3H), 1.01-0.92 (m, 1H), 0.86 (dd, J = 12.4, J = 6.8 Hz, 6H).

7.8. Preparation of LD-7

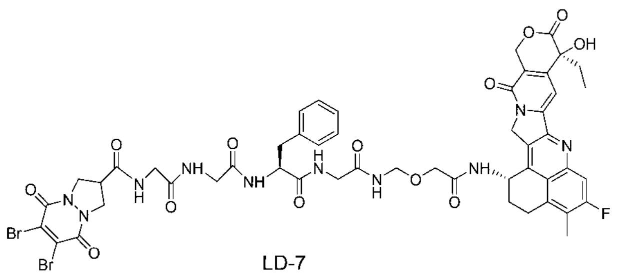

LD-7

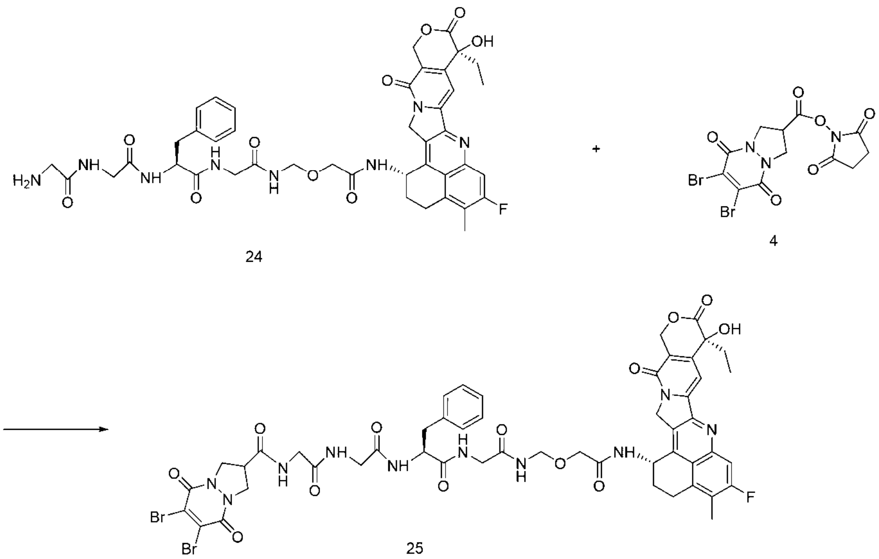

24

4

25

Compound 4 (12 mg) was added to a solution of compound 24 (21 mg, synthesized using the procedure described in US 10155821B2) in N,N'-dimethylformamide (1 mL), and diisopropylethylamine (10 μL) was then added. The reaction mixture was stirred at room temperature. After 1 h, the crude mixture was purified by RP-HPLC and lyophilized to give compound 25 (namely LD-7, 23 mg) as a yellow solid.

MS m/z:1177.4 $[M+H]^+$.

7.9. Preparation of LD-8

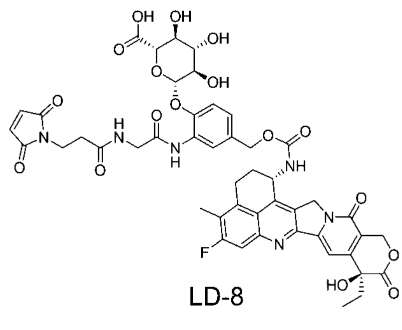

LD-8

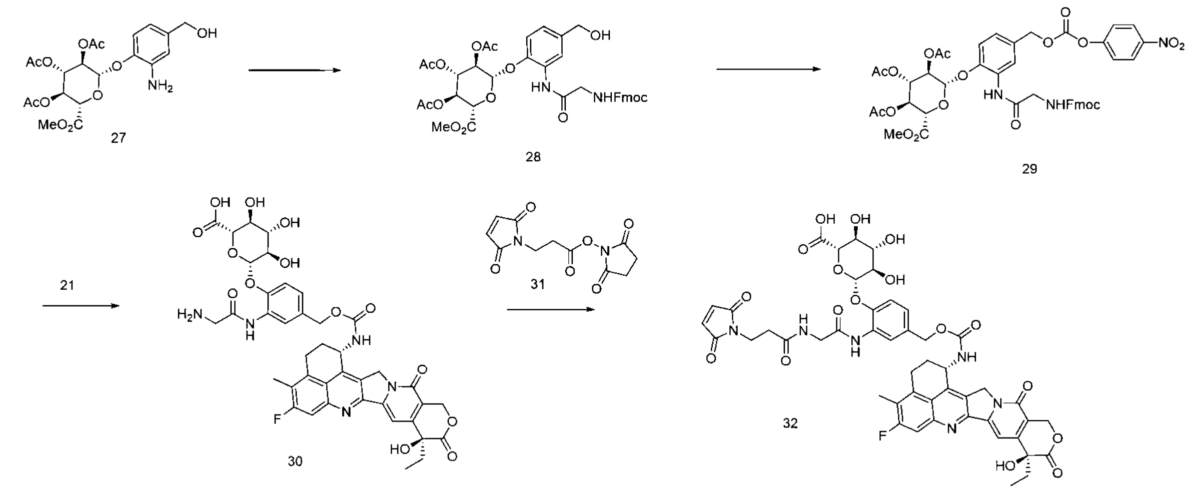

27 28 29 30 32

Diisopropylethylamine (0.1 mL) was added to a solution of compound 27 (92 mg, 0.2 mmol, purchased from ChemScene, CS-0105172) and Fmoc-Gly-OH (90 mg, purchased from Combi-Blocks, San Diego) in dimethylformamide (3 mL), and HATU (114 mg, 0.3 mmol) was then added. The reaction mixture was stirred at room temperature for 6 h, and the crude product was purified by RP-HPLC and lyophilized to give compound 28 as a white powder (76 mg).

Compound 28 (73 mg) was dissolved in anhydrous dimethylformamide (1 mL). Bis(4-nitrophenol) carbonate (Sigma-Aldrich, 45 mg, 0.15 mmol) was added, and diisopropylethylamine (0.02 mL) was then added. The mixture was stirred at room temperature for 16 h, and the crude reaction mixture was directly purified by RP-HPLC to give compound 29 as a white powder (75 mg).

Diisopropylethylamine (0.02 mL) was added to a solution of compound 29 (54 mg) and exatecan mesylate (compound 21, exatecan mesylate, 27 mg) in anhydrous dimethylformamide (1 mL). The mixture was stirred at room temperature for 3 h and then diluted with water (20 mL). The mixture was extracted with ethyl acetate (40 mL), and the organic layer was dried over sodium sulfate and concentrated to dryness under reduced pressure. The residue was suspended in acetonitrile/water (2 mL, 6/4, v/v). Sodium hydroxide (aqueous, 1 M, 0.35 mL) was added to the solution, and the mixture was stirred at room temperature for 2 h. Hydrochloric acid (1 N, 0.2 mL) was added, and the reaction mixture was purified by RP-HPLC to give compound 30 (33 mg, TFA salt) as a yellow powder.

Compound 31 (10 mg, purchased from Ambeed) was added to a solution of compound 30 (32 mg) in dimethylformamide (1 mL), and diisopropylethylamine (20 μL) was then added. The reaction mixture was stirred at room temperature. After 1 h, the crude mixture was purified by RP-HPLC and lyophilized to give compound 32 (namely LD-8, 28 mg) as a yellow solid.

MS m/z: 985.4 $[M+H]^+$.

### 7.10. Preparation of LD-9

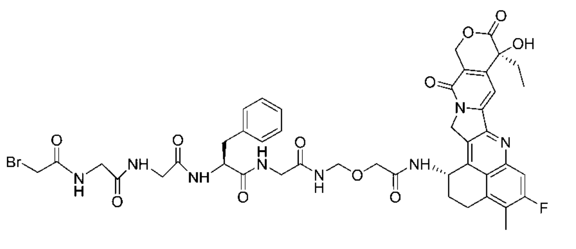

LD-9

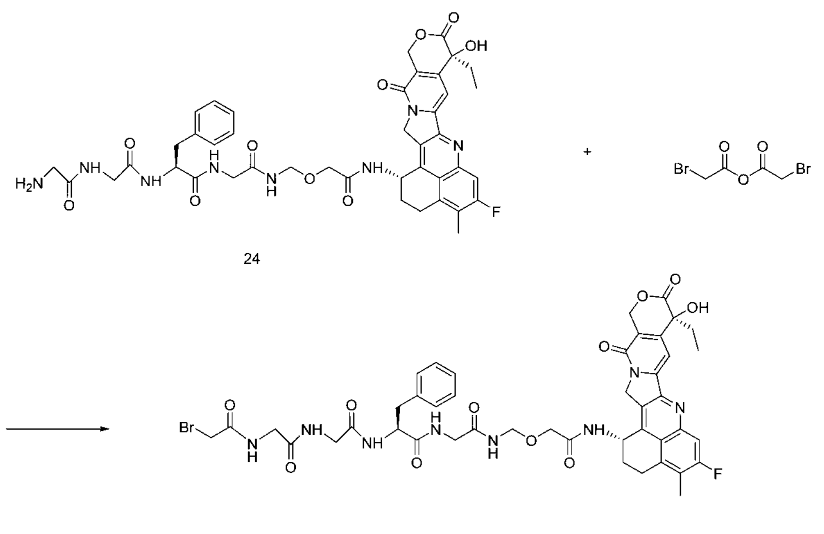


24

26

A saturated aqueous sodium bicarbonate solution (0.02 mL) and bromoacetic anhydride (10 mg) were added to a solution of compound 24 (21 mg, synthesized using the procedure described in US10155821 B2) in acetonitrile/water (6/4, v/v, 1 mL). The reaction mixture was stirred at room temperature for 15 min, and the mixture was directly purified by RP-HPLC to give compound 26 (19 mg) as a yellow powder.
MS m/z: m/z 961.4 $[M+H]^+$.

7.11. Preparation **of LD-10**

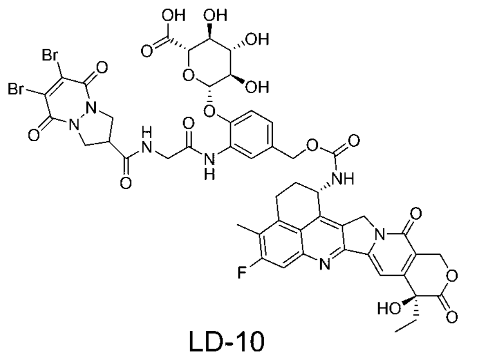

LD-10

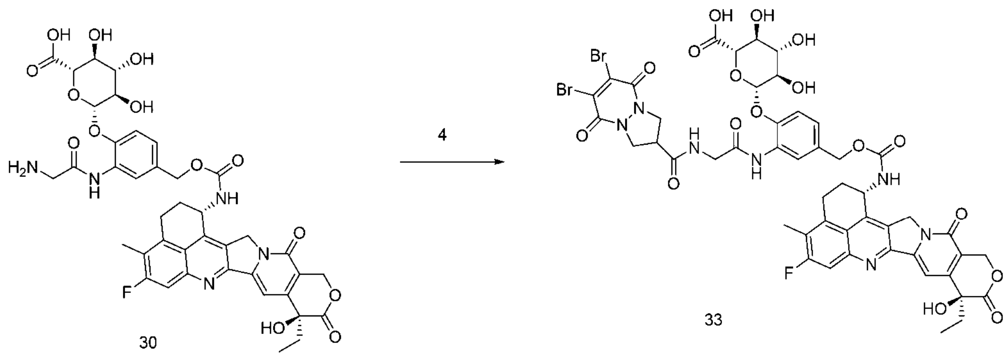


30

33

Compound 4 (12 mg, see the preparation process of LD-7) was added to a solution of compound 30 (20 mg) in dimethylformamide (1 mL), and diisopropylethylamine (10 μL) was then added. The reaction mixture was stirred at room temperature. After 1 h, the crude mixture was purified by RP-HPLC and lyophilized to give compound 33 (namely LD-10, 19 mg) as a yellow solid.

MS m/z: 1170.4 $[M+H]^+$.

**7.12. Preparation of LD-11**

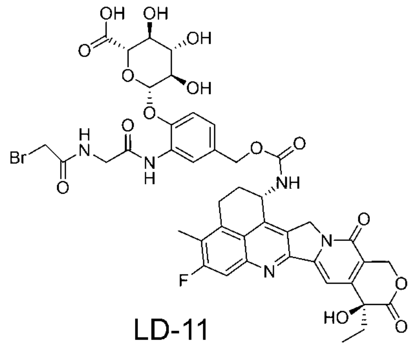

LD-11

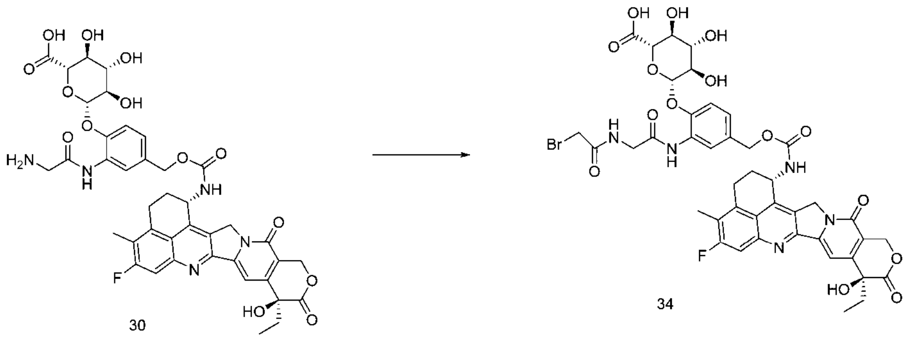

30

34

A saturated aqueous sodium bicarbonate solution (0.02 mL) and bromoacetic anhydride (10 mg) were added to a solution of compound 30 (19 mg) in acetonitrile/water (6/4, v/v, 1 mL). The reaction mixture was stirred at room temperature for 15 min, and the mixture was directly purified by RP-HPLC to give compound 34 (namely LD-11, 17 mg) as a yellow powder.

MS m/z: 954.2 $[M+H]^+$.

**7.13. Preparation of LD-12**

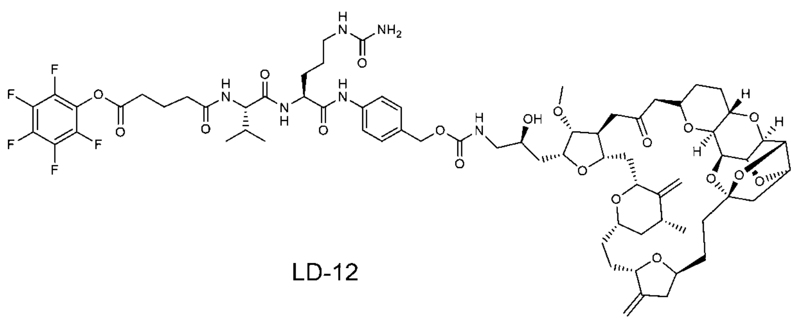

LD-12

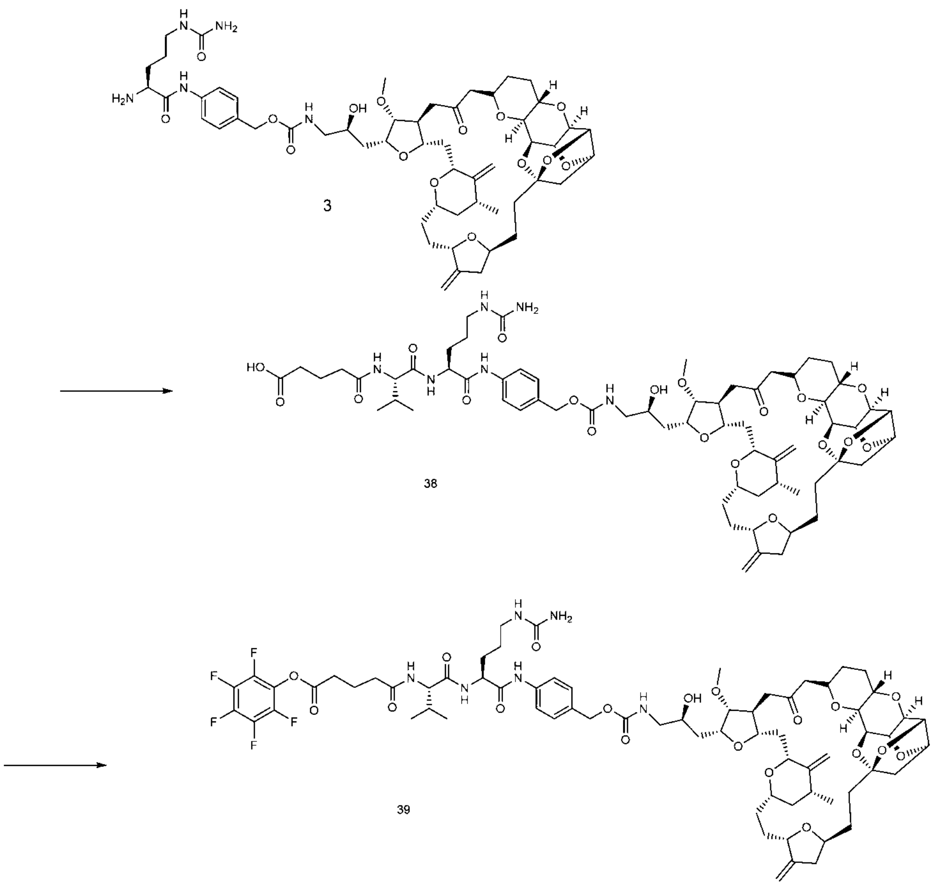

3

38

39

Cyclopentane-1,2-dicarboxylic anhydride (5 mg) was added to a solution of compound 3 (25 mg) in anhydrous dimethylformamide (1 mL), and diisopropylethylamine (0.01 mL) was then added. The reaction mixture was stirred at room temperature for 2 h and then diluted with DCM (4 mL). Pentafluorophenol (10 mg) and 1-ethyl-3-(3-dimethylaminopropyl) carbodiimide hydrochloride (20 mg) were added, and the mixture was stirred at room temperature. After 30 min, the reaction mixture was concentrated under reduced pressure, and the residue was purified by RP-HPLC to give compound 39 (namely LD-12, 23 mg) as a white powder.

MS m/z: 1415.7 $[M+H]^+$.

### 7.14. Preparation of LD-13

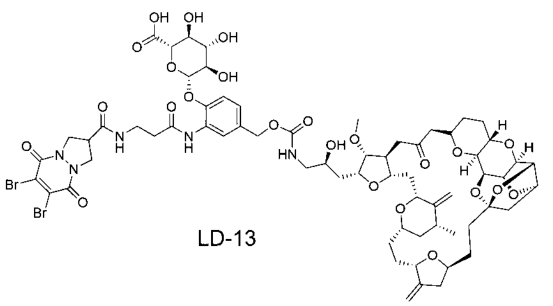

LD-13

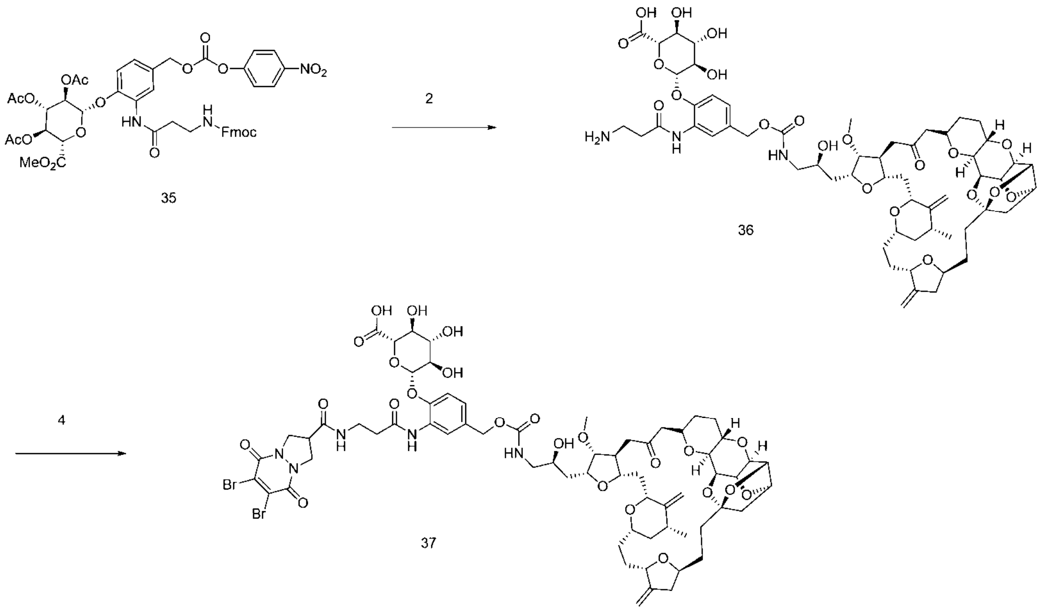


35

36

37

Diisopropylethylamine (0.04 mL) was added to a solution of compound 35 (92 mg, synthesized using the procedure described in Bioconjugate Chem., 2006, 17(3), 831-840) and eribulin mesylate (2, 82 mg) in anhydrous dimethylformamide (2 mL). The mixture was stirred at room temperature for 3 h and then diluted with water (30 mL). The mixture was extracted with ethyl acetate (50 mL), and the organic layer was dried over sodium sulfate and concentrated to dryness under reduced pressure. The residue was dissolved in acetonitrile/water (4 mL, 6/4, v/v). Sodium hydroxide (aqueous, 1 M, 0.7 mL) was added to the solution, and the mixture was stirred at room temperature for 2 h. Hydrochloric acid (1 N, 0.4 mL) was added, and the reaction mixture was purified by RP-HPLC to give compound 36 as a white powder (97 mg, TFA salt).

Compound 4 (6 mg) was added to a solution of compound 36 (12 mg) in dimethylformamide (1 mL), and diisopropylethylamine (5 μL) was then added. The reaction mixture was stirred at room temperature. After 1 h, the crude mixture was purified by RP-HPLC and lyophilized to give compound 37 (namely LD-13, 12 mg) as a white solid.

MS m/z: 1478.6 $[M+H]^+$.

### 7.15. Preparation of LD-15

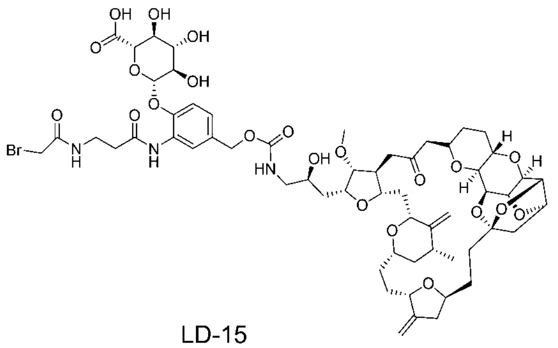

LD-15

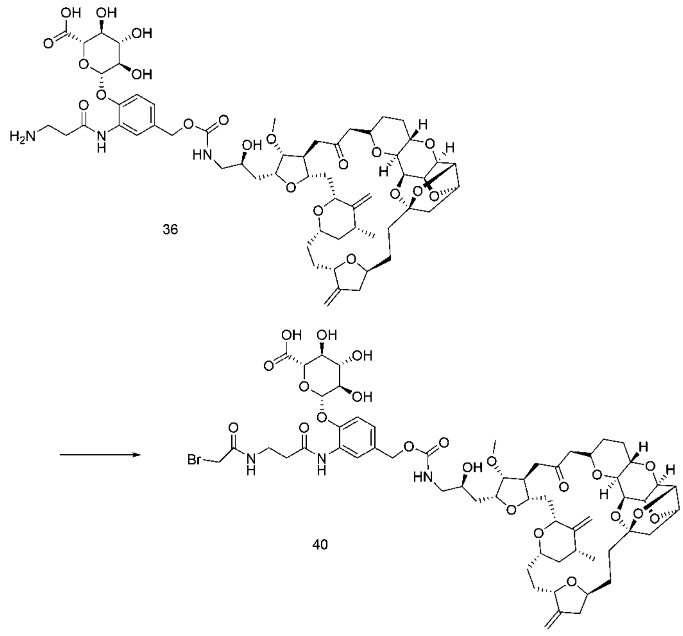

36

40

A saturated aqueous sodium bicarbonate solution (0.01 mL) and bromoacetic anhydride (5 mg) were added to a solution of compound 36 (13 mg) in acetonitrile/water (6/4, v/v, 1 mL). The reaction mixture was stirred at room temperature for 15 min, and the mixture was directly purified by RP-HPLC to give compound 40 (namely LD-15) as a white powder (10 mg).

MS m/z: 1262.2 $[M+H]^+$.

### 7.16. Preparation of LD-16

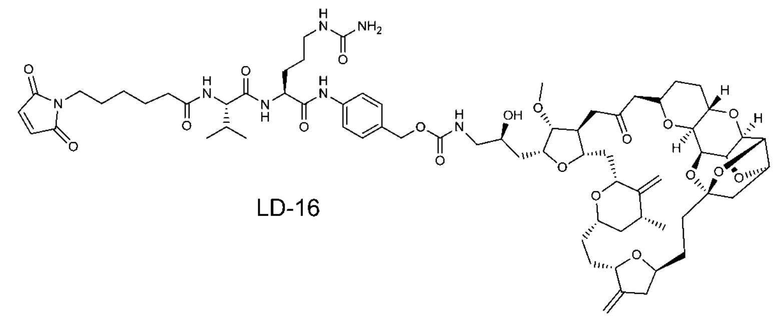

LD-16

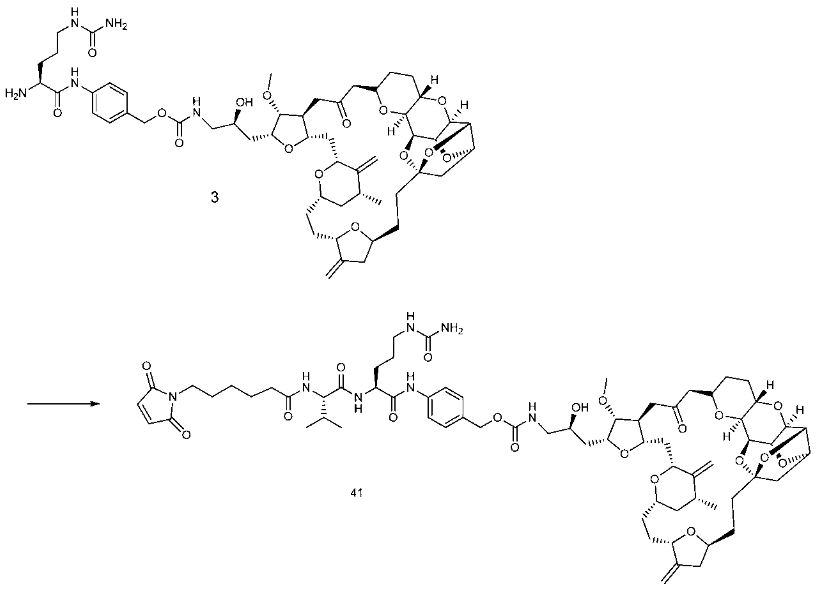

3

41

6-Maleimidohexanoic acid N-hydroxysuccinimide ester (5 mg, Sigma-Aldrich) was added to a solution of compound 3 (12 mg) in dimethylformamide (1 mL), and diisopropylethylamine (5 μL) was then added. The reaction mixture was stirred at room temperature. After 1 h, the crude mixture was purified by RP-HPLC and lyophilized to give compound 41 (namely LD-16, 11 mg) as a white solid.

MS m/z: 1328.8 $[M+H]^+$.

### 7.17. Preparation of LD-17

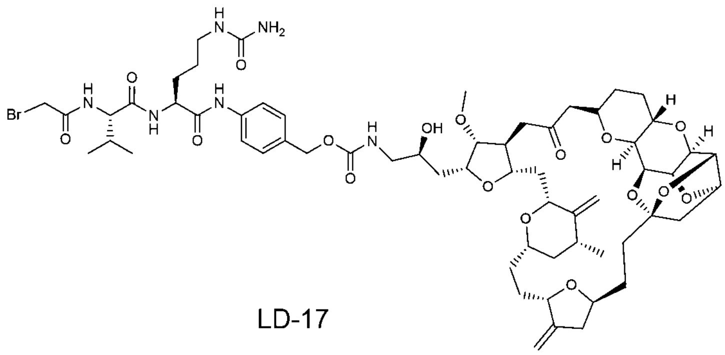

LD-17

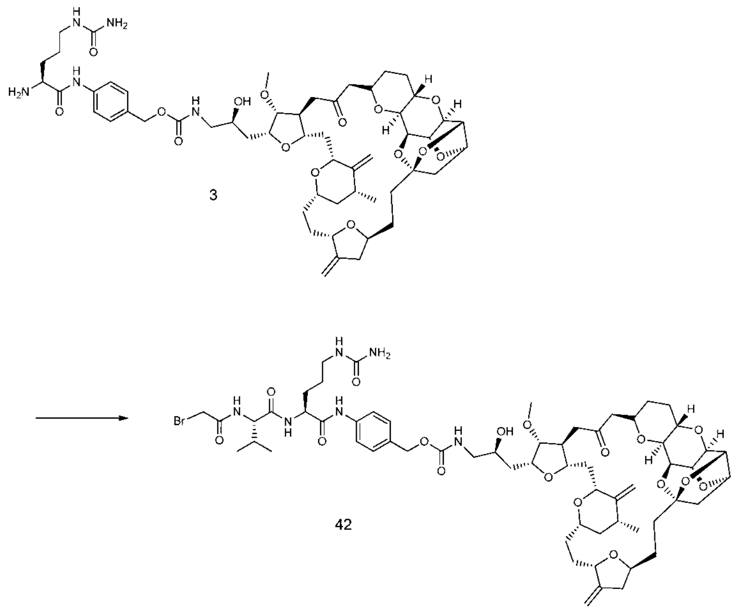

3

42

A saturated aqueous sodium bicarbonate solution (0.01 mL) and bromoacetic anhydride (5 mg) were added to a solution of compound 3 (13 mg) in acetonitrile/water (6/4, v/v, 1 mL). The reaction mixture was stirred at room temperature for 15 min, and the mixture was directly purified by RP-HPLC to give compound 42 (namely LD-17, 9 mg) as a white powder.

MS m/z: 1255.6 $[M+H]^+$.

**Example 8: Preparation of ADCs**

The antibodies of the present disclosure were reacted with the linker-drug (LD) compounds to prepare ADC drugs, and the DAR values of the prepared ADCs were determined.

In short, the antibodies were treated with a reducing agent (e.g., tris(2-carboxyethyl)phosphine hydrochloride (TCEP) or dithiothreitol (DTT)) to reduce some or all of the cysteine disulfide residues to form cysteine sulfhydryl ($-CH_2SH$), which is highly nucleophilic. Thus, the partially or fully reduced antibodies were reacted with the linker-drug compounds or linker reagents and electrophilic functional groups (e.g., maleimide or acetyl bromide) to finally obtain the ADCs.

Method of determining DAR values of ADCs:

The DAR values of the ADCs of the present disclosure were analyzed using hydrophobic interaction chromatography-high performance liquid chromatography (HIC-HPLC). The ADCs were separated in a chromatography column using an MabPac HIC-Butyl analytical column (4.6 × 100 mm, 5 μm, Cat. No. 088558, ThermoFisher, USA). A 25 mM sodium phosphate buffer (pH 6.8) containing 1.5 M ammonium sulfate was used as buffer solution A, a 25 mM sodium phosphate buffer (pH 6.8) containing 25% acetonitrile was used as buffer B, and 85% A and 15% B were stabilized as initial conditions. Linear gradient elution was performed for 30 min using 85% A and 15% B compared to 5% A and 95% B, and elution was performed for an additional 5 min using 5% A and 95% B. The flow rate and the temperature were set to 0.5 mL/min and 25 °C. The ADC drug distribution was determined at 214 nm and 280 nm and used to calculate the DAR values.

**8.1. Preparation of ADC-1**

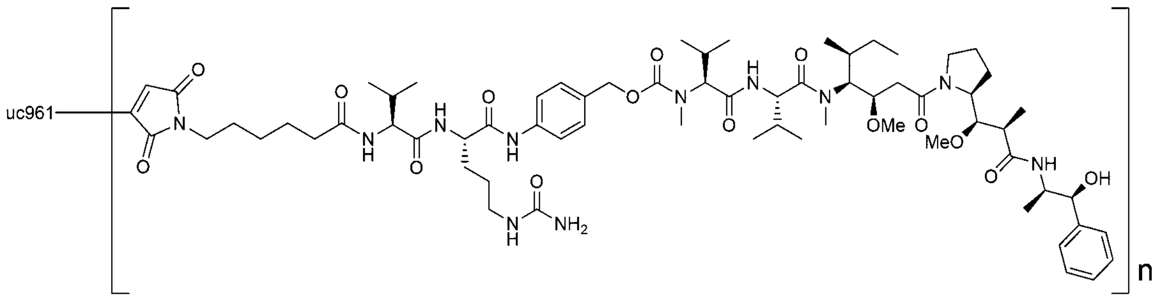

**ADC-1**

Antibody UC961 was dissolved in PBS (pH 7.2), 2 mM EDTA was added, and the antibody was reduced with tris(2-carboxyethyl)phosphine hydrochloride (the molar ratio of tris(2-carboxyethyl)phosphine hydrochloride to the antibody was 2.8:1). After the mixture was incubated at 37 °C for about 120 min, linker-drug compound LD-4 was added to the reduced antibody (the molar ratio of the linker-drug compound to the antibody was 5:1). After 1 h of incubation at room temperature, the mixture was purified and desalted by elution using G25 resin and filtered using a 0.2 μm filter under sterile conditions to give ADC-1, which was then cryopreserved. The average DAR value of ADC-1 was determined to be between 3.7 and 4.3 using the hydrophobic interaction chromatography-high performance liquid chromatography analysis described above.

**8.1. Preparation of ADC-2 (preparation of monoclonal antibody conjugate drug)**

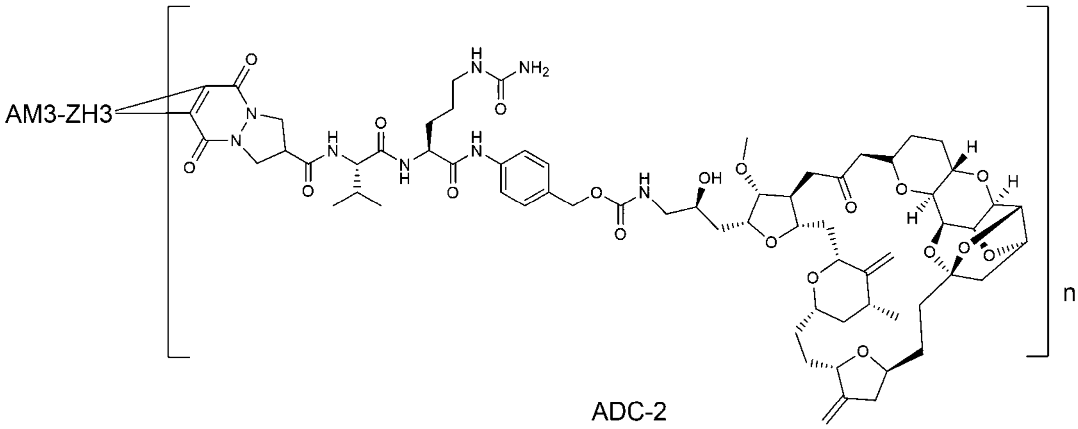

ADC-2

Antibody **AM3-ZH3** was dissolved in PBS (pH 7.2), 2 mM EDTA was added, and the antibody was reduced with tris(2-carboxyethyl)phosphine hydrochloride (the molar ratio of tris(2-carboxyethyl)phosphine hydrochloride to the antibody was 10:1). After the mixture was incubated at 37 °C for about 120 min, linker-drug compound LD-14 was added to the reduced antibody (the molar ratio of the linker-drug compound to the antibody was 5:1). After 1 h of incubation at room temperature, the mixture was purified and desalted by elution using G25 resin and filtered using a 0.2 μm filter under sterile conditions to give ADC-2, which was then cryopreserved. The average DAR value of ADC-2 was determined to be between 3.7 and 4.3 using the hydrophobic interaction chromatography-high performance liquid chromatography analysis described above.

**8.2. Preparation of ADC-3 (preparation of bispecific antibody conjugate drug)**

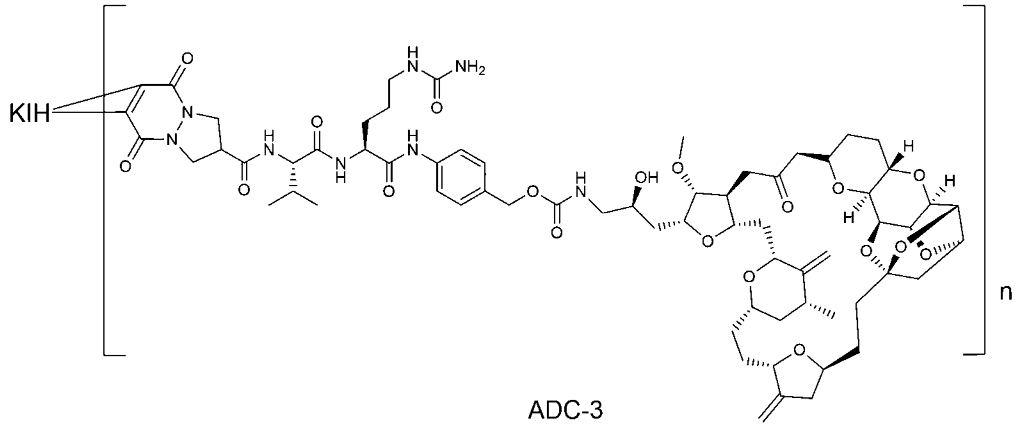

ADC-3

YR-8-ES antibody variant 1 and AM3-ZH3 antibody variant 1 were separately dissolved in PBS (pH 7.2), and the resulting solutions were first mixed at a molar ratio of 1:1. 2 mM EDTA was added, and the antibody was reduced with tris(2-carboxyethyl)phosphine hydrochloride (the molar ratio of tris(2-carboxyethyl)phosphine hydrochloride to the antibody was 10:1). After the mixture was incubated at 25 °C for about 16 h, linker-drug compound LD-14 was added to the reduced antibody KIHYR8/ZH3 (the molar ratio of the linker-drug compound to the antibody KIHYR8/ZH3 was 5:1). After 1 h of incubation at room temperature, the mixture was purified and desalted by elution using G25 resin and filtered using a 0.2 μm filter under sterile conditions to give ADC-3, which was then cryopreserved. The average DAR value of ADC-3 was determined to be between 3.7 and 4.3 using the hydrophobic interaction chromatography-high performance liquid chromatography analysis described above.

**8.3. Preparation of ADC-4**

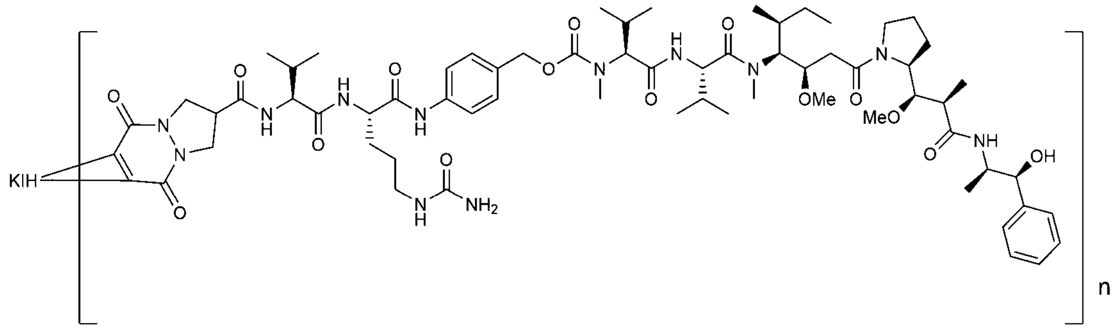

ADC-4

YR-8-ES antibody variant 1 and AM3-ZH3 antibody variant 1 were separately dissolved in PBS (pH 7.2), and the resulting solutions were first mixed at a molar ratio of 1:1. 2 mM EDTA was added, and the antibody was reduced with tris(2-carboxyethyl)phosphine hydrochloride (the molar ratio of tris(2-carboxyethyl)phosphine hydrochloride to the antibody was 10:1). After the mixture was incubated at 25 °C for about 16 h, linker-drug compound LD-1 was added to the reduced antibody KIHYR8/ZH3 (the molar ratio of the linker-drug compound to the antibody KIHYR8/ZH3 was 5:1). After 1 h of incubation at room temperature, the mixture was purified and desalted by elution using G25 resin and filtered using a 0.2 μm filter under sterile conditions to give ADC-4, which was then cryopreserved. The average DAR value of ADC-4 was determined to be between 3.7 and 4.3 using the hydrophobic interaction chromatography-high performance liquid chromatography analysis described above.

**8.5. Preparation of ADC-5 (preparation of monoclonal antibody conjugate drug)**

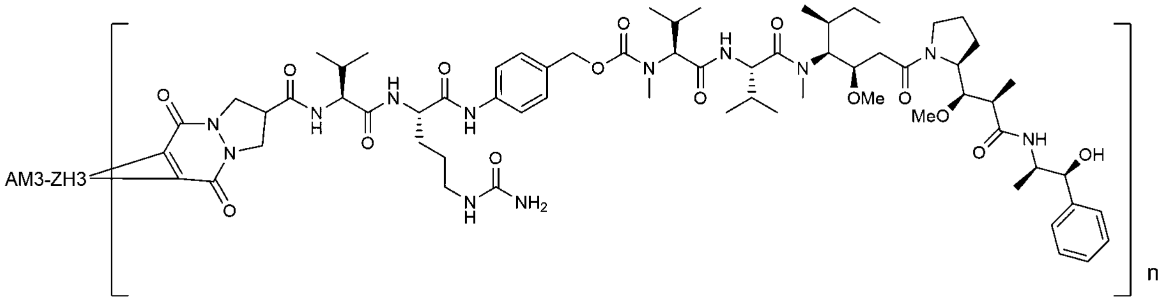

ADC-5

Antibody AM3-ZH3 was dissolved in PBS (pH 7.2), 2 mM EDTA was added, and the antibody was reduced with tris(2-carboxyethyl)phosphine hydrochloride (the molar ratio of tris(2-carboxyethyl)phosphine hydrochloride to the antibody was 10:1). After the mixture was incubated at 37 °C for about 120 min, linker-drug compound LD-1 was added to the reduced antibody (the molar ratio of LD-1 to the antibody was 5:1). After 1 h of incubation at room temperature, the mixture was purified and desalted by elution using G25 resin and filtered using a 0.2 μm filter under sterile conditions to give ADC-5, which was then cryopreserved. The average DAR value of ADC-5 was determined to be between 3.7 and 4.3 using the hydrophobic interaction chromatography-high performance liquid chromatography analysis described above.

In addition, ADCs in which the negative control IgG and the positive control antibody UC961 are conjugated to LD-14 and LD-1 were prepared using the same method as ADC-2 and ADC-5 and designated ADC-6 (UC961 was conjugated to LD-14), ADC-7 (UC961 was conjugated to LD-1), ADC-8 (IgG was conjugated to LD-14), and ADC-9 (IgG was conjugated to LD-1), and their drug loadings were 3.7-4.3.

Exemplary prepared ADCs are summarized in Table 7 below:

Table 7

| ADC | DAR | Antibody | LD |
|---|---|---|---|
| ADC-1 | 3.7~4.3 | UC961 | LD-4 |
| ADC-2 | 3.7~4.3 | AM3-ZH-3 | LD-14 |
| ADC-3 | 3.7~4.3 | KIH YR8/ZH3 | LD-14 |
| ADC-4 | 3.7~4.3 | KIH YR8/ZH3 | LD-1 |
| ADC-5 | 3.7~4.3 | AM3-ZH-3 | LD-1 |
| ADC-6 | 3.7~4.3 | UC961 | LD-14 |
| ADC-7 | 3.7~4.3 | UC961 | LD-1 |
| ADC-8 | 3.7~4.3 | IgG | LD-14 |
| ADC-9 | 3.7~4.3 | IgG | LD-1 |

In addition, the present disclosure prepared the following ADCs using the preparation methods for ADCs in Example 8. In these ADCs, Ab was an anti-ROR1 antibody of the present disclosure or a bispecific antibody of the present disclosure, and n was 1-8.

**Ab-LD-2:**

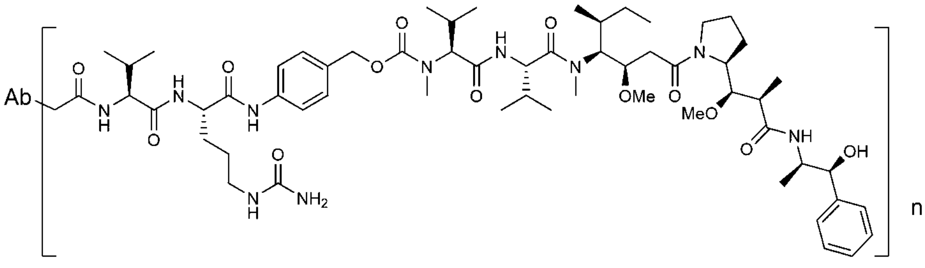

**Ab-LD-3:**

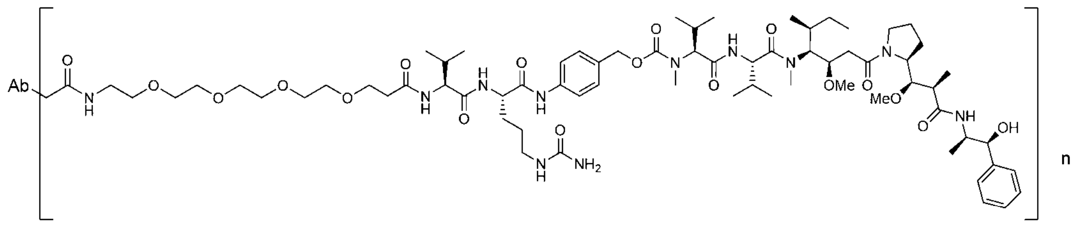

Ab-LD-4:

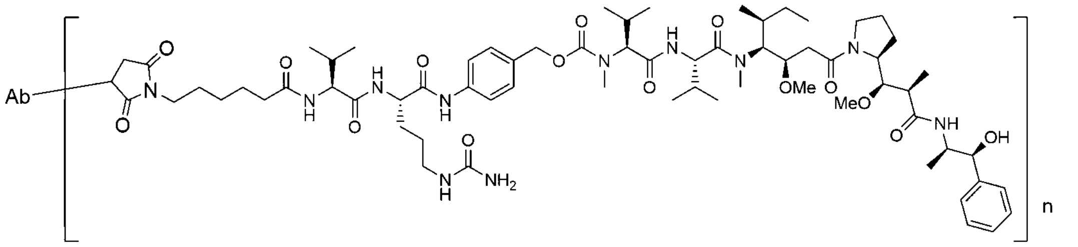

Ab-LD-5:

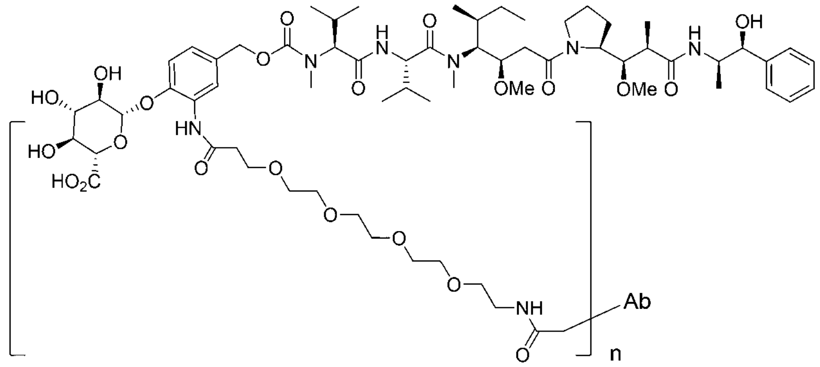

Ab-LD-6:

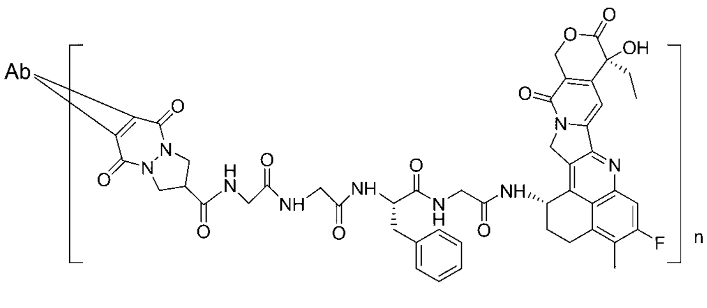

Ab-LD-7:

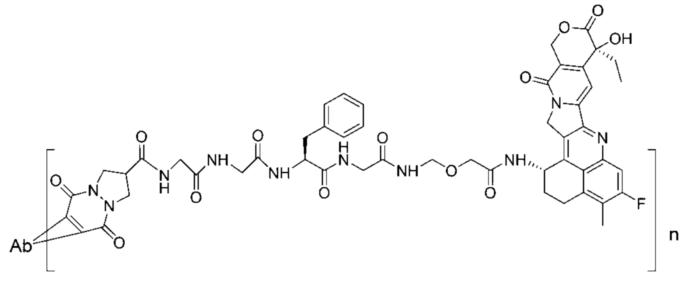

**Ab-LD-8:**

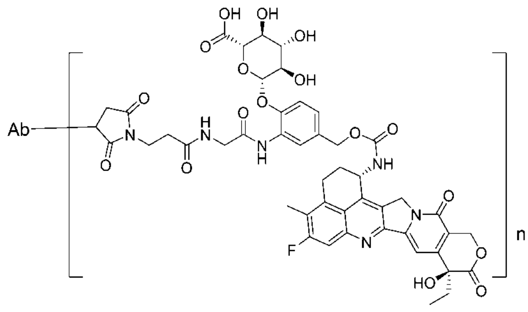

**Ab-LD-9:**

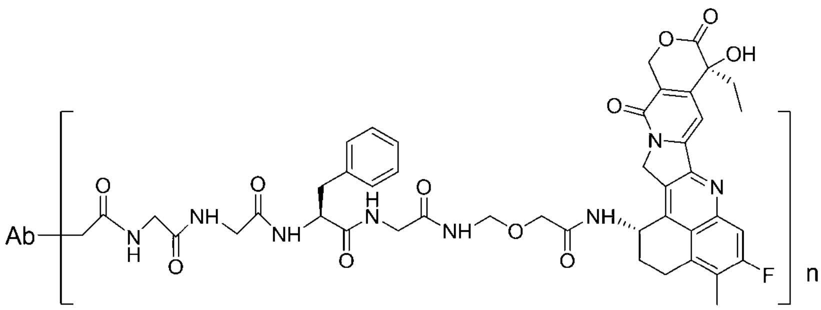

**Ab-LD-10:**

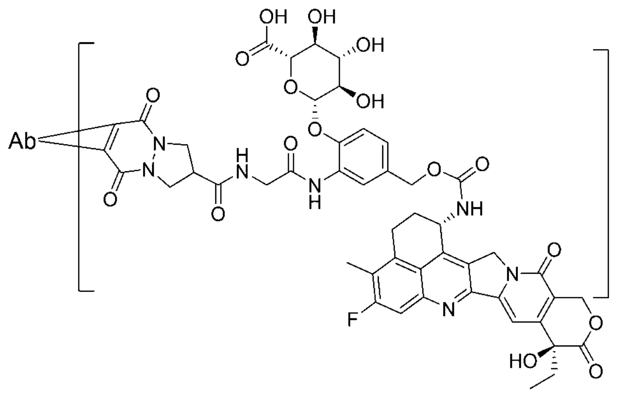

**Ab-LD-11:**

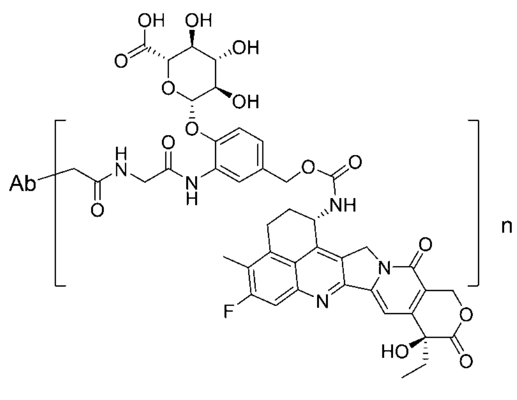

**Ab-LD-12:**

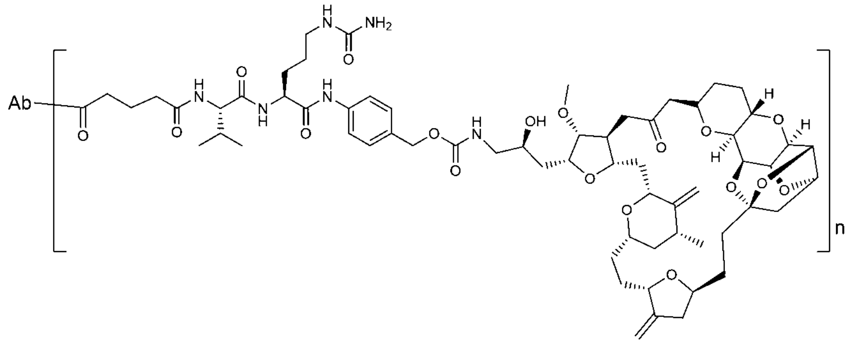

**Ab-LD-13:**

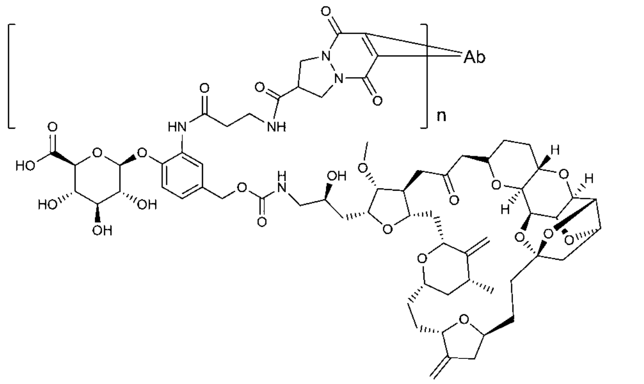

**Ab-LD-15:**

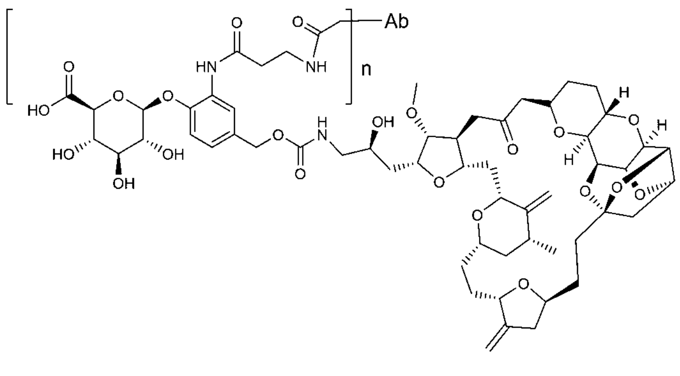

**Ab-LD-16:**

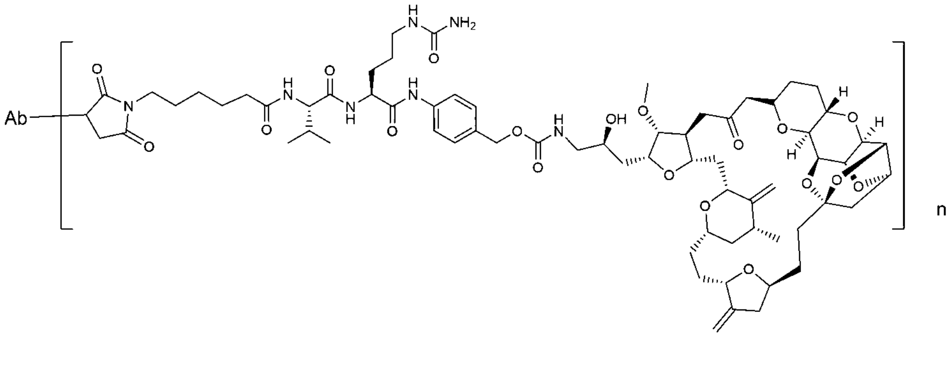

**Ab-LD-17:**

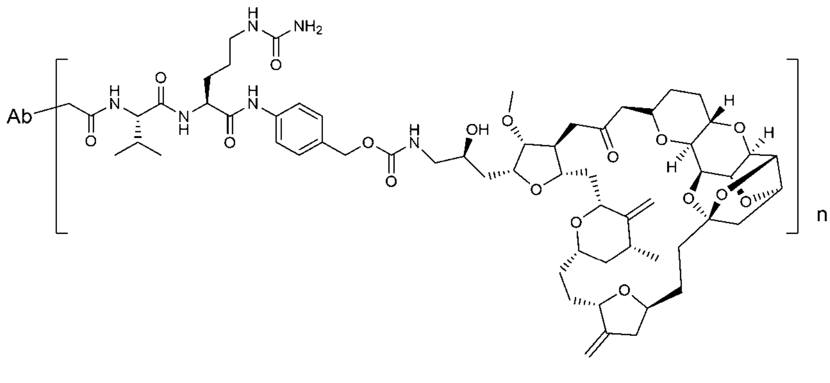

**Example** 9: **Determination of Biological Activity of Monoclonal Antibodies**

**9.1. Binding activity of monoclonal antibodies to human ROR1 protein**

The determination of the affinity for the ROR1 protein used OctetR8 (manufacturer: Sartorius) as the test instrument. An AHC (Anti-hIgG Fc Capture) biosensor (manufacturer: Sartorius) was used to capture an antibody, and the sensor was immediately immersed in the analyte (the ROR1 antigen). This experiment included a total of five steps: 1. baseline (60 s), 2. loading (antibody capture) (150 s, 1.5 nm), 3. baseline (100 s), 4. association (association with the ROR1 antigen, 60 s), and 5. dissociation (dissociation of the ROR1 antigen, 60 s). After the test was completed, the sensor was regenerated by 3 cycles of alternate 5-second immersions in a regeneration buffer (glycine, pH 1.5) and a neutralization buffer (PBS). The running buffer in this experiment was PBS.

The results are shown in Tables 8-11 below.

**9.2. Binding activity of monoclonal antibodies to cells expressing human ROR1**

The binding of the anti-ROR1 antibodies of the present disclosure to MDA-MB-231 cells (purchased from the Cell Bank of Chinese Academy of Sciences) and Jeko-1 cells (purchased from the Cell Bank of Chinese Academy of Sciences), which express human ROR1, was assessed by flow cytometry. MDA-MB-231 cells/Jeko-1 cells were incubated with the antibodies, at different concentrations (starting from 66.67 nM, 6-fold dilution, 8 concentrations in total), at 4 °C for 30 min. After the cells were washed twice with 2% BSA-PBS, PE-labeled goat anti-human IgG Fc (manufacturer: Invitrogen, Cat. No. 12-4998-82, 1:100 dilution) was added, and the cells were incubated in a dark place at 4 °C for 30 min. After the cells were washed twice with 2% BSA-PBS, the median fluorescence intensity was measured using a BD C6 plus flow cytometer.

The results are shown in Tables 8-11 below.

**9.3. Binding activity of monoclonal antibodies to human ROR2 protein**

The binding of humanized antibodies to ROR2, a protein homologous with ROR1, was assessed by ELISA. hROR2 (manufacturer: Kactus Biosystems, Cat. No. ROR-HM402) was diluted to 1 μg/mL in PBS, and a microplate was coated with the dilution and incubated at 37 °C for 1 h. The plate was then blocked with a 5% BSA-PBS blocking solution at 37 °C for 1 h. After the plate was washed with PBST, the antibodies were diluted to different concentrations (starting from 333.33 nM, 3-fold dilution, 8 concentrations in total) and added to the plate, and the plate was incubated at 37 °C for 1 h. After the plate was washed, HRP-labeled goat anti-human IgG (manufacturer: Sigma-Aldrich, Cat. No. A0170, 1:10,000 dilution) was added, and the plate was incubated at 37 °C for 1 h. After the plate was washed, the TMB solution was added. The plate was incubated in a dark place at room temperature for 15 min, and the ELISA stopping solution was then added to stop the reaction. The plate was placed on a microplate reader, and the absorbance at a wavelength of 450 nm was measured.

The results are shown in Tables 8-11 below.

Table 8. Activity of BRHu-3, a humanized antibody of D10

| Antibody | Affinity KD (M) for ROR1 | Binding to MDA-MB-231 (EC50, μg/mL) | Binding to ROR2 |
|---|---|---|---|
| ch-D10 | 2.66E-08 | 0.0634 | No binding |
| BRHu-3 | 3.23E-08 | 0.0818 | No binding |

The results in Table 8 show that humanized antibody BRHu-3 and antibody ch-D10 shared similar affinities for the ROR1 protein and similar abilities to bind to MDA-MB-231 cells, and neither antibody bound to ROR2.

Table 9. Activity of affinity-matured molecules of BRHu-3

| Antibody | Affinity KD (M) for ROR1 | Binding to MDA-MB-231 (EC50, μg/mL) | Binding to Jeko-1 (EC50, μg/mL) | Binding to ROR2 |
|---|---|---|---|---|
| BRHu-3 | 6.36E-08 | 0.09 | 0.03 | No binding |
| YR-4 | 1.00E-08 | Not determined | Not determined | Binding |
| YR-8 | 9.82E-09 | 0.02 | 0.005 | No binding |
| YR-10 | 1.13E-08 | 0.06 | 0.01 | Binding |
| YR-11 | 7.63E-09 | 0.02 | 0.007 | Binding |
| YR-21 | 6.21E-09 | Not determined | Not determined | Binding |

The results in Table 9 show that the affinity of affinity-matured antibodies YR-4, YR-8, YR-10, YR-11, and YR-21 for ROR1 was significantly higher than that of BRHu-3. YR-8, YR-10, and YR-11 all exhibited greater abilities to bind to MDA-MB-231 and Jeko-1 cells than humanized antibody BRHu-3 did. YR-4, YR-10, YR-11, and YR-21 all bound to ROR2, and YR-8 did not bind to ROR2.

Table 10. Activity of mutant molecules of YR-8

| Antibody | Affinity KD (M) for ROR1 | Binding to ROR1 (EC50, μg/mL) | Binding to MDA-MB-231 (EC50, μg/mL) | Binding to ROR2 |
|---|---|---|---|---|
| YR-8 | 1.8E-08 | 0.01 | 0.03 | No binding |
| YR-8-ES | 1.9E-08 | 0.01 | 0.05 | No binding |
| YR-8-NA | 1.4E-08 | Not determined | Not determined | Binding |
| YR-8-NV | 1.6E-08 | Not determined | Not determined | Binding |
| YR-8-NT | 1.6E-08 | Not determined | Not determined | Binding |
| YR-8-NL | 1.6E-08 | Not determined | Not determined | Binding |
| YR-8-NI | 1.4E-08 | Not determined | Not determined | Binding |

The results in Table 10 show that all mutants, except for YR-8 and YR-8-ES, bound to ROR2. The affinity of YR-8-ES for ROR1 and its binding activity to cells were comparable to those of YR-8.

Table 11. Activity of affinity-matured molecules of H10-VH4VL4

| Antibody | Affinity KD (M) for ROR1 | Binding to ROR1 (EC50, μg/mL) | Binding to MDA-MB-231 (EC50, μg/mL) | Binding to ROR2 |
|---|---|---|---|---|
| H10-hVH4-h VL4 | 4.7E-08 | 0.0144 | 0.0304 | No binding |
| AM3-ZH1 | 7.9E-09 | 0.0127 | 0.0169 | Binding |
| AM3-ZH3 | 5.0E-09 | 0.0123 | 0.0110 | No binding |
| AM3-ZH4 | 2.7E-08 | 0.0161 | 0.0163 | No binding |
| AM3-ZH5 | 1.0E-08 | 0.0124 | 0.0148 | No binding |
| AM3-ZH6 | 1.1E-08 | 0.0120 | 0.0130 | No binding |
| AM3-ZH7 | 8.5E-09 | 0.0115 | 0.0101 | No binding |

Table 11 shows that compared to H10-hVH4-hVL4, the affinity-matured antibodies exhibited varying degrees of improvement in both their affinity for ROR1 and their binding to MDA-MB-231 cells; particularly, the affinity of AM3-ZH3 was more than 9 times as high. No molecules bound to ROR2 except for AM3-ZH1.

**Example 10: Binding of Antibodies to ROR1-Expressing Cells**

Measuring the ability of anti-hROR1 bispecific antibodies to specifically bind to the hROR1 antigen expressed on the cell surface is crucial for antibodies, especially for those that are used as therapeutic antibodies *in vivo.* The cell lines selected in this experiment included PA-1 (ATCC CRL- 1572 human ovarian teratoma cells) and Jeko-1 (ATCC CRL- 3006, human mantle cell lymphoma cells). In an incubator at 37 °C with 5% $CO_2$, PA-1 cells were cultured in an ATCC EMEM culture medium containing 10% fetal bovine serum, and Jeko-1 cells were cultured in an RPMI-1640 culture medium containing 20% fetal bovine serum. The experimental process was as follows:
Cells in the logarithmic growth phase were dissociated with 0.25% trypsin and then resuspended in a staining buffer (Biolengend) for washing. The cells were counted, and the density was adjusted to $4.5 \times 10^5$ cells/100 μL of the staining buffer. The suspension was added to a 96-well plate at 45 μL/well. Then 5 μL of human TruStain (BioLegend) receptor blocking agent was added to each well to prevent antibodies from non-specifically binding to human tumor cells. The test antibodies were diluted from a starting concentration of 200 nM in the staining buffer and then 3-fold diluted to obtain a total of 11 concentration points, including 200 nM and zero. The diluted test antibodies were added to the 96-well plate at 50 μL/well, with the highest final concentration being 100 nM. After thorough mixing, the plate was incubated at 4 °C for 15 min. After the incubation, the cells were washed in the staining buffer. Then a PE-labeled constant region (Fc)-specific antibody (rabbit anti-human IgG PE conjugate, BioLegend, 410707) was suspended in 5 μL/$2 \times 10^5$ cells/100 μL of the staining buffer, and the mixture was incubated at 4 °C for 15 min. After the incubation, the cells were washed in the staining buffer and resuspended in 100 μL of the staining buffer. The readings for single cells in the PE channel were analyzed using a Novocyte 3000 (Agilent) apparatus. The corresponding PE channel reading was plotted against the antibody protein concentration using a Sigmoidal, 4PL four-parameter equation to obtain a dose-response curve. After analysis, an $EC_{50}$ value was generated. The equation was:

$$Y=Bottom + (X^{\wedge}Hillslope)*(Top-Bottom)/(X^{\wedge}HillSlope+EC50^{\wedge}HillSlope),$$

MFI fold = highest PE reading of antibody-treated experimental group/PE reading of antibody-free group.

The FACS binding activity of the anti-hROR1 antibody test substances to human ROR1 is shown in Table 12. The results show that the anti-ROR1 antibodies of the present disclosure were all able to specifically bind to PA1 and Jeko1 cells, which express ROR1, and their binding abilities were relatively high. In addition, on the same cell line, the total number of antigens that can be recognized and labeled by the biepitopic antibody was higher than that by the monoclonal antibodies, which was specifically shown by a significant increase in the MFI fold ratio.

Table 12. The abilities of anti-hROR1 antibodies to bind to cell lines

| Cell | Parameter | YR8-ES | AM3-ZH3 | KIH-YR8/ZH3 |
|---|---|---|---|---|
| PA1 | EC50 (nM) | 0.25 | 0.33 | 0.41 |
| | MFI fold | 2.6 | 2.5 | 7.0 |
| Jeko 1 | EC50 (nM) | 0.15 | 0.031 | 0.19 |
| | MFI fold | 3.6 | 2.8 | 8.2 |

**Example 11: Cytotoxicity of ADC Molecules**

In this experiment, the inhibitory effects of anti-ROR1 antibody-MMAE and anti-ROR1 antibody-eribulin conjugates on the proliferation of various tumor cell lines were studied. In this experiment, the anti-proliferative effects of the drugs were evaluated using the CellTiterGlo2 (Promega) reagent. The cell lines used in the experiment included PA-1 (ATCC, CRL- 1572 human ovarian teratoma cells), Jeko-1 (ATCC CRL-3006, human mantle cell lymphoma cells), and HCC-1187 (ATCC CRL-2322 human breast cancer cells). The experimental process was as follows:

In an incubator at 37 °C with 5% $CO_2$, PA-1 cells were cultured in an ATCC EMEM culture medium containing 10% fetal bovine serum, Jeko-1 cells were cultured in an RPMI-1640 culture medium containing 20% fetal bovine serum, and HCC-1187 cells were cultured in an RPMI-1640 culture medium containing 10% fetal bovine serum. The three types of cells were inoculated onto 96-well plates at a density of $2 \times 10^3$ to $5 \times 10^3$ cells/well (50 μL/well). After 24 h of culture, anti-ROR1 antibody-MMAE or anti-ROR1 antibody-eribulin conjugates or control antibody-MMAE or control antibody-eribulin conjugates diluted in culture media to different concentrations were added at 100 μL/well, with each concentration added

in duplicate, and vehicle control and cell-free culture medium wells were set at the corresponding concentrations. After the plates were incubated in an incubator at 37 °C with 5% $CO_2$ for 96 h (PA-1 and Jeko-1) or 144 h (HCC-1187), 100 μL of CellTiterGlo2 was added to each well, and the plates were placed on an orbital shaker at room temperature for 15 min for mixing. The luminescence was measured, and the $IC_{50}$ values (nM) of the anti-ROR1 antibody-MMAE or anti-ROR1 antibody-eribulin conjugates against the various cells were calculated. The results are shown in Table 13.

Table 13. The inhibitory $IC_{50}$ values (nM) of ADCs against the proliferation of different cells

| Antibody | **HCC1187** | | **PA-1** | | **Jeko1** | |
|---|---|---|---|---|---|---|
| | LD-14 | LD-1 | LD-14 | LD-1 | LD-14 | LD-1 |
| UC961 | 0.24 | 15.4 | 0.36 | 15.1 | 2.08 | 76.3 |
| AM3-ZH3 | 0.09 | 6.3 | 0.017 | 17.8 | 0.285 | 65.1 |
| KIH-YR8/ZH3 | 0.014 | 1.2 | 0.032 | 4.2 | 0.018 | 46.6 |
| IgG | 16.1 | 67 | 22.4 | 60.2 | 23.5 | 126 |

As can be seen from Table 13, the anti-ROR1 antibody-MMAE or -eribulin conjugates exhibited significant killing effects on all three types of tumor cells with different ROR1 expression levels, and the inhibitory effects were related to the different modes of conjugation or different toxins used. This indicates that the anti-ROR1 antibody conjugates described in the present disclosure can specifically kill positive cells through ROR1-mediated endocytosis. In addition, their significant inhibitory effects on the cancer cell lines from breast cancer, ovarian cancer, and lymphocytic cancer indicate that the obtained ADCs have relatively good inhibitory effects on various solid tumors and hematological tumors.

### Example 12: *In Vivo* Efficacy Tests of ADC Molecules

#### 12.1. ADC efficacy test in breast cancer cell line HCC1187 graft mouse model

$1 \times 10^7$ HCC1187 cells (a breast cancer cell line that expresses human ROR1, purchased from ATCC) were subcutaneously grafted into each female severe combined immunodeficiency CB17 SCID mouse (purchased from Charles River Laboratories). After the graft, when the average tumor size reached 162 $mm^3$ (day 0), the mice were divided into groups, and the ADC1, ADC2, ADC3, ADC4, or ADC5 prepared in Example 8 was intravenously injected into mice at 3.0 mg/kg once (single administration), or ADC2, ADC3, ADC4, or ADC5 was intravenously injected into mice at 1.0 mg/kg once every four days for a total of two doses (day 0 and day 4, Q4D × 2). In the control group, PBS was intravenously injected into mice at 4 mL/kg. During the 48 days thereafter, the tumor size and body weight of the mice were measured, and the tumor growth inhibition (TGI) rate was calculated. TGI was calculated as follows:

$$TGI (\%) = 1 - [(Td - T0) / (Cd - C0)] \times 100\%$$

where Td and Cd are the average tumor volumes of the treatment group and the control group on the day of tumor volume measurement, and T0 and C0 are the average tumor volumes of the treatment group and the control group on day 0.

The results are shown in FIG. 1 and Table 14.

Table 14. The tumor growth inhibition rates of different ADCs against subcutaneous HCC1187 tumors in mice

| ADC | Dose of administration | Mode of administration | Tumor growth inhibition rate TGI% (day 27) |
|---|---|---|---|
| ADC-1 | 3 mg/kg | Single administration | 82.26 |
| ADC-2 | 3 mg/kg | Single administration | 109.30 |
| ADC-2 | 1 mg/kg | Q4D×2 | 111.08 |
| ADC-3 | 3 mg/kg | Single administration | 111.26 |
| ADC-3 | 1 mg/kg | Q4D×2 | 111.01 |
| ADC-4 | 3 mg/kg | Single administration | 111.25 |
| ADC-4 | 1 mg/kg | Q4D×2, | 111.64 |
| ADC-5 | 3 mg/kg | Single administration | 111.77 |

(continued)

| ADC | Dose of administration | Mode of administration | Tumor growth inhibition rate TGI% (day 27) |
|---|---|---|---|
| ADC-5 | 1 mg/kg | Q4D×2 | 111.35 |

The results show that the ADCs of the present disclosure were able to significantly inhibit the growth of grafted HCC1187 tumors in mice.

**12.2. ADC efficacy test in mantle cell lymphoma cell line Jeko-1 graft mouse model**

$1 \times 10^7$ Jeko-1 cells (mantle cell lymphoma cells that express human ROR1, purchased from ATCC) were subcutaneously grafted into each female severe combined immunodeficiency CB17 SCID mouse (purchased from Charles River Laboratories). After the graft, when the average tumor size reached 156 $mm^3$ (day 0), the mice were divided into groups, and the ADC1, ADC2, ADC3, ADC4, or ADC5 prepared in Example 8 was intravenously injected into mice at 3.0 mg/kg once (single administration), or ADC2, ADC3, ADC4, or ADC5 was intravenously injected into mice at 1.0 mg/kg once every four days for a total of three doses (day 0, day 4, and day 8, Q4D × 3). In the control group, PBS was intravenously injected into mice at 4 mL/kg. During the 51 days thereafter, the size of the tumor grafts in the mice and the body weight of the mice were measured, and the tumor growth inhibition (TGI) rate was calculated. The results are shown in FIG. 2 and Table 15.

Table 15. The tumor growth inhibition rates of different ADCs against subcutaneous Jeko-1 tumors in mice

| ADC | Dose of administration | Mode of administration | Tumor growth inhibition rate TGI% (day 20) |
|---|---|---|---|
| ADC-1 | 3 mg/kg | Single administration | 28.01 |
| ADC-2 | 3 mg/kg | Single administration | 92.00 |
| ADC-2 | 1 mg/kg | Q4D × 3 intravenous infusion | 96.42 |
| ADC-3 | 3 mg/kg | Single administration | 101.26 |
| ADC-3 | 1 mg/kg | Q4D×3 | 102.16 |
| ADC-4 | 3 mg/kg | Single administration | 99.82 |
| ADC-4 | 1 mg/kg | Q4D×3 | 97.79 |
| ADC-5 | 3 mg/kg | Single administration | 87.48 |
| ADC-5 | 1 mg/kg | Q4D×3 | 81.55 |

The results show that the ADCs of the present disclosure were able to significantly inhibit the growth of grafted Jeko-1 tumors in mice.

**12.3. ADC efficacy test in ovarian cancer cell line PA-1 graft mouse model**

$5 \times 10^6$ PA-1 cells (an ovarian cancer cell line that expresses human ROR1, purchased from ATCC) were subcutaneously grafted into each female nude mouse (purchased from Charles River Laboratories). After the graft, when the average tumor size reached about 150 $mm^3$ (day 0), the mice were divided into groups, and the ADC1 or ADC3 prepared in Example 8 was intravenously injected into mice at 1.0 mg/kg or 3.0 mg/kg once (single administration). In the control group, PBS was intravenously injected into mice at 4 mL/kg. During the 13 days thereafter, the tumor size and body weight of the mice were measured, and TGI was calculated. The results are shown in FIG. 3 and Table 16.

Table 16. The tumor growth inhibition rates of different ADCs against subcutaneous PA-1 tumors in mice

| ADC | Dose of administration | Mode of administration | Tumor growth inhibition rate TGI% (day 13) |
|---|---|---|---|
| ADC-1 | 1 mg/kg | Single administration | 42.92 |
| ADC-1 | 3 mg/kg | Single administration | 54.57 |
| ADC-3 | 1 mg/kg | Single administration | 37.45 |
| ADC-3 | 3 mg/kg | Single administration | 103.88 |

The results show that the ADCs of the present disclosure were able to significantly inhibit the growth of grafted PA-1 tumors in mice.

All documents mentioned in the present disclosure are incorporated herein by reference, just as each document is individually incorporated by reference. In addition, it should be understood that various modifications or changes may be made by those skilled in the art after reading the above teachings of the present disclosure, and these equivalent forms also fall within the scope defined by the appended claims of the present disclosure.

## Claims

1. An anti-ROR1 antibody, comprising a heavy chain variable region and a light chain variable region, wherein:

(Z1) the heavy chain variable region comprises an HCDR1 set forth in SEQ ID NO: 61, an HCDR2 set forth in SEQ ID NO: 46, and an HCDR3 set forth in SEQ ID NO: 65, and the light chain variable region comprises an LCDR1 set forth in SEQ ID NO: 63, an LCDR2 set forth in SEQ ID NO: 64, and an LCDR3 set forth in SEQ ID NO: 50;
(Z2) the heavy chain variable region comprises an HCDR1 set forth in SEQ ID NO: 45, an HCDR2 set forth in SEQ ID NO: 46, and an HCDR3 set forth in SEQ ID NO: 66, and the light chain variable region comprises an LCDR1 set forth in SEQ ID NO: 63, an LCDR2 set forth in SEQ ID NO: 64, and an LCDR3 set forth in SEQ ID NO: 50;
(Z3) the heavy chain variable region comprises an HCDR1 set forth in SEQ ID NO: 45, an HCDR2 set forth in SEQ ID NO: 46, and an HCDR3 set forth in SEQ ID NO: 65, and the light chain variable region comprises an LCDR1 set forth in SEQ ID NO: 67, an LCDR2 set forth in SEQ ID NO: 68, and an LCDR3 set forth in SEQ ID NO: 50;
(Z4) the heavy chain variable region comprises an HCDR1 set forth in SEQ ID NO: 45, an HCDR2 set forth in SEQ ID NO: 46, and an HCDR3 set forth in SEQ ID NO: 65, and the light chain variable region comprises an LCDR1 set forth in SEQ ID NO: 63, an LCDR2 set forth in SEQ ID NO: 69, and an LCDR3 set forth in SEQ ID NO: 50;
(Z5) the heavy chain variable region comprises an HCDR1 set forth in SEQ ID NO: 45, an HCDR2 set forth in SEQ ID NO: 46, and an HCDR3 set forth in SEQ ID NO: 65, and the light chain variable region comprises an LCDR1 set forth in SEQ ID NO: 63, an LCDR2 set forth in SEQ ID NO: 64, and an LCDR3 set forth in SEQ ID NO: 50;
(Z6) the heavy chain variable region comprises an HCDR1 set forth in SEQ ID NO: 17, an HCDR2 set forth in SEQ ID NO: 32, and an HCDR3 set forth in SEQ ID NO: 19, and the light chain variable region comprises an LCDR1 set forth in SEQ ID NO: 20, an LCDR2 set forth in SEQ ID NO: 21, and an LCDR3 set forth in SEQ ID NO: 10; or
(Z7) the heavy chain variable region comprises an HCDR1 set forth in SEQ ID NO: 17, an HCDR2 set forth in SEQ ID NO: 18, and an HCDR3 set forth in SEQ ID NO: 19, and the light chain variable region comprises an LCDR1 set forth in SEQ ID NO: 20, an LCDR2 set forth in SEQ ID NO: 21, and an LCDR3 set forth in SEQ ID NO: 10.

2. The anti-ROR1 antibody according to claim 1, wherein:

the heavy chain variable region comprises a sequence having at least 85% sequence identity to the amino acid sequence set forth in SEQ ID NO: 72, and the light chain variable region comprises a sequence having at least 85% sequence identity to the amino acid sequence set forth in SEQ ID NO: 71; or
the heavy chain variable region comprises a sequence having at least 85% sequence identity to the amino acid sequence set forth in SEQ ID NO: 73, and the light chain variable region comprises a sequence having at least 85% sequence identity to the amino acid sequence set forth in SEQ ID NO: 71; or
the heavy chain variable region comprises a sequence having at least 85% sequence identity to the amino acid sequence set forth in SEQ ID NO: 74, and the light chain variable region comprises a sequence having at least 85% sequence identity to the amino acid sequence set forth in SEQ ID NO: 75; or
the heavy chain variable region comprises a sequence having at least 85% sequence identity to the amino acid sequence set forth in SEQ ID NO: 74, and the light chain variable region comprises a sequence having at least 85% sequence identity to the amino acid sequence set forth in SEQ ID NO: 76; or
the heavy chain variable region comprises a sequence having at least 85% sequence identity to the amino acid sequence set forth in SEQ ID NO: 74, and the light chain variable region comprises a sequence having at least 85% sequence identity to the amino acid sequence set forth in SEQ ID NO: 71; or
the heavy chain variable region comprises a sequence having at least 85% sequence identity to the amino acid sequence set forth in SEQ ID NO: 22, and the light chain variable region comprises a sequence having at least 85% sequence identity to the amino acid sequence set forth in SEQ ID NO: 23; or
the heavy chain variable region comprises a sequence having at least 85% sequence identity to the amino acid sequence set forth in SEQ ID NO: 38, and the light chain variable region comprises a sequence having at least 85% sequence identity to the amino acid sequence set forth in SEQ ID NO: 23.

**3.** The anti-ROR1 antibody according to claim 1 or 2, comprising a heavy chain constant region and a light chain constant region, preferably, wherein the heavy chain constant region comprises the amino acid sequence set forth in SEQ ID NO: 11 or a variant thereof, and/or the light chain constant region comprises the amino acid sequence set forth in SEQ ID NO: 12 or a variant thereof.

**4.** The anti-ROR1 antibody according to any one of claims 1 to 3, wherein the anti-ROR1 antibody comprises:

a heavy chain having an amino acid sequence as set forth in SEQ ID NO: 77 and a light chain having an amino acid sequence as set forth in SEQ ID NO: 78; or
a heavy chain having an amino acid sequence as set forth in SEQ ID NO: 30 and a light chain having an amino acid sequence as set forth in SEQ ID NO: 31; or
a heavy chain having an amino acid sequence as set forth in SEQ ID NO: 44 and a light chain having an amino acid sequence as set forth in SEQ ID NO: 31.

**5.** The anti-ROR1 antibody according to claim 1 or 2, wherein the anti-ROR1 antibody is an antibody fragment; preferably, the antibody fragment is selected from the group consisting of Fab, Fab', $F(ab')_2$, Fd, Fv, scFv, dsFv, and dAb.

**6.** A multispecific antibody, comprising the anti-ROR1 antibody according to any one of claims 1 to 5.

**7.** The multispecific antibody according to claim 6, wherein the multispecific antibody is a bispecific antibody comprising a first antigen-binding domain and a second antigen-binding domain, wherein the first antigen-binding domain and the second antigen-binding domain bind to different epitopes on human ROR1; preferably, the first antigen-binding domain binds to the amino acids at positions 130-165 of human ROR1, and the second antigen-binding domain binds to the amino acids at positions 70-130 of human ROR1; the amino acid sequence of the ROR1 protein is set forth in SEQ ID NO: 87.

**8.** The multispecific antibody according to claim 7, wherein the first antigen-binding domain comprises a heavy chain variable region VH1 and a light chain variable region VL1, and the second antigen-binding domain comprises a heavy chain variable region VH2 and a light chain variable region VL2, wherein:

the heavy chain variable region VH1 comprises an HCDR1 set forth in SEQ ID NO: 17, an HCDR2 set forth in SEQ ID NO: 32, and an HCDR3 set forth in SEQ ID NO: 19, and the light chain variable region VL1 comprises an LCDR1 set forth in SEQ ID NO: 20, an LCDR2 set forth in SEQ ID NO: 21, and an LCDR3 set forth in SEQ ID NO: 10; or
the heavy chain variable region VH1 comprises an HCDR1 set forth in SEQ ID NO: 17, an HCDR2 set forth in SEQ ID NO: 18, and an HCDR3 set forth in SEQ ID NO: 19, and the light chain variable region VL1 comprises an LCDR1 set forth in SEQ ID NO: 20, an LCDR2 set forth in SEQ ID NO: 21, and an LCDR3 set forth in SEQ ID NO: 10; and
the heavy chain variable region VH2 comprises an HCDR1 set forth in SEQ ID NO: 61, an HCDR2 set forth in SEQ ID NO: 46, and an HCDR3 set forth in SEQ ID NO: 65, and the light chain variable region VL2 comprises an LCDR1 set forth in SEQ ID NO: 63, an LCDR2 set forth in SEQ ID NO: 64, and an LCDR3 set forth in SEQ ID NO: 50; or
the heavy chain variable region VH2 comprises an HCDR1 set forth in SEQ ID NO: 45, an HCDR2 set forth in SEQ ID NO: 46, and an HCDR3 set forth in SEQ ID NO: 66, and the light chain variable region VL2 comprises an LCDR1 set forth in SEQ ID NO: 63, an LCDR2 set forth in SEQ ID NO: 64, and an LCDR3 set forth in SEQ ID NO: 50; or
the heavy chain variable region VH2 comprises an HCDR1 set forth in SEQ ID NO: 45, an HCDR2 set forth in SEQ ID NO: 46, and an HCDR3 set forth in SEQ ID NO: 65, and the light chain variable region VL2 comprises an LCDR1 set forth in SEQ ID NO: 67, an LCDR2 set forth in SEQ ID NO: 68, and an LCDR3 set forth in SEQ ID NO: 50; or
the heavy chain variable region VH2 comprises an HCDR1 set forth in SEQ ID NO: 45, an HCDR2 set forth in SEQ ID NO: 46, and an HCDR3 set forth in SEQ ID NO: 65, and the light chain variable region VL2 comprises an LCDR1 set forth in SEQ ID NO: 63, an LCDR2 set forth in SEQ ID NO: 69, and an LCDR3 set forth in SEQ ID NO: 50; or
the heavy chain variable region VH2 comprises an HCDR1 set forth in SEQ ID NO: 45, an HCDR2 set forth in SEQ ID NO: 46, and an HCDR3 set forth in SEQ ID NO: 65, and the light chain variable region VL2 comprises an LCDR1 set forth in SEQ ID NO: 63, an LCDR2 set forth in SEQ ID NO: 64, and an LCDR3 set forth in SEQ ID NO: 50; preferably,
the heavy chain variable region VH1 comprises an HCDR1 set forth in SEQ ID NO: 17, an HCDR2 set forth in SEQ ID NO: 32, and an HCDR3 set forth in SEQ ID NO: 19, and the light chain variable region VL1 comprises an LCDR1 set forth in SEQ ID NO: 20, an LCDR2 set forth in SEQ ID NO: 21, and an LCDR3 set forth in SEQ ID NO: 10; and the heavy chain variable region VH2 comprises an HCDR1 set forth in SEQ ID NO: 61, an HCDR2 set forth in SEQ ID NO: 46, and an HCDR3 set forth in SEQ ID NO: 65, and the light chain variable region VL2 comprises an LCDR1

set forth in SEQ ID NO: 63, an LCDR2 set forth in SEQ ID NO: 64, and an LCDR3 set forth in SEQ ID NO: 50.

**9.** The multispecific antibody according to claim 7 or 8, wherein the first antigen-binding domain comprises:

the heavy chain variable region VH1 comprises the amino acid sequence set forth in SEQ ID NO: 38, and the light chain variable region VL1 comprises the amino acid sequence set forth in SEQ ID NO: 23; or
the heavy chain variable region VH1 comprises the amino acid sequence set forth in SEQ ID NO: 22, and the light chain variable region VL1 comprises the amino acid sequence set forth in SEQ ID NO: 23;
and the second antigen-binding domain comprises:

the heavy chain variable region VH2 comprises the amino acid sequence set forth in SEQ ID NO: 72, and the light chain variable region VL2 comprises the amino acid sequence set forth in SEQ ID NO: 71; or
the heavy chain variable region VH2 comprises the amino acid sequence set forth in SEQ ID NO: 73, and the light chain variable region VL2 comprises the amino acid sequence set forth in SEQ ID NO: 71; or
the heavy chain variable region VH2 comprises the amino acid sequence set forth in SEQ ID NO: 74, and the light chain variable region VL2 comprises the amino acid sequence set forth in SEQ ID NO: 75; or
the heavy chain variable region VH2 comprises the amino acid sequence set forth in SEQ ID NO: 74, and the light chain variable region VL2 comprises the amino acid sequence set forth in SEQ ID NO: 76; or
the heavy chain variable region VH2 comprises the amino acid sequence set forth in SEQ ID NO: 74, and the light chain variable region VL2 comprises the amino acid sequence set forth in SEQ ID NO: 71; preferably, the first antigen-binding domain comprises a heavy chain variable region VH1 set forth in SEQ ID NO: 38 and a light chain variable region VL1 set forth in SEQ ID NO: 23, and the second antigen-binding domain comprises a heavy chain variable region VH2 set forth in SEQ ID NO: 72 and a light chain variable region VL2 set forth in SEQ ID NO: 71.

**10.** The multispecific antibody according to any one of claims 7 to 9, wherein the bispecific antibody comprises an Fc region comprising a first subunit Fc1 and a second subunit Fc2 that are capable of associating with each other, and the Fc1 and Fc2 each independently have one or more amino acid substitutions that reduce homodimerization of the Fc region;

preferably, the Fc1 has a knob structure according to the knob-in-hole technique, and the Fc2 has a hole structure according to the knob-in-hole technique;
more preferably, the amino acid at position 366 of Fc1 is W, and the amino acid at position 366 is S, the amino acid at position 368 is A, and the amino acid at position 407 is V of Fc2, the amino acid positions are numbered according to the EU index;
most preferably, the Fc1 comprises the amino acid sequence of SEQ ID NO: 79, and the Fc2 comprises the amino acid sequence of SEQ ID NO: 80.

**11.** The multispecific antibody according to claim 10, wherein the bispecific antibody comprises four chains represented by (a)-(d):

(a) [heavy chain variable region VH1]-[CH1]-[Fc1],
(b) [light chain variable region VL1]-[CL1],
(c) [heavy chain variable region VH2]-[CH1]-[Fc2], and
(d) [light chain variable region VL2]-[CL2];
or the bispecific antibody comprises four chains represented by (e), (b), (f), and (d):

(e) [heavy chain variable region VH1]-[CH1]-[Fc2],
(b) [light chain variable region VL1]-[CL1],
(f) [heavy chain variable region VH2]-[CH1]-[Fc1], and
(d) [light chain variable region VL2]-[CL2];
wherein the structures represented by formulas (a), (b), (c), (d), (e), and (f) are arranged from the N-terminus to the C-terminus; the CL1 and CL2 are each independently antibody light chain constant regions, and the CH1 is a first portion of an antibody heavy chain constant region; preferably,
the heavy chain variable region VH1 comprises the amino acid sequence set forth in SEQ ID NO: 38, the light chain variable region VL1 comprises the amino acid sequence set forth in SEQ ID NO: 23, the heavy chain variable region VH2 comprises the amino acid sequence set forth in SEQ ID NO: 72, and the light chain variable region VL2 comprises the amino acid sequence set forth in SEQ ID NO: 71;

more preferably, the bispecific antibody comprises the following four chains:

a chain 1 comprising the amino acid sequence set forth in SEQ ID NO: 81;
a chain 2 comprising the amino acid sequence set forth in SEQ ID NO: 82;
a chain 3 comprising the amino acid sequence set forth in SEQ ID NO: 83; and
a chain 4 comprising the amino acid sequence set forth in SEQ ID NO: 84.

**12.** A nucleic acid molecule, wherein the nucleic acid molecule encodes the anti-ROR1 antibody according to any one of claims 1 to 5 or the multispecific antibody according to any one of claims 6 to 11.

**13.** A biomaterial, wherein the biomaterial is:

(1) a vector comprising the nucleic acid molecule according to claim 12; or
(2) a host cell comprising the nucleic acid molecule according to claim 12 or the vector described in (1).

**14.** An antibody-drug conjugate represented by the following formula or a pharmaceutically acceptable salt thereof:

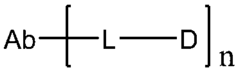

wherein:

Ab is an antibody comprising the anti-ROR1 antibody according to any one of claims 1 to 5 or the multispecific antibody according to any one of claims 6 to 11;
L is a linker;
D is a drug;
n is an integer or decimal from 1 to 10.

**15.** The antibody-drug conjugate or the pharmaceutically acceptable salt thereof according to claim 14, wherein L has a structure of $-L^a-L^b-L^c-L^d-$, wherein $L^a$ is attached to the antibody, and $L^d$ is attached to the drug, wherein:

$L^a$ is selected from the group consisting of

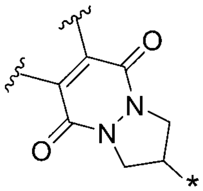, 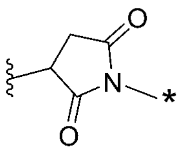, 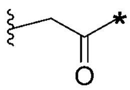,

and

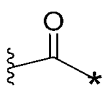,

wherein the wavy line represents a point of attachment to Ab, and * represents a point of attachment to $L^b$;
$L^b$ is selected from the group consisting of $-(CH_2)m-C(O)-$, $-NH-(CH_2CH_2O)p-(CH_2)s-C(O)-$, $-C(O)-NH-(CH_2)q-C(O)-$, $-NH-(CH_2)r-C(O)-$, and a bond, wherein:

m is an integer from 0 to 10; preferably, m is 0, 2, 3, or 5; p is an integer from 1 to 8, and s is an integer from 0 to 6; preferably, p is 4, and s is 2;
q is an integer from 1 to 5; preferably, q is 2;
r is an integer from 1 to 5; preferably, r is 2;
$L^c$ is a peptide residue composed of 1 to 7 amino acids, or is a bond, wherein the amino acids are selected

from the group consisting of valine, citrulline, glycine, phenylalanine, alanine, proline, isoleucine, lysine, serine, glutamic acid, and aspartic acid; the amino acids are unsubstituted or are each independently substituted with one or more substituents, wherein each of the substituents is independently selected from the group consisting of halogen, hydroxyl, cyano, amino, $C_{1-6}$ alkyl, $C_{1-6}$ haloalkyl, $C_{1-6}$ alkoxy, and $C_{3-7}$ cycloalkyl;

$L^d$ is selected from the group consisting of -NH-$CH_2$-O-$CH_2$-C(O)-, -NH-$R^a$-$CH_2$-O-C(O)-, and a bond, wherein $R^a$ is phenyl or 5-6 membered heterocyclyl, and the phenyl and 5-6 membered heterocyclyl are unsubstituted or are each independently substituted with one or more substituents, wherein each of the substituents is independently selected from the group consisting of

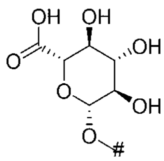,

halogen, oxo, hydroxyl, cyano, amino, $C_{1-6}$ alkyl, $C_{1-6}$ haloalkyl, and $C_{1-6}$ alkoxy, wherein # represents a point of attachment to the phenyl or 5-6 membered heterocyclyl.

**16.** The antibody-drug conjugate or the pharmaceutically acceptable salt according to claim 15, wherein the L has a structure selected from the group consisting of:

i) $L^a$ is

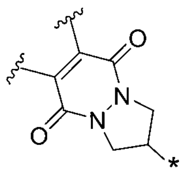,

$L^b$ is -C(O)- or -C(O)-NH-$(CH_2)_2$-C(O)-, L° is -glycine-glycine-phenylalanine-glycine-, -valine-citrulline-, -glycine-, or a bond, and $L^d$ is

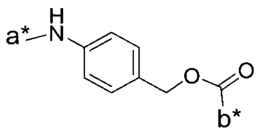, 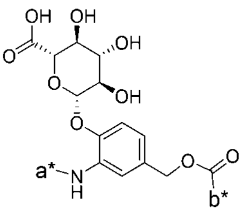,

-NH-$CH_2$-O-$CH_2$-C(O)-, or a bond; or

ii) $L^a$ is

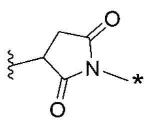,

and $L^b$ is -$(CH_2)$m-C(O)-, wherein m is 2 or 5, L° is -valine-citrulline- or -glycine-, and $L^d$ is

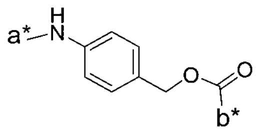 or 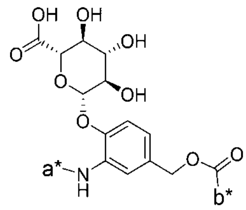 ;

or

iii) $L^a$ is

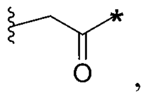 ,

$L^b$ is $-NH-(CH_2-CH_2-O)_4-(CH_2)_2-C(O)-$, $-NH-(CH_2)_2-C(O)-$, or a bond, L° is -valine-citrulline-, -glycine-, -glycine-glycine-phenylalanine-glycine-, or a bond, and $L^d$ is

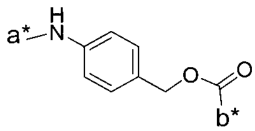 , 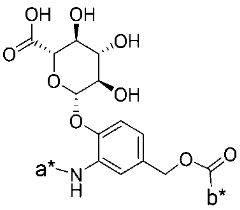 ,

or $-NH-CH_2-O-CH_2-C(O)-$; and

iiii) $L^a$ is

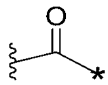 ,

$L^b$ is $-(CH_2)_3-C(O)-$, $L^c$ is -valine-citrulline-, and $L^d$ is

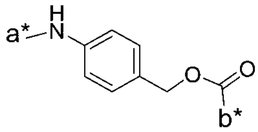 ;

wherein the wavy line represents a point of attachment to Ab, * represents a point of attachment to $L^b$, a* represents a point of attachment to L°, and b* represents a point of attachment to the drug;

preferably,

$L^a$ is

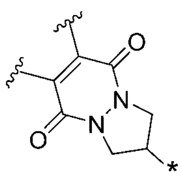 ,

$L^b$ is -C(O)-, L° is -valine-citrulline-, and $L^d$ is

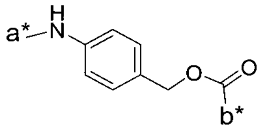 .

**17.** The antibody-drug conjugate or the pharmaceutically acceptable salt according to any one of claims 14 to 16, wherein the drug is selected from the group consisting of a cytotoxic compound, an immunomodulator, an enzyme, and a hormone inhibitor;
preferably, the drug is selected from the group consisting of monomethyl auristatin E (MMAE), monomethyl auristatin F (MMAF), eribulin, exatecan, maytansine, SN-38, and a combination thereof.

**18.** The antibody-drug conjugate or the pharmaceutically acceptable salt according to claim 17, having the following structure: or

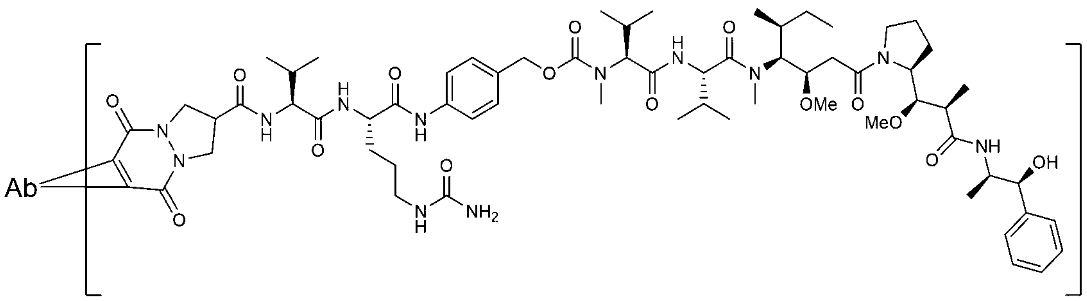 ,

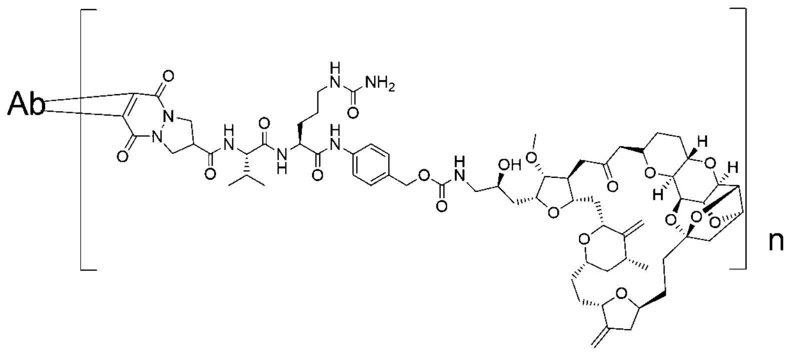 ,

or

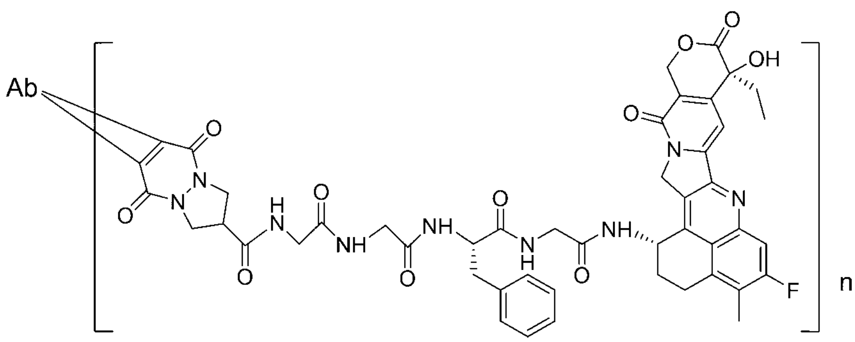 ,

wherein:

Ab and n are as defined in claim 14;
preferably, Ab comprises a heavy chain set forth in SEQ ID NO: 77 and a light chain set forth in SEQ ID NO: 78, or Ab comprises the following four polypeptide chains:

a chain 1 comprising the amino acid sequence set forth in SEQ ID NO: 81;
a chain 2 comprising the amino acid sequence set forth in SEQ ID NO: 82;
a chain 3 comprising the amino acid sequence set forth in SEQ ID NO: 83; and
a chain 4 comprising the amino acid sequence set forth in SEQ ID NO: 84;
n is 3.5-4.5.

**19.** A pharmaceutical composition, comprising: (a) the anti-ROR1 antibody according to any one of claims 1 to 5, or the multispecific antibody according to any one of claims 6 to 11, or the antibody-drug conjugate or the pharmaceutically acceptable salt thereof according to any one of claims 14 to 18; and (b) a pharmaceutically acceptable carrier.

**20.** Use of the anti-ROR1 antibody according to any one of claims 1 to 5, or the multispecific antibody according to any one of claims 6 to 11, or the antibody-drug conjugate or the pharmaceutically acceptable salt thereof according to any one of claims 14 to 18, or the pharmaceutical composition according to claim 19 in the preparation of a medicament for treating a tumor or cancer, preferably, wherein the tumor or cancer is a solid tumor or a hematological tumor; more preferably, the tumor or cancer is selected from the group consisting of breast cancer, pancreatic cancer, lung cancer, esophageal cancer, non-small cell lung cancer, laryngeal tumors, sarcoma, pharyngeal tumors, oral tumors, gastric cancer, ovarian cancer, prostate cancer, bladder cancer, colon cancer, colorectal cancer, urothelial cancer, cervical cancer, lymphoma, and leukemia; most preferably, the tumor or cancer expresses ROR1.

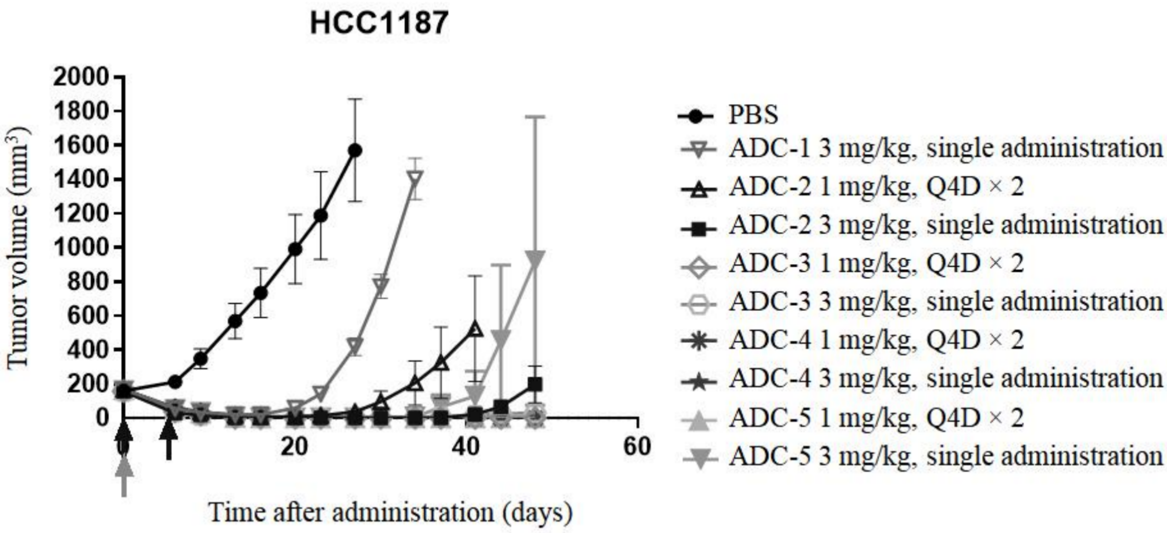
HCC1187
Tumor volume (mm3)
2000
1800
1600
1400
1200
1000
800
600
400
200
0
0
20
40
60
Time after administration (days)
PBS
ADC-1 3 mg/kg, single administration
ADC-2 1 mg/kg, Q4D × 2
ADC-2 3 mg/kg, single administration
ADC-3 1 mg/kg, Q4D × 2
ADC-3 3 mg/kg, single administration
ADC-4 1 mg/kg, Q4D × 2
ADC-4 3 mg/kg, single administration
ADC-5 1 mg/kg, Q4D × 2
ADC-5 3 mg/kg, single administration

FIG. 1

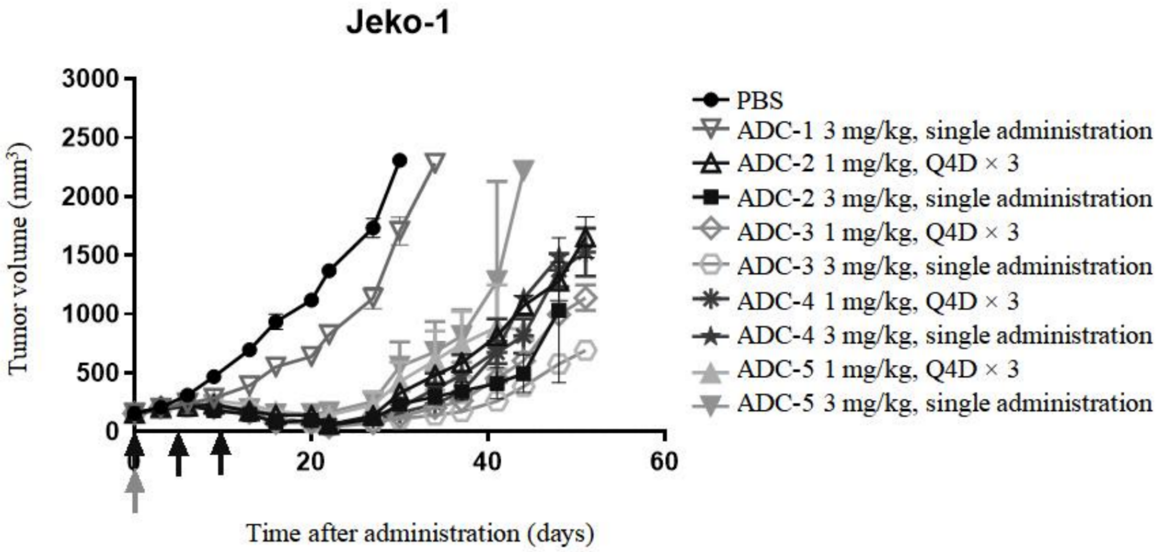
Jeko-1
Tumor volume (mm3)
3000
2500
2000
1500
1000
500
0
0
20
40
60
Time after administration (days)
PBS
ADC-1 3 mg/kg, single administration
ADC-2 1 mg/kg, Q4D × 3
ADC-2 3 mg/kg, single administration
ADC-3 1 mg/kg, Q4D × 3
ADC-3 3 mg/kg, single administration
ADC-4 1 mg/kg, Q4D × 3
ADC-4 3 mg/kg, single administration
ADC-5 1 mg/kg, Q4D × 3
ADC-5 3 mg/kg, single administration

FIG. 2

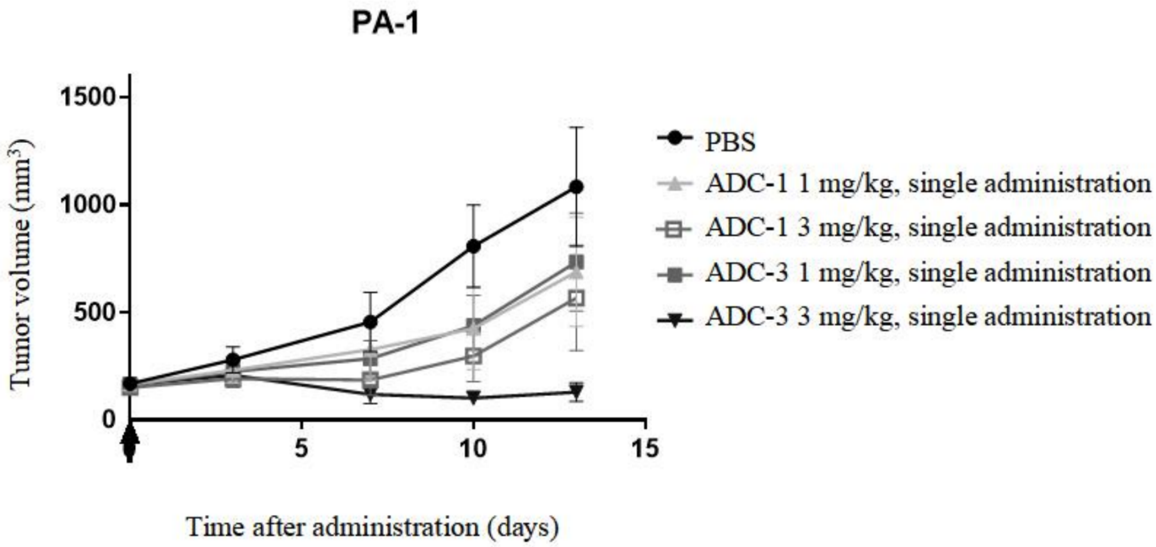
PA-1
Tumor volume (mm3)
1500
1000
500
0
5
10
15
Time after administration (days)
PBS
ADC-1 1 mg/kg, single administration
ADC-1 3 mg/kg, single administration
ADC-3 1 mg/kg, single administration
ADC-3 3 mg/kg, single administration

FIG. 3

## INTERNATIONAL SEARCH REPORT

International application No.
**PCT/CN2024/087225**

**A. CLASSIFICATION OF SUBJECT MATTER**

A61K47/68(2017.01)i; A61K45/00(2006.01)i; A61P35/00(2006.01)i; C07K16/28(2006.01)i

According to International Patent Classification (IPC) or to both national classification and IPC

**B. FIELDS SEARCHED**

Minimum documentation searched (classification system followed by classification symbols)

IPC: A61K; A61P; C07K

Documentation searched other than minimum documentation to the extent that such documents are included in the fields searched

Electronic data base consulted during the international search (name of data base and, where practicable, search terms used)

CNKI, CNTXT, DWPI, ENTXT, ELSEVIER, ISI Web of Knowledge, 中国专利生物序列检索系统, Chinese Patent Biological Sequence Retrieval System, Genbank, EMBL, STN, PubMed: 抗体, 抗原, 抗原结合片段, 单克隆抗体, 单抗, 人源化, antibody, McAb, monoclonal antibody, humanized antibody, CDR, 互补决定区, 重链, 轻链, 相关序列, ROR1, NTRKR1, 抗体药物偶联物, ADC

**C. DOCUMENTS CONSIDERED TO BE RELEVANT**

| Category* | Citation of document, with indication, where appropriate, of the relevant passages | Relevant to claim No. |
|---|---|---|
| A | US 2018369406 A1 (VELOSBIO INC.) 27 December 2018 (2018-12-27)<br>entire document | 1-20 |
| A | CN 113521300 A (CSTONE PHARMACEUTICALS LIMITED et al.) 22 October 2021 (2021-10-22)<br>entire document | 1-20 |
| A | US 2017226183 A1 (CELLECTIS) 10 August 2017 (2017-08-10)<br>entire document | 1-20 |
| | US 2017283497 A1 (CELLECTIS) 05 October 2017 (2017-10-05) | |
| A | CN 114829402 A (NANJING IMMUNOPHAGE BIOTECH CO., LTD.) 29 July 2022 (2022-07-29)<br>entire document | 1-20 |
| A | US 2022323598 A1 (NATIONAL UNIVERSITY CORPORATION TOKAI NATIONAL HIGHER EDUCATION AND RESEARCH SYSTEM) 13 October 2022 (2022-10-13)<br>entire document | 1-20 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **25 June 2024** | **01 July 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/087225**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2024/087225**

| Patent document cited in search report | Publication date (day/month/year) | Patent family member(s) | Publication date (day/month/year) |
|---|---|---|---|
| US 2018369406 A1 | 27 December 2018 | AU 2018289581 A1 | 16 January 2020 |
| | | MX 2019015057 A | 03 August 2020 |
| | | ZA 201908351 B | 28 April 2022 |
| | | IL 271575 A | 27 February 2020 |
| | | BR 112019027448 A2 | 07 July 2020 |
| | | JP 2020525542 A | 27 August 2020 |
| | | JP 7245239 B2 | 23 March 2023 |
| | | KR 20200062161 A | 03 June 2020 |
| | | WO 2018237335 A1 | 27 December 2018 |
| | | KR 20210079427 A | 29 June 2021 |
| | | JP 2023036631 A | 14 March 2023 |
| | | US 2020030454 A1 | 30 January 2020 |
| | | EP 3641830 A1 | 29 April 2020 |
| | | MX 2021007620 A | 11 August 2021 |
| | | CA 3067829 A1 | 27 December 2018 |
| | | TW 201919709 A | 01 June 2019 |
| | | TWI 804499 B | 11 June 2023 |
| | | US 10335496 B2 | 02 July 2019 |
| | | US 2022133901 A1 | 05 May 2022 |
| CN 113521300 A | 22 October 2021 | JP 2021063058 A | 22 April 2021 |
| | | CA 3153069 A1 | 11 March 2021 |
| | | EP 4025257 A1 | 13 July 2022 |
| | | EP 4025257 A4 | 22 November 2023 |
| | | KR 20210028544 A | 12 March 2021 |
| | | AU 2020343888 A1 | 17 March 2022 |
| | | US 2023405139 A1 | 21 December 2023 |
| | | MX 2022002592 A | 25 March 2022 |
| | | JP 2023179421 A | 19 December 2023 |
| | | WO 2021044208 A1 | 11 March 2021 |
| | | WO 2021044208 A8 | 01 April 2021 |
| | | TW 202122115 A | 16 June 2021 |
| | | TW 202408591 A | 01 March 2024 |
| | | US 2021069342 A1 | 11 March 2021 |
| | | US 11707533 B2 | 25 July 2023 |
| | | BR 112022003589 A2 | 24 May 2022 |
| | | IL 290979 A | 01 May 2022 |
| US 2017226183 A1 | 10 August 2017 | EP 3194432 A1 | 26 July 2017 |
| | | EP 3194432 B1 | 10 April 2019 |
| | | WO 2016016343 A1 | 04 February 2016 |
| | | JP 2017522893 A | 17 August 2017 |
| | | CA 2956482 A1 | 04 February 2016 |
| | | US 10544201 B2 | 28 January 2020 |
| US 2017283497 A1 | 05 October 2017 | ES 2715413 T3 | 04 June 2019 |
| | | EA 201790280 A1 | 31 July 2017 |
| | | EA 034081 B1 | 25 December 2019 |
| | | KR 20170062446 A | 07 June 2017 |
| | | KR 102048855 B1 | 26 November 2019 |
| | | AU 2015295349 A1 | 08 December 2016 |
| | | AU 2015295349 B2 | 24 September 2020 |
| | | ES 2876925 T3 | 15 November 2021 |

Form PCT/ISA/210 (patent family annex) (July 2022)

**INTERNATIONAL SEARCH REPORT**
**Information on patent family members**

International application No.
**PCT/CN2024/087225**

| Patent document cited in search report | Publication date (day/month/year) | Patent family member(s) | Publication date (day/month/year) |
|---|---|---|---|
| | | DK 3174557 T3 | 04 February 2019 |
| | | WO 2016016344 A1 | 04 February 2016 |
| | | MX 2017001211 A | 27 April 2017 |
| | | MX 367787 B | 06 September 2019 |
| | | DK 3453406 T3 | 12 July 2021 |
| | | BR 112017001821 A2 | 21 November 2017 |
| | | US 10752684 B2 | 25 August 2020 |
| | | CA 2950381 A1 | 04 February 2016 |
| | | CA 2950381 C | 23 February 2021 |
| | | EP 3453406 A1 | 13 March 2019 |
| | | EP 3453406 B1 | 14 April 2021 |
| | | JP 2017527275 A | 21 September 2017 |
| | | JP 6721568 B2 | 15 July 2020 |
| | | EP 3174557 A1 | 07 June 2017 |
| | | EP 3174557 B1 | 17 October 2018 |
| | | IL 250161 A0 | 30 March 2017 |
| | | IL 250161 B | 31 May 2020 |
| CN 114829402 A | 29 July 2022 | US 2022356246 A1 | 10 November 2022 |
| | | WO 2021057822 A1 | 01 April 2021 |
| US 2022323598 A1 | 13 October 2022 | EP 3831944 A1 | 09 June 2021 |
| | | EP 3831944 A4 | 10 November 2021 |
| | | WO 2020026987 A1 | 06 February 2020 |
| | | JPWO 2020026987 A1 | 19 August 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 1996027011 A1
- WO 1998050431 A
- EP 1870459 A
- WO 2007110205 A
- WO 2009089004 A
- WO 2010129304 A
- WO 201190754 A
- WO 2011143545 A
- WO 2012058768 A
- WO 2013157954 A
- WO 2013096291 A
- US 2010286374 A1
- CN 201110459100
- WO 2022026915 A
- US 2022078563 W
- US 10155821 B2

### Non-patent literature cited in the description

- **SAMBROOK et al.** Molecular Cloning: A Laboratory Manual. Cold Spring Harbor Laboratory Press, 1989
- Current Protocols in Molecular Biology
- Fundamental Immunology. Raven Press, 1989
- **ARAN F. LABRIJN et al.** *Nature Reviews Drug Discovery*, 2019, vol. 18, 585-608
- **CHEN S1 et al.** *J Immunol Res.*, 11 February 2019, vol. 2019, 4516041
- **KABAT et al.** Sequences of Proteins of Immunological Interest. Public Health Service, National Institutes of Health, 1991
- **MARTIN, ACR**. *Protein Sequence and Structure Analysis of Antibody Variable Domains [J*, 2001
- **LEFRANC, M.P. et al.** *Dev. Comp. Immunol.*, 2003, vol. 27, 55-77
- *Front Immunol*, 16 October 2018, vol. 9, 2278
- Epitope Mapping Protocols. Methods in Molecular Biology. 1996, vol. 66
- **JUNGHANS et al.** *Cancer Res.*, 1990, vol. 50, 1495
- Fundamental Immunology. Lippincott-Raven, 1997
- **UCHIDA et al.** *J. Exp. Med.*, 2004, vol. 199, 1659-69
- **AKEWANLOP et al.** *Cancer Res.*, 2001, vol. 61, 4061-65
- **WATANABE et al.** *Breast Cancer Res. Treat.*, 1999, vol. 53, 199-207
- **JANEWAY et al.** Immunobiology. Garland Science, 2005
- **CATER et al.** *Protein Engineering*, 1996, vol. 9 (7), 617-621
- **JONATHAN H. DAVIS et al.** *Protein Engineering, Design & Selection*, 2010, 1-8
- *Science*, 2007, vol. 317 (5844)
- Bioconjugation Technology. Elsevier
- *Bioconjugate Chem*, 2006, vol. 17 (3), 831-840